# EUROPEAN PATENT APPLICATION

(11) **EP 4 821 948 A1**
(43) Date of publication of application: **14.10.2026**
(21) Application number: 24901047.1
(22) Date of filing: 04.12.2024
(51) Int. Cl.: C07K 16/32, C07K 14/725, A61K 47/68, A61P 35/00, A61K 39/00

(54) **NOVEL ANTI-MYCT1 HUMAN ANTIBODY AND USE THEREOF**

(30) Priority: 04.12.2023 KR 20230173228; 03.12.2024 KR 20240177322
(71) Applicant: Atheon Bio, Inc., Seongnam-si, Gyeonggi-do 13487 (KR)
(72) Inventor: CHOE, Soheui, Seongnam-si, Gyeonggi-do 13487 (KR); LEE, Hyun Soo, Seongnam-si, Gyeonggi-do 13487 (KR)
(74) Representative: Dantz, Jan Henning
(86) International application number: PCT/KR2024/019670
(87) International publication number: WO 2025/121861

(57) **Abstract**

The present invention relates to anti-MYCT1 human antibodies and uses thereof.

## Description

### TECHNICAL FIELD

The present invention relates to anti-MYCT1 human antibodies and uses thereof.

The present invention claims priority to and the benefit of Korean Patent Application No. 2023-0173228, filed on December 4, 2023, and Korean Patent Application No. 2024-0177322, filed on December 3, 2024, the disclosures of which are incorporated herein by reference in their entirety.

### BACKGROUND ART

Angiogenesis is a physiological process in which new blood vessels develop from existing blood vessels. Angiogenesis is known to play a role in both normal processes and pathological processes. The regulation of angiogenesis is highly regulated by pro-angiogenic factors and anti-angiogenic factors, and this process is disrupted and dysregulated in cancer. Hypoxia caused by tumors increases the expression of angiogenic factors that induce the formation of new blood vessels essential for tumor survival and proliferation. The VEGF family, consisting of six growth factors (VEGFA-F), binds to receptors VEGFR1-3 and neuropilins and plays the most important role in angiogenesis. Angiogenesis can also be mediated by the angiopoietin (Ang1-2)/Tie-2 pathway independently of the VEGF pathway. Accordingly, over the past decade, drug development has focused on anti-angiogenesis as a strategy for depriving tumors of nutrients and inhibiting tumor growth. However, despite the modest activity of these agents as single agents or in combination with chemotherapy, problems still exist in that tumors overcome their effects and develop resistance.

Immunotherapy for cancer has emerged as a treatment capable of effectively treating various cancers through the discovery of immune checkpoints. Immune checkpoint inhibitors (ICI) have been proven to have longlasting clinical activity against malignant tumors. ICI activate effector immune effector cells that combat cancer by blocking another mechanism hijacked by tumor "immune exhaustion." Primary resistance to ICI occurs in tumors lacking tumor-infiltrating lymphocytes. In addition, tumors that initially respond to ICI may develop secondary resistance due to defects in antigen presentation mechanisms and overexpression of coinhibitory molecules.

Therefore, it is necessary to overcome the problems of VEGF inhibitors and normalize tumor blood vessels to reprogram the tumor microenvironment (TME) so that immune cells can migrate well to the TME.

To this end, drug development targeting MYCT1 (MYC Target 1) is needed. To date, no anti-MYCT1 antibody has been approved as a therapeutic agent for human diseases. Therefore, the development of anti-MYCT1 antibodies having strong binding affinity for MYCT1, excellent specificity, strong ADCC and CDC activity, and excellent anti-tumor activity and neovascularization inhibitory activity through TME reprogramming is required.

### Prior Art Documents

(Non-patent Document) J. Cell. Mol. Med., 2016 Vol 20, No 3, pp. 471-481

### DISCLOSURE

### Technical problem

The present invention relates to anti-MYCT1 human antibodies and uses thereof.

An object of the present invention is to provide a human antibody or an antigen-binding fragment thereof that binds to MYCT1.

Another object of the present invention is to provide a nucleic acid molecule encoding the antibody or antigen-binding fragment, an expression vector comprising the same, and a host cell transfected with the vector.

Still another object of the present invention is to provide an immunoconjugate comprising the antibody or antigen-binding fragment and a cytotoxic agent.

Still another object of the present invention is to provide the antibody or antigen-binding fragment for the purpose of diagnosis or treatment of MYCT1-related diseases.

Still another object of the present invention is to provide the antibody or antigen-binding fragment for the purpose of diagnosing sensitivity to immuno-anticancer agents.

Still another object of the present invention is to provide a chimeric antigen receptor (CAR) comprising the antigen-binding fragment and immune effector cells expressing the same.

However, the technical problems to be achieved by the present invention are not limited to the problems mentioned above, and other problems not mentioned can be clearly understood by those of ordinary skill in the art from the following description.

### Technical Solution

In order to solve the above problems, one aspect of the present invention provides
an anti-MYCT1 human antibody or an antigen-binding fragment thereof specific to human MYCT1, comprising:
a heavy chain CDR1 (HCDR-1) comprising at least one amino acid sequence selected from the group consisting of SEQ ID NOs: 4 to 43;
a heavy chain CDR2 (HCDR-2) comprising at least one amino acid sequence selected from the group consisting of SEQ ID NOs: 44 to 85;
a heavy chain CDR3 (HCDR-3) comprising at least one amino acid sequence selected from the group consisting of SEQ ID NOs: 86 to 187;
a light chain CDR1 (LCDR-1) comprising at least one amino acid sequence selected from the group consisting of SEQ ID NOs: 188 to 264;
a light chain CDR2 (LCDR-2) comprising at least one amino acid sequence selected from the group consisting of SEQ ID NOs: 265 to 320; and
a light chain CDR3 (LCDR-3) comprising at least one amino acid sequence selected from the group consisting of SEQ ID NOs: 321 to 411.

In one embodiment, the HCDR-1, HCDR-2, HCDR-3, LCDR-1, LCDR-2, and LCDR-3 may, in order, comprise a CDR combination of the amino acid sequences set forth in any of the following:
SEQ ID NOs: 13, 76, 105, 222, 301, and 346; SEQ ID NOs: 40, 53, 160, 262, 278, and 404;
SEQ ID NOs: 42, 56, 113, 262, 283, and 407; SEQ ID NOs: 26, 44, 147, 201, 266, and 358;
SEQ ID NOs: 25, 77, 110, 251, 293, and 388; SEQ ID NOs: 6, 68, 143, 230, 304, and 325;
SEQ ID NOs: 25, 77, 109, 252, 293, and 382; SEQ ID NOs: 42, 56, 112, 262, 283, and 409;
SEQ ID NOs: 40, 52, 160, 245, 279, and 401; SEQ ID NOs: 42, 56, 98, 233, 272, and 338;
SEQ ID NOs: 42, 56, 95, 237, 310, and 324; SEQ ID NOs: 25, 77, 114, 246, 275, and 403;
SEQ ID NOs: 25, 77, 126, 251, 293, and 386; SEQ ID NOs: 30, 48, 130, 194, 320, and 378;
SEQ ID NOs: 25, 77, 128, 249, 307, and 328; SEQ ID NOs: 33, 72, 91, 205, 296, and 368;
SEQ ID NOs: 42, 56, 156, 222, 301, and 346; SEQ ID NOs: 16, 63, 131, 262, 282, and 397;
SEQ ID NOs: 25, 77, 125, 251, 293, and 384; SEQ ID NOs: 42, 56, 118, 264, 277, and 392;
SEQ ID NOs: 25, 77, 117, 251, 293, and 387; SEQ ID NOs: 42, 56, 95, 239, 310, and 324;
SEQ ID NOs: 20, 74, 138, 200, 297, and 354; SEQ ID NOs: 6, 65, 123, 193, 319, and 373;
SEQ ID NOs: 42, 56, 95, 239, 310, and 322; SEQ ID NOs: 42, 59, 90, 254, 279, and 395;
SEQ ID NOs: 42, 56, 94, 239, 310, and 325; SEQ ID NOs: 42, 56, 88, 261, 276, and 393;
SEQ ID NOs: 8, 66, 116, 189, 315, and 340; SEQ ID NOs: 42, 56, 133, 260, 295, and 394;
SEQ ID NOs: 42, 56, 87, 241, 290, and 334; SEQ ID NOs: 24, 79, 137, 262, 283, and 405;
SEQ ID NOs: 7, 64, 186, 227, 273, and 342; SEQ ID NOs: 30, 48, 129, 197, 319, and 378;
SEQ ID NOs: 25, 77, 99, 251, 293, and 387; SEQ ID NOs: 25, 77, 164, 203, 296, and 369;
SEQ ID NOs: 42, 56, 163, 250, 294, and 383; SEQ ID NOs: 34, 54, 146, 229, 303, and 333;
SEQ ID NOs: 39, 81, 127, 244, 312, and 335; SEQ ID NOs: 5, 67, 144, 228, 310, and 321;
SEQ ID NOs: 26, 44, 150, 235, 309, and 330; SEQ ID NOs: 42, 56, 104, 247, 293, and 385;
SEQ ID NOs: 27, 77, 172, 262, 278, and 408; SEQ ID NOs: 42, 56, 101, 195, 319, and 379;
SEQ ID NOs: 26, 44, 161, 239, 310, and 324; SEQ ID NOs: 42, 56, 97, 197, 319, and 375;
SEQ ID NOs: 43, 80, 149, 243, 317, and 323; SEQ ID NOs: 27, 51, 152, 210, 268, and 357;
SEQ ID NOs: 25, 78, 119, 231, 293, and 387; SEQ ID NOs: 15, 47, 107, 215, 301, and 341;
SEQ ID NOs: 42, 56, 102, 258, 274, and 402; SEQ ID NOs: 35, 61, 151, 214, 301, and 347;
SEQ ID NOs: 42, 56, 95, 239, 310, and 325; SEQ ID NOs: 8, 66, 116, 188, 314, and 340;
SEQ ID NOs: 14, 85, 153, 220, 299, and 349; SEQ ID NOs: 39, 81, 127, 244, 313, and 335;
SEQ ID NOs: 42, 57, 132, 191, 305, and 389; SEQ ID NOs: 36, 75, 121, 236, 280, and 337;
SEQ ID NOs: 25, 77, 168, 221, 301, and 345; SEQ ID NOs: 31, 56, 148, 197, 319, and 378;
SEQ ID NOs: 42, 56, 106, 256, 285, and 396; SEQ ID NOs: 26, 44, 167, 255, 284, and 398;
SEQ ID NOs: 42, 56, 95, 190, 310, and 324; SEQ ID NOs: 6, 65, 124, 197, 319, and 373;
SEQ ID NOs: 17, 62, 120, 196, 319, and 380; SEQ ID NOs: 4, 65, 166, 226, 318, and 336;
SEQ ID NOs: 42, 57, 96, 239, 310, and 325; SEQ ID NOs: 25, 77, 154, 217, 302, and 343;
SEQ ID NOs: 42, 56, 115, 263, 281, and 411; SEQ ID NOs: 32, 49, 108, 225, 307, and 410;
SEQ ID NOs: 42, 56, 95, 242, 308, and 331; SEQ ID NOs: 26, 44, 150, 234, 316, and 326;
SEQ ID NOs: 42, 56, 95, 192, 311, and 322; SEQ ID NOs: 26, 44, 155, 235, 309, and 330;
SEQ ID NOs: 25, 77, 168, 213, 301, and 345; SEQ ID NOs: 24, 79, 137, 262, 283, and 406;
SEQ ID NOs: 42, 56, 100, 248, 307, and 391; SEQ ID NOs: 26, 44, 183, 197, 319, and 378;
SEQ ID NOs: 25, 77, 93, 251, 293, and 387; SEQ ID NOs: 42, 55, 103, 259, 283, and 400;
SEQ ID NOs: 42, 56, 92, 262, 278, and 399; SEQ ID NOs: 26, 44, 170, 198, 288, and 352;
SEQ ID NOs: 25, 77, 165, 251, 291, and 387; SEQ ID NOs: 42, 56, 102, 257, 274, and 402;
SEQ ID NOs: 25, 77, 111, 253, 292, and 381; SEQ ID NOs: 38, 58, 86, 219, 301, and 348;
SEQ ID NOs: 42, 56, 122, 262, 283, and 405; SEQ ID NOs: 42, 56, 134, 209, 289, and 364;
SEQ ID NOs: 32, 50, 162, 216, 300, and 344; SEQ ID NOs: 20, 69, 141, 211, 269, and 356;
SEQ ID NOs: 12, 73, 140, 199, 297, and 353; SEQ ID NOs: 28, 70, 145, 218, 300, and 390;
SEQ ID NOs: 21, 74, 135, 210, 267, and 370; SEQ ID NOs: 20, 45, 177, 210, 289, and 363;
SEQ ID NOs: 28, 46, 184, 204, 287, and 366; SEQ ID NOs: 20, 45, 175, 209, 265, and 350;
SEQ ID NOs: 26, 74, 178, 209, 286, and 377; SEQ ID NOs: 21, 74, 187, 209, 268, and 374;
SEQ ID NOs: 19, 44, 142, 200, 297, and 355; SEQ ID NOs: 23, 74, 174, 211, 268, and 351;
SEQ ID NOs: 10, 74, 89, 209, 289, and 372; SEQ ID NOs: 37, 60, 139, 206, 286, and 339;
SEQ ID NOs: 41, 83, 157, 232, 306, and 329; SEQ ID NOs: 18, 74, 178, 209, 268, and 372;
SEQ ID NOs: 32, 49, 179, 224, 300, and 344; SEQ ID NOs: 20, 74, 176, 210, 287, and 359;
SEQ ID NOs: 26, 74, 178, 209, 287, and 361; SEQ ID NOs: 22, 71, 171, 210, 286, and 360;
SEQ ID NOs: 11, 44, 159, 208, 286, and 371; SEQ ID NOs: 20, 44, 185, 212, 287, and 365;
SEQ ID NOs: 41, 84, 136, 238, 271, and 332; SEQ ID NOs: 20, 44, 173, 202, 298, and 367;
SEQ ID NOs: 20, 74, 182, 209, 286, and 376; SEQ ID NOs: 9, 74, 180, 207, 286, and 362;
SEQ ID NOs: 41, 82, 158, 240, 270, and 327; or SEQ ID NOs: 29, 44, 181, 209, 286, and 376.

In one embodiment, the antibody or antigen-binding fragment thereof may comprise at least one variable heavy chain region (VH) selected from the group consisting of SEQ ID NOs: 412 to 527.

In another embodiment, the antibody or antigen-binding fragment thereof may comprise at least one variable light chain region (VL) selected from the group consisting of SEQ ID NOs: 528 to 643.

In still another embodiment, the antibody or antigen-binding fragment thereof may comprise:
the VH sequence of SEQ ID NO: 412 and the VL sequence of SEQ ID NO: 528;
the VH sequence of SEQ ID NO: 413 and the VL sequence of SEQ ID NO: 529;
the VH sequence of SEQ ID NO: 414 and the VL sequence of SEQ ID NO: 530;
the VH sequence of SEQ ID NO: 415 and the VL sequence of SEQ ID NO: 531;
the VH sequence of SEQ ID NO: 416 and the VL sequence of SEQ ID NO: 532;
the VH sequence of SEQ ID NO: 417 and the VL sequence of SEQ ID NO: 533;
the VH sequence of SEQ ID NO: 418 and the VL sequence of SEQ ID NO: 534;
the VH sequence of SEQ ID NO: 419 and the VL sequence of SEQ ID NO: 535;
the VH sequence of SEQ ID NO: 420 and the VL sequence of SEQ ID NO: 536;
the VH sequence of SEQ ID NO: 421 and the VL sequence of SEQ ID NO: 537;
the VH sequence of SEQ ID NO: 422 and the VL sequence of SEQ ID NO: 538;
the VH sequence of SEQ ID NO: 423 and the VL sequence of SEQ ID NO: 539;
the VH sequence of SEQ ID NO: 424 and the VL sequence of SEQ ID NO: 540;
the VH sequence of SEQ ID NO: 425 and the VL sequence of SEQ ID NO: 541;
the VH sequence of SEQ ID NO: 426 and the VL sequence of SEQ ID NO: 542;
the VH sequence of SEQ ID NO: 427 and the VL sequence of SEQ ID NO: 543;
the VH sequence of SEQ ID NO: 428 and the VL sequence of SEQ ID NO: 544;
the VH sequence of SEQ ID NO: 429 and the VL sequence of SEQ ID NO: 545;
the VH sequence of SEQ ID NO: 430 and the VL sequence of SEQ ID NO: 546;
the VH sequence of SEQ ID NO: 431 and the VL sequence of SEQ ID NO: 547;
the VH sequence of SEQ ID NO: 432 and the VL sequence of SEQ ID NO: 548;
the VH sequence of SEQ ID NO: 433 and the VL sequence of SEQ ID NO: 549; or
the VH sequence of SEQ ID NO: 434 and the VL sequence of SEQ ID NO: 550.

In still another embodiment, the antibody or antigen-binding fragment thereof may comprise:
the VH sequence of SEQ ID NO: 435 and the VL sequence of SEQ ID NO: 551;
the VH sequence of SEQ ID NO: 436 and the VL sequence of SEQ ID NO: 552;
the VH sequence of SEQ ID NO: 437 and the VL sequence of SEQ ID NO: 553;
the VH sequence of SEQ ID NO: 438 and the VL sequence of SEQ ID NO: 554;
the VH sequence of SEQ ID NO: 439 and the VL sequence of SEQ ID NO: 555;
the VH sequence of SEQ ID NO: 440 and the VL sequence of SEQ ID NO: 556;
the VH sequence of SEQ ID NO: 441 and the VL sequence of SEQ ID NO: 557;
the VH sequence of SEQ ID NO: 442 and the VL sequence of SEQ ID NO: 558;
the VH sequence of SEQ ID NO: 443 and the VL sequence of SEQ ID NO: 559;
the VH sequence of SEQ ID NO: 444 and the VL sequence of SEQ ID NO: 560;
the VH sequence of SEQ ID NO: 445 and the VL sequence of SEQ ID NO: 561;
the VH sequence of SEQ ID NO: 446 and the VL sequence of SEQ ID NO: 562;
the VH sequence of SEQ ID NO: 447 and the VL sequence of SEQ ID NO: 563;
the VH sequence of SEQ ID NO: 448 and the VL sequence of SEQ ID NO: 564;
the VH sequence of SEQ ID NO: 449 and the VL sequence of SEQ ID NO: 565;
the VH sequence of SEQ ID NO: 450 and the VL sequence of SEQ ID NO: 566;
the VH sequence of SEQ ID NO: 451 and the VL sequence of SEQ ID NO: 567;
the VH sequence of SEQ ID NO: 452 and the VL sequence of SEQ ID NO: 568;
the VH sequence of SEQ ID NO: 453 and the VL sequence of SEQ ID NO: 569;
the VH sequence of SEQ ID NO: 454 and the VL sequence of SEQ ID NO: 570;
the VH sequence of SEQ ID NO: 455 and the VL sequence of SEQ ID NO: 571;
the VH sequence of SEQ ID NO: 456 and the VL sequence of SEQ ID NO: 572;
the VH sequence of SEQ ID NO: 457 and the VL sequence of SEQ ID NO: 573;
the VH sequence of SEQ ID NO: 458 and the VL sequence of SEQ ID NO: 574;
the VH sequence of SEQ ID NO: 459 and the VL sequence of SEQ ID NO: 575;
the VH sequence of SEQ ID NO: 460 and the VL sequence of SEQ ID NO: 576;
the VH sequence of SEQ ID NO: 461 and the VL sequence of SEQ ID NO: 577;
the VH sequence of SEQ ID NO: 462 and the VL sequence of SEQ ID NO: 578;
the VH sequence of SEQ ID NO: 463 and the VL sequence of SEQ ID NO: 579;
the VH sequence of SEQ ID NO: 464 and the VL sequence of SEQ ID NO: 580;
the VH sequence of SEQ ID NO: 465 and the VL sequence of SEQ ID NO: 581;
the VH sequence of SEQ ID NO: 466 and the VL sequence of SEQ ID NO: 582;
the VH sequence of SEQ ID NO: 467 and the VL sequence of SEQ ID NO: 583;
the VH sequence of SEQ ID NO: 468 and the VL sequence of SEQ ID NO: 584;
the VH sequence of SEQ ID NO: 469 and the VL sequence of SEQ ID NO: 585;
the VH sequence of SEQ ID NO: 470 and the VL sequence of SEQ ID NO: 586;
the VH sequence of SEQ ID NO: 471 and the VL sequence of SEQ ID NO: 587;
the VH sequence of SEQ ID NO: 472 and the VL sequence of SEQ ID NO: 588;
the VH sequence of SEQ ID NO: 473 and the VL sequence of SEQ ID NO: 589;
the VH sequence of SEQ ID NO: 474 and the VL sequence of SEQ ID NO: 590;
the VH sequence of SEQ ID NO: 475 and the VL sequence of SEQ ID NO: 591;
the VH sequence of SEQ ID NO: 476 and the VL sequence of SEQ ID NO: 592;
the VH sequence of SEQ ID NO: 477 and the VL sequence of SEQ ID NO: 593;
the VH sequence of SEQ ID NO: 478 and the VL sequence of SEQ ID NO: 594;
the VH sequence of SEQ ID NO: 479 and the VL sequence of SEQ ID NO: 595;
the VH sequence of SEQ ID NO: 480 and the VL sequence of SEQ ID NO: 596;
the VH sequence of SEQ ID NO: 481 and the VL sequence of SEQ ID NO: 597;
the VH sequence of SEQ ID NO: 482 and the VL sequence of SEQ ID NO: 598;
the VH sequence of SEQ ID NO: 483 and the VL sequence of SEQ ID NO: 599;
the VH sequence of SEQ ID NO: 484 and the VL sequence of SEQ ID NO: 600;
the VH sequence of SEQ ID NO: 485 and the VL sequence of SEQ ID NO: 601;
the VH sequence of SEQ ID NO: 486 and the VL sequence of SEQ ID NO: 602;
the VH sequence of SEQ ID NO: 487 and the VL sequence of SEQ ID NO: 603;
the VH sequence of SEQ ID NO: 488 and the VL sequence of SEQ ID NO: 604;
the VH sequence of SEQ ID NO: 489 and the VL sequence of SEQ ID NO: 605;
the VH sequence of SEQ ID NO: 490 and the VL sequence of SEQ ID NO: 606;
the VH sequence of SEQ ID NO: 491 and the VL sequence of SEQ ID NO: 607;
the VH sequence of SEQ ID NO: 492 and the VL sequence of SEQ ID NO: 608;
the VH sequence of SEQ ID NO: 493 and the VL sequence of SEQ ID NO: 609;
the VH sequence of SEQ ID NO: 494 and the VL sequence of SEQ ID NO: 610;
the VH sequence of SEQ ID NO: 495 and the VL sequence of SEQ ID NO: 611;
the VH sequence of SEQ ID NO: 496 and the VL sequence of SEQ ID NO: 612;
the VH sequence of SEQ ID NO: 497 and the VL sequence of SEQ ID NO: 613;
the VH sequence of SEQ ID NO: 498 and the VL sequence of SEQ ID NO: 614;
the VH sequence of SEQ ID NO: 499 and the VL sequence of SEQ ID NO: 615;
the VH sequence of SEQ ID NO: 500 and the VL sequence of SEQ ID NO: 616;
the VH sequence of SEQ ID NO: 501 and the VL sequence of SEQ ID NO: 617;
the VH sequence of SEQ ID NO: 502 and the VL sequence of SEQ ID NO: 618;
the VH sequence of SEQ ID NO: 503 and the VL sequence of SEQ ID NO: 619;
the VH sequence of SEQ ID NO: 504 and the VL sequence of SEQ ID NO: 620;
the VH sequence of SEQ ID NO: 505 and the VL sequence of SEQ ID NO: 621;
the VH sequence of SEQ ID NO: 506 and the VL sequence of SEQ ID NO: 622;
the VH sequence of SEQ ID NO: 507 and the VL sequence of SEQ ID NO: 623;
the VH sequence of SEQ ID NO: 508 and the VL sequence of SEQ ID NO: 624;
the VH sequence of SEQ ID NO: 509 and the VL sequence of SEQ ID NO: 625;
the VH sequence of SEQ ID NO: 510 and the VL sequence of SEQ ID NO: 626;
the VH sequence of SEQ ID NO: 511 and the VL sequence of SEQ ID NO: 627;
the VH sequence of SEQ ID NO: 512 and the VL sequence of SEQ ID NO: 628;
the VH sequence of SEQ ID NO: 513 and the VL sequence of SEQ ID NO: 629;
the VH sequence of SEQ ID NO: 514 and the VL sequence of SEQ ID NO: 630;
the VH sequence of SEQ ID NO: 515 and the VL sequence of SEQ ID NO: 631;
the VH sequence of SEQ ID NO: 516 and the VL sequence of SEQ ID NO: 632;
the VH sequence of SEQ ID NO: 517 and the VL sequence of SEQ ID NO: 633;
the VH sequence of SEQ ID NO: 518 and the VL sequence of SEQ ID NO: 634;
the VH sequence of SEQ ID NO: 519 and the VL sequence of SEQ ID NO: 635;
the VH sequence of SEQ ID NO: 520 and the VL sequence of SEQ ID NO: 636;
the VH sequence of SEQ ID NO: 521 and the VL sequence of SEQ ID NO: 637;
the VH sequence of SEQ ID NO: 522 and the VL sequence of SEQ ID NO: 638;
the VH sequence of SEQ ID NO: 523 and the VL sequence of SEQ ID NO: 639;
the VH sequence of SEQ ID NO: 524 and the VL sequence of SEQ ID NO: 640;
the VH sequence of SEQ ID NO: 525 and the VL sequence of SEQ ID NO: 641;
the VH sequence of SEQ ID NO: 526 and the VL sequence of SEQ ID NO: 642; or
the VH sequence of SEQ ID NO: 527 and the VL sequence of SEQ ID NO: 643.

The antigen-binding fragment may include, but is not limited to, Fd, Fab, Fab', F(ab')2, dsFv, scFv, and single domain antibody (sdAb), and any fragment may be used without limitation so long as it retains at least antigen-binding activity.

In another aspect, the present invention provides a nucleic acid molecule encoding the antibody or antigen-binding fragment thereof.

In still another aspect, the present invention provides a recombinant expression vector comprising the nucleic acid molecule; and a host cell transfected with the expression vector.

In still another aspect, the present invention provides an immunoconjugate comprising the antibody or antigen-binding fragment and a cytotoxic agent.

In still another aspect, the present invention provides a pharmaceutical composition for preventing or treating MYCT1-related diseases comprising the antibody or antigen-binding fragment as an active ingredient.

In one embodiment, the MYCT1-related disease may be an angiogenesis-related disease or cancer.

In another embodiment, the pharmaceutical composition may be administered in combination with at least one additional therapeutic agent or therapeutic procedure.

The at least one additional therapeutic agent or therapeutic procedure may be selected from one or more of chemotherapy, targeted anticancer therapy, oncolytic drugs, cytotoxic agents, immune-based therapy, cytokines, surgical procedures, radiation procedures, vaccines, or cell therapy.

In still another aspect, the present invention provides a composition for diagnosing MYCT1-related diseases comprising the antibody or antigen-binding fragment thereof as an active ingredient.

In still another aspect, the present invention provides a pharmaceutical composition for enhancing sensitivity to immuno-anticancer agents in carcinomas, comprising the antibody or antigen-binding fragment thereof as an active ingredient.

In still another aspect, the present invention provides
a chimeric antigen receptor (CAR) comprising an antigen-binding domain that specifically binds to MYCT1. Specifically, the antigen-binding domain may comprise:
a heavy chain CDR1 (HCDR-1) comprising one amino acid sequence selected from the group consisting of SEQ ID NOs: 4 to 43;
a heavy chain CDR2 (HCDR-2) comprising one amino acid sequence selected from the group consisting of SEQ ID NOs: 44 to 85;
a heavy chain CDR3 (HCDR-3) comprising one amino acid sequence selected from the group consisting of SEQ ID NOs: 86 to 187;
a light chain CDR1 (LCDR-1) comprising one amino acid sequence selected from the group consisting of SEQ ID NOs: 188 to 264;
a light chain CDR2 (LCDR-2) comprising one amino acid sequence selected from the group consisting of SEQ ID NOs: 265 to 320; and
a light chain CDR3 (LCDR-3) comprising one amino acid sequence selected from the group consisting of SEQ ID NOs: 321 to 411.

In still another aspect, the present invention provides an immune effector cell comprising the chimeric antigen receptor.

In still another aspect, the present invention provides a method for treating MYCT1-related diseases comprising administering to a subject a composition comprising a therapeutically effective amount of the antibody or antigen-binding fragment.

In still another aspect, the present invention provides a method for enhancing sensitivity to immuno-anticancer agents in an individual, comprising administering to a subject having resistance to immuno-anticancer agents a composition comprising a therapeutically effective amount of the antibody or antigen-binding fragment. However, the technical problems to be achieved by the present invention are not limited to the problems mentioned above, and other problems not mentioned can be clearly understood by those of ordinary skill in the art from the following description.

### Advantageous Effects

The human antibody or antigen-binding fragment thereof that specifically binds to MYCT1 provided by the present invention exhibits excellent affinity and binding capacity for MYCT1, and therefore, antibody-drug conjugates, bispecific antibodies, or chimeric antigen receptors (CARs) comprising the antibody or antigen-binding fragment can be developed. In addition, the antibody or antigen-binding fragment according to the present invention can be usefully applied to the treatment or diagnosis of MYCT1-related diseases, such as, for example, cancer or angiogenesis-related diseases. Furthermore, the antibody or antigen-binding fragment according to the present invention can be used in combination with immuno-anticancer agents and can enhance sensitivity to immuno-anticancer agents.

The effects of the composition according to one embodiment of the present invention are not limited to those mentioned above, and other effects not mentioned can be clearly understood by those of ordinary skill in the art from the following description.

### DESCRIPTION OF DRAWINGS

FIG. 1 is a comparison of human and mouse MYCT1 sequences.
FIGs. 2A, 2B, 2C, and 2D and FIGs. 3A, 3B, 3C and 3D show the results of measuring the viable cell density and viability of ExpiCHO-S cells transfected with the anti-MYCT1 gene.
FIG. 4 shows the results of Protein A HPLC titer measurement of anti-MYCT1 antibodies.
FIGs. 5A, 5B, 5C, 5D, 5E and 5F are Protein A FPLC chromatograms of anti-MYCT1 antibodies.
FIGs. 6A, 6B, 6C, 6D, FIGs. 7A, 7B, 7C, 7D, and FIG. 8 show the results of confirming the purity (SDS-PAGE) of anti-MYCT1 antibodies according to one embodiment of the present invention.
FIGs. 9A, 9B, and 9C show the results of antigen-binding specificity analysis (ELISA) of anti-MYCT1 antibodies according to one embodiment of the present invention.
FIGs. 10A, 10B, 10C, 10D, 10E, 10F, and 10G show the results of cell-binding specificity analysis (FACS) of anti-MYCT1 antibodies according to one embodiment of the present invention.
FIGs. 11A, 11B, and 11C show the results of SPR full kinetics analysis of anti-MYCT1 antibodies according to one embodiment of the present invention.
FIGs. 12A, 12B and FIGs. 13A, 13B show the results of the neovascularization inhibition efficacy evaluation (Migration Assay) of anti-MYCT1 antibodies according to one embodiment of the present invention.

### MODES OF THE INVENTION

The antibody or antigen-binding fragment according to the present invention specifically binds to MYCT1 and through i) angiogenesis inhibition, ii) vascular normalization, iii) increased immune cell activation within the tumor microenvironment (TME), and iv) increased immune cell infiltration into the TME, ultimately reprograms the TME and can overcome the limitations of existing immuno-anticancer agents.

The MYCT1 (hMYCT1) sequence is as follows (SEQ ID NO: 1).

MRTQVYEGLCKNYFSLAVLQRDRIKLLFFDILVFLSVFLLFLLFLVDIMANNTTSLGSPWPENFWED
LIMSFTVSMAIGLVLGGFIWAVFICLSRRRRASAPISQWSSSRRSRSSYTHGLNRTGFYRHSGCERRSNL

SLASLTFQRQASLEQANSFPRKSSFRASTFHPFLQCPPLPVETESQLVTLPSSNISPTISTSHSLSRPDYWS
SNSLRVGLSTPPPPAYESIIKAFPDS

SEQ ID NO: 1 is an example of the MYCT1 sequence, and known MYCT1 sequences, such as mouse MYCT1 sequence (SEQ ID NO: 2), may all be included in the present invention. The human antibody specifically binding to MYCT1 provided in the present invention can specifically bind to the amino acid sequence at positions 49 to 67 of the aforementioned sequence (MANNTTSLGSPWPENFWED, SEQ ID NO: 3).

### Antibody or Antigen-Binding Fragment

In the present specification, the term "antibody" refers to a protein molecule that functions as a ligand that specifically recognizes an antigen, including an immunoglobulin molecule that is immunologically reactive with a specific antigen, and includes polyclonal antibodies, monoclonal antibodies, full-length antibodies, and antibody fragments. Further, in the present invention, the antibody may be a chimeric antibody, bivalent, bispecific molecule, minibody, domain antibody, bispecific or multispecific antibody, immune cell engaging bispecific or multispecific antibody, antibody mimetic, unibody, diabody, triabody, or tetrabody. The term further includes single-chain antibodies with binding function to FcRn (neonatal Fc receptor), scFab, derivatives of antibody constant regions, and artificial antibodies based on protein scaffolds. A full-length antibody has a structure of 2 full-length light chains (LC) and 2 full-length heavy chains (HC), and each light chain is connected to a heavy chain by a disulfide bond. The full-length antibody includes IgA, IgD, IgE, IgM, and IgG, and IgG includes subtypes IgG1, IgG2, IgG3, and IgG4. In one embodiment, the antibody provided in the present invention may be an IgG antibody.

In the present invention, the term "human antibody" refers to a molecule derived from human immunoglobulin, where all amino acid sequences constituting the antibody, including complementarity determining regions (CDR) and structural regions, are composed of amino acid sequences of human immunoglobulin. Human antibodies are typically used in the treatment of human diseases and may have the following advantages. First, they interact more favorably with the human immune system, and thus can more efficiently destroy target cells through, for example, complement-dependent cytotoxicity (CDC) or antibodydependent cell-mediated cytotoxicity (ADCC). Second, the human immune system does not recognize the antibody as foreign. Third, even when administering less drug at lower frequencies, the half-life in human circulation is similar to naturally occurring antibodies.

Furthermore, when the antibody of the present invention includes a constant region, it may include a constant region derived from IgG, IgA, IgD, IgE, IgM, or a combination thereof, or a hybrid thereof.

The antibodies of the present invention can be readily produced using known antibody production techniques. For example, the method of producing a monoclonal antibody can be performed by producing hybridomas using B lymphocytes obtained from immunized animals (Koehler and Milstein, 1976, *Nature*, 256:495), or can be performed using phage display technology, etc., but is not limited thereto, and can be readily produced using other known antibody production techniques.

Antibody libraries using phage display technology are a method in which antibody genes are directly obtained from B lymphocytes without producing hybridomas and antibodies are expressed on the surface of phage (phage). Using phage display technology, many existing difficulties associated with producing monoclonal antibodies through B-cell immortalization can be overcome. Generally, phage display technology includes 1) a step of inserting oligonucleotides of random sequences into the gene region corresponding to the N-terminal of the coat protein pIII (or pIV) of the phage; 2) a step of expressing fusion proteins of part of the native coat protein and a polypeptide encoded by the oligonucleotide of the random sequence; 3) a step of processing a substance capable of binding to the polypeptide encoded by the oligonucleotide; 4) a step of eluting antibodyphage particles bound to the substance using low pH or binding competitive molecules; 5) a step of amplifying the eluted phage in host cells by panning; 6) a step of repeating the method to obtain the desired amount; and 7) a step of determining the sequence of an active antibody from the DNA sequence of phage clones selected by panning.

The method of producing a monoclonal antibody of the present invention can be performed using phage display technology. One skilled in the art can readily perform each step of the production method of the present invention by referring to known phage display technology methods, such as those disclosed in Barbas et al. (METHODS: A Companion to Methods in Enzymology 2:119, 1991 and J. Virol. 2001 Jul;75(14):6692-9) and Winter et al. (Ann. Rev. Immunol. 12:433, 1994). The phage that can be used to construct an antibody library includes, for example, filamentous phage such as fd, M13, f1, If1, Ike, Zj/Z, Ff, Xf, Pf1, or Pf3 phage, but is not limited thereto. Furthermore, vectors that can be used for the expression of heterologous genes on the surface of the filamentous phage include, for example, phage vectors such as fUSE5, fAFF1, fd-CAT1, or fdtetDOG, or phagemid vectors such as pHEN1, pComb3, pComb8, or pSEX, but are not limited thereto. Furthermore, helper phage that can be used to provide wild-type coat protein required for successful reinfection of recombinant phage for amplification include, for example, M13K07 or VSCM13, but are not limited thereto.

The polynucleotide encoding the monoclonal antibody or phage display clone of the present invention can be readily isolated and sequenced using conventional procedures. For example, oligonucleotide primers designed to specifically amplify the corresponding heavy chain and light chain coding regions from phage template DNA can be used. Once the polynucleotide is isolated, it can be inserted into an expression vector, and thereafter the expression vector can be introduced into an appropriate host cell to produce the desired monoclonal antibody from transfected host cells (i.e., transfectants). Therefore, the method of producing the human monoclonal antibody of the present invention comprises a step of amplifying a polynucleotide encoding the human monoclonal antibody in an expression vector comprising a polynucleotide encoding the human monoclonal antibody, and may include this, but is not limited thereto.

In the present invention, "humanized antibody" refers to an antibody that exhibits reduced immunogenicity or non-immunogenicity to humans. The humanized antibody may be prepared by combining CDRs derived from non-human individuals (non-human species) with framework regions (FR) from human antibody-derived constant regions and human antibody-derived variable regions. The humanized antibody can be produced through CDR-grafting methods where CDRs of non-human species antibodies are grafted between FR sequences of human antibodies.

In the present invention, the term "monoclonal antibody" refers to an antibody molecule of a single molecular composition obtained from a substantially identical population of antibodies, and such monoclonal antibodies exhibit single binding specificity and affinity for a specific antigenic determinant (epitope). Typically, immunoglobulins have heavy and light chains, and each heavy chain and light chain contains constant regions and variable regions (these regions are also known as domains). The variable regions of the light chain and heavy chain comprise 3 hypervariable regions, referred to as complementarity-determining regions (CDR), and 4 framework regions.

In the present specification, the term "complementarity determining region" or "CDR region" or "CDR" refers to a portion of very high variability in the sequence within the antibody variable region that structurally forms a defined loop ("hypervariable loop") and/or contains antigen contact residues ("antigen contact sites"). The CDR is primarily responsible for binding to the antigen epitope. The complementarity determining regions are arranged between relatively conserved regions called framework regions (FR). Each heavy chain variable region (VH) and light chain variable region (VL) consist of three CDRs and four FRs, and are arranged from the amino terminus to the carboxyl terminus in the following order: FR1, CDR1, FR2, CDR2, FR3, CDR3, FR4. The variable regions of the heavy and light chains contain binding domains that interact with antigens. The CDRs of the heavy chain variable region are referred to as HCDR-1, HCDR-2, HCDR-3, the CDRs of the light chain variable region are referred to as LCDR-1, LCDR-2, LCDR-3, the FRs of the heavy chain variable region are referred to as HFR1, HFR2, HFR3, HFR4, and the FRs of the light chain variable region are referred to as LFR1, LFR2, LFR3, LFR4. At this time, the numbering is sequential from the N-terminus. The precise amino acid sequence boundaries of each CDR in the amino acid sequence of a given light chain variable region and heavy chain variable region can be determined using one or a combination of known antibody CDR assignment systems, such as Chothia (Chothia et al. (1989) Nature 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th Edition, U.S. Department of Health and Human Services, National Institutes of Health (1987)), AbM (University of Bath), Contact (University College London), International ImMunoGeneTics database (IMGT) (imgt.cines.fr/ on the World Wide Web), or North CDR definition based on affinity propagation clustering using multiple crystal structures.

The antibodies of the present invention may include a constant region, and the constant region may be a constant region derived from IgG, IgA, IgD, IgE, IgM, or a combination thereof, or a hybrid thereof, but is not limited thereto. Here, the term "combination" means that when forming dimers or multimers, a polypeptide encoding the same origin single-chain immunoglobulin constant region forms a bond with single-chain polypeptides of different origins. For example, dimers or multimers can be formed from two or more constant regions selected from the group consisting of constant regions of IgG, IgA, IgD, IgE, and IgM. Furthermore, the term "hybrid" or "hybrid" means that within a single-chain immunoglobulin heavy chain constant region, sequences corresponding to two or more immunoglobulin heavy chain constant regions of different origins are present, and for example, a hybrid of domains selected from the group consisting of 1 to 4 domains selected from CH1, CH2, CH3, and CH4 of IgG, IgA, IgD, IgE, and IgM is possible.

In the present specification, the term "full-length antibody" refers to a structure having 2 full-length light chains and 2 full-length heavy chains, and each light chain is connected to a heavy chain by a disulfide bond, and includes IgA, IgD, IgE, IgM, and IgG. The IgG includes subtypes IgG1, IgG2, IgG3, and IgG4.

In the present specification, the terms "fragment," "binding fragment of a polypeptide," and "antibody fragment" are used interchangeably to refer to any fragment of the antibody of the present invention that retains antigen-binding activity. Exemplary antibody fragments include Fd, Fab, Fab', F(ab')2, dsFv, scFv, and single domain antibodies (sdAb), but are not limited thereto. The Fd refers to the heavy chain portion contained in the Fab fragment. In the present invention, the term "Fab" typically refers to a fragment comprising a heavy chain variable domain and a light chain variable domain, and further includes a light chain constant domain and a heavy chain first constant domain (CH1). The term "Fab'" typically refers to a fragment different from Fab in that a small number of residues (including one or more cysteines from the antibody hinge region) are added to the carboxyl terminus of the heavy chain CH1 domain. The term "F(ab')2" typically refers to a dimeric antibody fragment of Fab' comprising two Fab fragments connected through disulfide bridges in the hinge region. The term "Fv" typically refers to the smallest antibody fragment that contains a complete antigen recognition and binding site. In certain cases, the fragment may consist of a dimer in which the heavy chain variable region and light chain variable region are closely non-covalently bound. The term "dsFv" typically refers to a Fv fragment in which the disulfide bond is stable, and the bond between its single light chain variable region and single heavy chain variable region is a disulfide bond. The term "dAb fragment" typically refers to an antibody fragment consisting of a VH domain. In the present invention, the term "scFv" typically refers to a monovalent molecule formed by covalently linking the heavy chain variable domain and light chain variable domain of an antibody through a flexible peptide linker. Such scFv molecules may have a general structure of NH2-VL-linker-VH-COOH or NH2-VH-linker-VL-COOH. The term "single domain antibody" is known as a nanobody (for example, sdAb, sdFv, nanobody) and refers to an antibody fragment consisting of a single monomeric variable antibody domain.

The terms "human anti-MYCT1 antibody," "anti-MYCT1 human antibody," "anti-MYCT1," "MYCT1 antibody," or "antibody binding to MYCT1" used in the present specification refer to human antibodies that can bind to MYCT1 with sufficient affinity to be used as a therapeutic agent targeting MYCT1. In one embodiment of the present invention, the anti-MYCT1 human antibody binds to MYCT1 with high affinity in vitro or in vivo. Here, the binding can be determined by, for example, radioimmunoassay (RIA), biolayer interferometry (BLI), MSD assay, surface plasmon resonance (SPR), or flow cytometry, but is not limited thereto.

The anti-MYCT1 human antibody or antigen-binding fragment thereof of the present invention may comprise:
heavy chain CDR1 (HCDR-1) comprising an amino acid sequence selected from the group consisting of SEQ ID NOs: 4 to 43;
heavy chain CDR2 (HCDR-2) comprising an amino acid sequence selected from the group consisting of SEQ ID NOs: 44 to 85;
heavy chain CDR3 (HCDR-3) comprising an amino acid sequence selected from the group consisting of SEQ ID NOs: 86 to 187;
light chain CDR1 (LCDR-1) comprising an amino acid sequence selected from the group consisting of SEQ ID NOs: 188 to 264;
light chain CDR2 (LCDR-2) comprising an amino acid sequence selected from the group consisting of SEQ ID NOs: 265 to 320; and
light chain CDR3 (LCDR-3) comprising an amino acid sequence selected from the group consisting of SEQ ID NOs: 321 to 411.

In another embodiment, the HCDR-1, HCDR-2, HCDR-3, LCDR-1, LCDR-2, and LCDR-3 may comprise CDR combinations of amino acid sequences presented below in order, respectively:
SEQ ID NO: 13, 76, 105, 222, 301, and 346; SEQ ID NO: 40, 53, 160, 262, 278, and 404;
SEQ ID NO: 42, 56, 113, 262, 283, and 407; SEQ ID NO: 26, 44, 147, 201, 266, and 358;
SEQ ID NO: 25, 77, 110, 251, 293, and 388; SEQ ID NO: 6, 68, 143, 230, 304, and 325;
SEQ ID NO: 25, 77, 109, 252, 293, and 382; SEQ ID NO: 42, 56, 112, 262, 283, and 409;
SEQ ID NO: 40, 52, 160, 245, 279, and 401; SEQ ID NO: 42, 56, 98, 233, 272, and 338;
SEQ ID NO: 42, 56, 95, 237, 310, and 324; SEQ ID NO: 25, 77, 114, 246, 275, and 403;
SEQ ID NO: 25, 77, 126, 251, 293, and 386; SEQ ID NO: 30, 48, 130, 194, 320, and 378;
SEQ ID NO: 25, 77, 128, 249, 307, and 328; SEQ ID NO: 33, 72, 91, 205, 296, and 368;
SEQ ID NO: 42, 56, 156, 222, 301, and 346; SEQ ID NO: 16, 63, 131, 262, 282, and 397;
SEQ ID NO: 25, 77, 125, 251, 293, and 384; SEQ ID NO: 42, 56, 118, 264, 277, and 392;
SEQ ID NO: 25, 77, 117, 251, 293, and 387; SEQ ID NO: 42, 56, 95, 239, 310, and 324;
SEQ ID NO: 20, 74, 138, 200, 297, and 354; SEQ ID NO: 6, 65, 123, 193, 319, and 373;
SEQ ID NO: 42, 56, 95, 239, 310, and 322; SEQ ID NO: 42, 59, 90, 254, 279, and 395;
SEQ ID NO: 42, 56, 94, 239, 310, and 325; SEQ ID NO: 42, 56, 88, 261, 276, and 393;
SEQ ID NO: 8, 66, 116, 189, 315, and 340; SEQ ID NO: 42, 56, 133, 260, 295, and 394;
SEQ ID NO: 42, 56, 87, 241, 290, and 334; SEQ ID NO: 24, 79, 137, 262, 283, and 405;
SEQ ID NO: 7, 64, 186, 227, 273, and 342; SEQ ID NO: 30, 48, 129, 197, 319, and 378;
SEQ ID NO: 25, 77, 99, 251, 293, and 387; SEQ ID NO: 25, 77, 164, 203, 296, and 369;
SEQ ID NO: 42, 56, 163, 250, 294, and 383; SEQ ID NO: 34, 54, 146, 229, 303, and 333;
SEQ ID NO: 39, 81, 127, 244, 312, and 335; SEQ ID NO: 5, 67, 144, 228, 310, and 321;
SEQ ID NO: 26, 44, 150, 235, 309, and 330; SEQ ID NO: 42, 56, 104, 247, 293, and 385;
SEQ ID NO: 27, 77, 172, 262, 278, and 408; SEQ ID NO: 42, 56, 101, 195, 319, and 379;
SEQ ID NO: 26, 44, 161, 239, 310, and 324; SEQ ID NO: 42, 56, 97, 197, 319, and 375;
SEQ ID NO: 43, 80, 149, 243, 317, and 323; SEQ ID NO: 27, 51, 152, 210, 268, and 357;
SEQ ID NO: 25, 78, 119, 231, 293, and 387; SEQ ID NO: 15, 47, 107, 215, 301, and 341;
SEQ ID NO: 42, 56, 102, 258, 274, and 402; SEQ ID NO: 35, 61, 151, 214, 301, and 347;
SEQ ID NO: 42, 56, 95, 239, 310, and 325; SEQ ID NO: 8, 66, 116, 188, 314, and 340;
SEQ ID NO: 14, 85, 153, 220, 299, and 349; SEQ ID NO: 39, 81, 127, 244, 313, and 335;
SEQ ID NO: 42, 57, 132, 191, 305, and 389; SEQ ID NO: 36, 75, 121, 236, 280, and 337;
SEQ ID NO: 25, 77, 168, 221, 301, and 345; SEQ ID NO: 31, 56, 148, 197, 319, and 378;
SEQ ID NO: 42, 56, 106, 256, 285, and 396; SEQ ID NO: 26, 44, 167, 255, 284, and 398;
SEQ ID NO: 42, 56, 95, 190, 310, and 324; SEQ ID NO: 6, 65, 124, 197, 319, and 373;
SEQ ID NO: 17, 62, 120, 196, 319, and 380; SEQ ID NO: 4, 65, 166, 226, 318, and 336;
SEQ ID NO: 42, 57, 96, 239, 310, and 325; SEQ ID NO: 25, 77, 154, 217, 302, and 343;
SEQ ID NO: 42, 56, 115, 263, 281, and 411; SEQ ID NO: 32, 49, 108, 225, 307, and 410;
SEQ ID NO: 42, 56, 95, 242, 308, and 331; SEQ ID NO: 26, 44, 150, 234, 316, and 326;
SEQ ID NO: 42, 56, 95, 192, 311, and 322; SEQ ID NO: 26, 44, 155, 235, 309, and 330;
SEQ ID NO: 25, 77, 168, 213, 301, and 345; SEQ ID NO: 24, 79, 137, 262, 283, and 406;
SEQ ID NO: 42, 56, 100, 248, 307, and 391; SEQ ID NO: 26, 44, 183, 197, 319, and 378;
SEQ ID NO: 25, 77, 93, 251, 293, and 387; SEQ ID NO: 42, 55, 103, 259, 283, and 400;
SEQ ID NO: 42, 56, 92, 262, 278, and 399; SEQ ID NO: 26, 44, 170, 198, 288, and 352;
SEQ ID NO: 25, 77, 165, 251, 291, and 387; SEQ ID NO: 42, 56, 102, 257, 274, and 402;
SEQ ID NO: 25, 77, 111, 253, 292, and 381; SEQ ID NO: 38, 58, 86, 219, 301, and 348;
SEQ ID NO: 42, 56, 122, 262, 283, and 405; SEQ ID NO: 42, 56, 134, 209, 289, and 364;
SEQ ID NO: 32, 50, 162, 216, 300, and 344; SEQ ID NO: 20, 69, 141, 211, 269, and 356;
SEQ ID NO: 12, 73, 140, 199, 297, and 353; SEQ ID NO: 28, 70, 145, 218, 300, and 390;
SEQ ID NO: 21, 74, 135, 210, 267, and 370; SEQ ID NO: 20, 45, 177, 210, 289, and 363;
SEQ ID NO: 28, 46, 184, 204, 287, and 366; SEQ ID NO: 20, 45, 175, 209, 265, and 350;
SEQ ID NO: 26, 74, 178, 209, 286, and 377; SEQ ID NO: 21, 74, 187, 209, 268, and 374;
SEQ ID NO: 19, 44, 142, 200, 297, and 355; SEQ ID NO: 23, 74, 174, 211, 268, and 351;
SEQ ID NO: 10, 74, 89, 209, 289, and 372; SEQ ID NO: 37, 60, 139, 206, 286, and 339;
SEQ ID NO: 41, 83, 157, 232, 306, and 329; SEQ ID NO: 18, 74, 178, 209, 268, and 372;
SEQ ID NO: 32, 49, 179, 224, 300, and 344; SEQ ID NO: 20, 74, 176, 210, 287, and 359;
SEQ ID NO: 26, 74, 178, 209, 287, and 361; SEQ ID NO: 22, 71, 171, 210, 286, and 360;
SEQ ID NO: 11, 44, 159, 208, 286, and 371; SEQ ID NO: 20, 44, 185, 212, 287, and 365;
SEQ ID NO: 41, 84, 136, 238, 271, and 332; SEQ ID NO: 20, 44, 173, 202, 298, and 367;
SEQ ID NO: 20, 74, 182, 209, 286, and 376; SEQ ID NO: 9, 74, 180, 207, 286, and 362;
SEQ ID NO: 41, 82, 158, 240, 270, and 327; or SEQ ID NO: 29, 44, 181, 209, 286, and 376.

In another embodiment, the antibody or antigen-binding fragment thereof may comprise at least one variable heavy chain region (VH) selected from the group consisting of SEQ ID NOs: 412 to 527.

In another embodiment, the antibody or antigen-binding fragment thereof may comprise at least one variable light chain region (VL) selected from the group consisting of SEQ ID NOs: 528 to 643.

In another embodiment, the antibody or antigen-binding fragment thereof may:
comprise the VH sequence of SEQ ID NO: 412 and the VL sequence of SEQ ID NO: 528; or
comprise the VH sequence of SEQ ID NO: 413 and the VL sequence of SEQ ID NO: 529; or
comprise the VH sequence of SEQ ID NO: 414 and the VL sequence of SEQ ID NO: 530; or
comprise the VH sequence of SEQ ID NO: 415 and the VL sequence of SEQ ID NO: 531; or
comprise the VH sequence of SEQ ID NO: 416 and the VL sequence of SEQ ID NO: 532; or
comprise the VH sequence of SEQ ID NO: 417 and the VL sequence of SEQ ID NO: 533; or
comprise the VH sequence of SEQ ID NO: 418 and the VL sequence of SEQ ID NO: 534; or
comprise the VH sequence of SEQ ID NO: 419 and the VL sequence of SEQ ID NO: 535; or
comprise the VH sequence of SEQ ID NO: 420 and the VL sequence of SEQ ID NO: 536; or
comprise the VH sequence of SEQ ID NO: 421 and the VL sequence of SEQ ID NO: 537; or
comprise the VH sequence of SEQ ID NO: 422 and the VL sequence of SEQ ID NO: 538; or
comprise the VH sequence of SEQ ID NO: 423 and the VL sequence of SEQ ID NO: 539; or
comprise the VH sequence of SEQ ID NO: 424 and the VL sequence of SEQ ID NO: 540; or
comprise the VH sequence of SEQ ID NO: 425 and the VL sequence of SEQ ID NO: 541; or
comprise the VH sequence of SEQ ID NO: 426 and the VL sequence of SEQ ID NO: 542; or
comprise the VH sequence of SEQ ID NO: 427 and the VL sequence of SEQ ID NO: 543; or
comprise the VH sequence of SEQ ID NO: 428 and the VL sequence of SEQ ID NO: 544; or
comprise the VH sequence of SEQ ID NO: 429 and the VL sequence of SEQ ID NO: 545; or
comprise the VH sequence of SEQ ID NO: 430 and the VL sequence of SEQ ID NO: 546; or
comprise the VH sequence of SEQ ID NO: 431 and the VL sequence of SEQ ID NO: 547; or
comprise the VH sequence of SEQ ID NO: 432 and the VL sequence of SEQ ID NO: 548; or
comprise the VH sequence of SEQ ID NO: 433 and the VL sequence of SEQ ID NO: 549; or
comprise the VH sequence of SEQ ID NO: 434 and the VL sequence of SEQ ID NO: 550.

In another embodiment, the antibody or antigen-binding fragment thereof may:
comprise the VH sequence of SEQ ID NO: 435 and the VL sequence of SEQ ID NO: 551; or
comprise the VH sequence of SEQ ID NO: 436 and the VL sequence of SEQ ID NO: 552; or
comprise the VH sequence of SEQ ID NO: 437 and the VL sequence of SEQ ID NO: 553; or
comprise the VH sequence of SEQ ID NO: 438 and the VL sequence of SEQ ID NO: 554; or
comprise the VH sequence of SEQ ID NO: 439 and the VL sequence of SEQ ID NO: 555; or
comprise the VH sequence of SEQ ID NO: 440 and the VL sequence of SEQ ID NO: 556; or
comprise the VH sequence of SEQ ID NO: 441 and the VL sequence of SEQ ID NO: 557; or
comprise the VH sequence of SEQ ID NO: 442 and the VL sequence of SEQ ID NO: 558; or
comprise the VH sequence of SEQ ID NO: 443 and the VL sequence of SEQ ID NO: 559; or
comprise the VH sequence of SEQ ID NO: 444 and the VL sequence of SEQ ID NO: 560; or
comprise the VH sequence of SEQ ID NO: 445 and the VL sequence of SEQ ID NO: 561; or
comprise the VH sequence of SEQ ID NO: 446 and the VL sequence of SEQ ID NO: 562; or
comprise the VH sequence of SEQ ID NO: 447 and the VL sequence of SEQ ID NO: 563; or
comprise the VH sequence of SEQ ID NO: 448 and the VL sequence of SEQ ID NO: 564; or
comprise the VH sequence of SEQ ID NO: 449 and the VL sequence of SEQ ID NO: 565; or
comprise the VH sequence of SEQ ID NO: 450 and the VL sequence of SEQ ID NO: 566; or
comprise the VH sequence of SEQ ID NO: 451 and the VL sequence of SEQ ID NO: 567; or
comprise the VH sequence of SEQ ID NO: 452 and the VL sequence of SEQ ID NO: 568; or
comprise the VH sequence of SEQ ID NO: 453 and the VL sequence of SEQ ID NO: 569; or
comprise the VH sequence of SEQ ID NO: 454 and the VL sequence of SEQ ID NO: 570; or
comprise the VH sequence of SEQ ID NO: 455 and the VL sequence of SEQ ID NO: 571; or
comprise the VH sequence of SEQ ID NO: 456 and the VL sequence of SEQ ID NO: 572; or
comprise the VH sequence of SEQ ID NO: 457 and the VL sequence of SEQ ID NO: 573; or
comprise the VH sequence of SEQ ID NO: 458 and the VL sequence of SEQ ID NO: 574; or
comprise the VH sequence of SEQ ID NO: 459 and the VL sequence of SEQ ID NO: 575; or
comprise the VH sequence of SEQ ID NO: 460 and the VL sequence of SEQ ID NO: 576; or
comprise the VH sequence of SEQ ID NO: 461 and the VL sequence of SEQ ID NO: 577; or
comprise the VH sequence of SEQ ID NO: 462 and the VL sequence of SEQ ID NO: 578; or
comprise the VH sequence of SEQ ID NO: 463 and the VL sequence of SEQ ID NO: 579; or
comprise the VH sequence of SEQ ID NO: 464 and the VL sequence of SEQ ID NO: 580; or
comprise the VH sequence of SEQ ID NO: 465 and the VL sequence of SEQ ID NO: 581; or
comprise the VH sequence of SEQ ID NO: 466 and the VL sequence of SEQ ID NO: 582; or
comprise the VH sequence of SEQ ID NO: 467 and the VL sequence of SEQ ID NO: 583; or
comprise the VH sequence of SEQ ID NO: 468 and the VL sequence of SEQ ID NO: 584; or
comprise the VH sequence of SEQ ID NO: 469 and the VL sequence of SEQ ID NO: 585; or
comprise the VH sequence of SEQ ID NO: 470 and the VL sequence of SEQ ID NO: 586; or
comprise the VH sequence of SEQ ID NO: 471 and the VL sequence of SEQ ID NO: 587; or
comprise the VH sequence of SEQ ID NO: 472 and the VL sequence of SEQ ID NO: 588; or
comprise the VH sequence of SEQ ID NO: 473 and the VL sequence of SEQ ID NO: 589; or
comprise the VH sequence of SEQ ID NO: 474 and the VL sequence of SEQ ID NO: 590; or
comprise the VH sequence of SEQ ID NO: 475 and the VL sequence of SEQ ID NO: 591; or
comprise the VH sequence of SEQ ID NO: 476 and the VL sequence of SEQ ID NO: 592; or
comprise the VH sequence of SEQ ID NO: 477 and the VL sequence of SEQ ID NO: 593; or
comprise the VH sequence of SEQ ID NO: 478 and the VL sequence of SEQ ID NO: 594; or
comprise the VH sequence of SEQ ID NO: 479 and the VL sequence of SEQ ID NO: 595; or
comprise the VH sequence of SEQ ID NO: 480 and the VL sequence of SEQ ID NO: 596; or
comprise the VH sequence of SEQ ID NO: 481 and the VL sequence of SEQ ID NO: 597; or
comprise the VH sequence of SEQ ID NO: 482 and the VL sequence of SEQ ID NO: 598; or
comprise the VH sequence of SEQ ID NO: 483 and the VL sequence of SEQ ID NO: 599; or
comprise the VH sequence of SEQ ID NO: 484 and the VL sequence of SEQ ID NO: 600; or
comprise the VH sequence of SEQ ID NO: 485 and the VL sequence of SEQ ID NO: 601; or
comprise the VH sequence of SEQ ID NO: 486 and the VL sequence of SEQ ID NO: 602; or
comprise the VH sequence of SEQ ID NO: 487 and the VL sequence of SEQ ID NO: 603; or
comprise the VH sequence of SEQ ID NO: 488 and the VL sequence of SEQ ID NO: 604; or
comprise the VH sequence of SEQ ID NO: 489 and the VL sequence of SEQ ID NO: 605; or
comprise the VH sequence of SEQ ID NO: 490 and the VL sequence of SEQ ID NO: 606; or
comprise the VH sequence of SEQ ID NO: 491 and the VL sequence of SEQ ID NO: 607; or
comprise the VH sequence of SEQ ID NO: 492 and the VL sequence of SEQ ID NO: 608; or
comprise the VH sequence of SEQ ID NO: 493 and the VL sequence of SEQ ID NO: 609; or
comprise the VH sequence of SEQ ID NO: 494 and the VL sequence of SEQ ID NO: 610; or
comprise the VH sequence of SEQ ID NO: 495 and the VL sequence of SEQ ID NO: 611; or
comprise the VH sequence of SEQ ID NO: 496 and the VL sequence of SEQ ID NO: 612; or
comprise the VH sequence of SEQ ID NO: 497 and the VL sequence of SEQ ID NO: 613; or
comprise the VH sequence of SEQ ID NO: 498 and the VL sequence of SEQ ID NO: 614; or
comprise the VH sequence of SEQ ID NO: 499 and the VL sequence of SEQ ID NO: 615; or
comprise the VH sequence of SEQ ID NO: 500 and the VL sequence of SEQ ID NO: 616; or
comprise the VH sequence of SEQ ID NO: 501 and the VL sequence of SEQ ID NO: 617; or
comprise the VH sequence of SEQ ID NO: 502 and the VL sequence of SEQ ID NO: 618; or
comprise the VH sequence of SEQ ID NO: 503 and the VL sequence of SEQ ID NO: 619; or
comprise the VH sequence of SEQ ID NO: 504 and the VL sequence of SEQ ID NO: 620; or
comprise the VH sequence of SEQ ID NO: 505 and the VL sequence of SEQ ID NO: 621; or
comprise the VH sequence of SEQ ID NO: 506 and the VL sequence of SEQ ID NO: 622; or
comprise the VH sequence of SEQ ID NO: 507 and the VL sequence of SEQ ID NO: 623; or
comprise the VH sequence of SEQ ID NO: 508 and the VL sequence of SEQ ID NO: 624; or
comprise the VH sequence of SEQ ID NO: 509 and the VL sequence of SEQ ID NO: 625; or
comprise the VH sequence of SEQ ID NO: 510 and the VL sequence of SEQ ID NO: 626; or
comprise the VH sequence of SEQ ID NO: 511 and the VL sequence of SEQ ID NO: 627; or
comprise the VH sequence of SEQ ID NO: 512 and the VL sequence of SEQ ID NO: 628; or
comprise the VH sequence of SEQ ID NO: 513 and the VL sequence of SEQ ID NO: 629; or
comprise the VH sequence of SEQ ID NO: 514 and the VL sequence of SEQ ID NO: 630; or
comprise the VH sequence of SEQ ID NO: 515 and the VL sequence of SEQ ID NO: 631; or
comprise the VH sequence of SEQ ID NO: 516 and the VL sequence of SEQ ID NO: 632; or
comprise the VH sequence of SEQ ID NO: 517 and the VL sequence of SEQ ID NO: 633; or
comprise the VH sequence of SEQ ID NO: 518 and the VL sequence of SEQ ID NO: 634; or
comprise the VH sequence of SEQ ID NO: 519 and the VL sequence of SEQ ID NO: 635; or
comprise the VH sequence of SEQ ID NO: 520 and the VL sequence of SEQ ID NO: 636; or
comprise the VH sequence of SEQ ID NO: 521 and the VL sequence of SEQ ID NO: 637; or
comprise the VH sequence of SEQ ID NO: 522 and the VL sequence of SEQ ID NO: 638; or
comprise the VH sequence of SEQ ID NO: 523 and the VL sequence of SEQ ID NO: 639; or
comprise the VH sequence of SEQ ID NO: 524 and the VL sequence of SEQ ID NO: 640; or
comprise the VH sequence of SEQ ID NO: 525 and the VL sequence of SEQ ID NO: 641; or
comprise the VH sequence of SEQ ID NO: 526 and the VL sequence of SEQ ID NO: 642; or
comprise the VH sequence of SEQ ID NO: 527 and the VL sequence of SEQ ID NO: 643.

In one embodiment, the antibody provided in the present invention may be a monoclonal antibody.

In one embodiment, the antigen-binding fragment provided in the present invention may be scFv.

The aforementioned amino acid sequences may include variants having different sequences through deletion, insertion, substitution, or combination thereof of amino acid residues without affecting the structure, function, activity, etc. of the polypeptide containing them. Furthermore, the aforementioned amino acid sequences may include amino acids that have undergone conventional modifications known in the art, and such amino acid modifications may include, for example, phosphorylation, sulfation, acrylation, glycosylation, methylation, farnesylation, etc.

The antibody or antigen-binding fragment of the present invention includes not only those containing the aforementioned amino acid sequences but also those having substantially identical amino acid sequences thereto or variants thereof. The meaning of having substantially identical amino acid sequences means including amino acid sequences having 90% or more, 91% or more, 92% or more, 93% or more, 94% or more, 95% or more, 96% or more, 97% or more, 98% or more, 99% or more, or 99.5% or more homology to the aforementioned amino acid sequences, but is not limited thereto.

The antibody or antigen-binding fragment of the present invention may further include a linker between the heavy chain variable region and the light chain variable region. Here, the linker may be a peptide linker and may have a length of about 10-25 aa. For example, hydrophilic amino acids such as glycine and/or serine may be included, but is not limited thereto.

Specifically, in the present invention, the linker may include, for example, (GS)n, (GGS)n, (GSGGS)n, or (GnS)m (where n and m are each 1 to 10), and preferably may include GGGGS.

The antibody or antigen-binding fragment of the present invention may include, within a range in which it can specifically recognize MYCT1, not only the sequences of the anti-MYCT1 antibody or antigen-binding fragment of the present invention described herein but also biological equivalents thereof. For example, to further improve the binding affinity and/or other biological characteristics of the antibody, additional modifications can be made to the amino acid sequence of the antibody. Such modifications include, for example, deletion, insertion, and/or substitution of amino acid sequence residues of the antibody or antigen-binding fragment. Such amino acid variations are made based on the relative similarity of amino acid side chain substituents, such as hydrophobicity, hydrophilicity, charge, size, etc. Based on analysis of the size, it can be known that shape, and type of amino acid side chain substituents, arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine can be said to be biologically functional equivalents.

Considering the aforementioned variations with biological equivalent activity, the antibody or antigen-binding fragment of the present invention or the nucleic acid molecule encoding them is interpreted to include sequences that exhibit substantial identity to the sequences set forth in the SEQ ID NOs. The aforementioned substantial identity means, when aligning the sequences of the present invention and any other sequence to correspond as much as possible and analyzing the aligned sequences using algorithms commonly used in the art, sequences showing at least 90% homology, most preferably at least 95% homology, 96% or more, 97% or more, 98% or more, 99% or more homology. Alignment methods for sequence comparison are known in the art. The NCBI Basic Local Alignment Search Tool (BLAST) is accessible from NCBI, etc., and can be used in conjunction with sequence analysis programs such as blastp, blastm, blastx, tblastn, and tblastx on the Internet. BLAST is accessible at www.ncbi.nlm.nih.gov/BLAST/. The method of sequence homology comparison using this program can be found at www.ncbi.nlm.nih.gov/BLAST/blast_help.html.

Based on this, the antibody or antigen-binding fragment of the present invention may have 90%, 91%, 92%, 93%, 94%, 95%, 96%, 97%, 98%, 99%, or greater homology compared to the specific sequence set forth in the specification or to the whole thereof. Such homology can be determined by sequence comparison and/or alignment by methods known in the art. For example, the percent sequence homology of the nucleic acid or protein of the present invention can be determined using sequence comparison algorithms (i.e., BLAST or BLAST 2.0), manual alignment, and visual inspection.

The binding affinity of the anti-MYCT1 human antibody or antigen-binding fragment thereof provided in the present invention to MYCT1 is within the range of 10⁻⁵ M to 10⁻¹² M. For example, the binding affinity of the anti-MYCT1 human antibody or antigen-binding fragment to MYCT1 may be 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M, but is not limited thereto.

In another embodiment of the present invention, it relates to a nucleic acid molecule encoding the antibody or antigen-binding fragment provided in the present invention.

In the present specification, the term "nucleic acid" comprehensively includes DNA (gDNA and cDNA) and RNA molecules, and the nucleotide which is the basic structural unit in nucleic acids includes not only naturally occurring nucleotides but also analogs with modified sugar or base moieties. The sequences of nucleic acids encoding the heavy chain and light chain variable regions of the present invention can be modified. The modification includes addition, deletion, or non-conservative substitution or conservative substitution of nucleotides.

In the present invention, the DNA encoding the antibody or antigen-binding fragment can be readily isolated or synthesized using conventional molecular biological methods (for example, by using oligonucleotide probes capable of specifically binding to DNA encoding the antibody and heavy and light chains), and the nucleic acid can be isolated, inserted into a replicable vector for further cloning (amplification of DNA) or for further expression.

In another embodiment according to the present invention, it relates to a recombinant expression vector comprising a nucleic acid molecule provided in the present invention.

In the present invention, the "vector" refers to a gene construct that is a recombinant vector that can be introduced into appropriate host cells to express a desired protein and contains essential regulatory elements operably linked for expression of the genetic insert. Here, "operably linked" means that a nucleic acid expression control sequence and a nucleic acid sequence encoding a desired protein are functionally linked to perform a general function. Operative linkage with recombinant vectors can be prepared using genetic recombination techniques well known in the art, and site-specific DNA cleavage and ligation can be readily accomplished using enzymes commonly known in the art.

In the present invention, various types of vectors such as nanoparticles, plasmids, viruses, and cosmids can be used as recombinant expression vectors for inserting the foreign gene. The type of recombinant vector is not particularly limited as long as it functions to express the desired gene in various host cells of prokaryotes and eukaryotes and produce the desired protein, but specifically, vectors with powerful promoters and strong expression capacity that can produce large amounts of foreign proteins in a form similar to the natural state can be used.

Various gene transfer vehicles are known in the art, and include both viral and non-viral (e.g., naked DNA, plasmid) vectors. Viral vectors suitable for gene transfer are known to those skilled in the art. Non-limiting examples of the viral vectors include retroviral vectors (derived from Moloney murine leukemia virus vectors (MoMLV), MSCV, SFFV, MPSV, SNV, etc.), lentiviral vectors (for example, derived from HIV-1, HIV-2, SIV, BIV, FIV, etc.), adenovirus (Ad) vectors including replication-competent, replication-deficient, and defective forms thereof, adeno-associated virus (AAV) vectors, Simian virus 40 (SV-40) vectors, bovine papillomavirus vectors, Epstein-Barr virus vectors, herpes virus vectors, varicella virus vectors, Harvey murine sarcoma virus vectors, murine mammary tumor virus vectors, Rous sarcoma virus vectors, parvovirus vectors, poliovirus vectors, vesicular stomatitis virus vectors, maraba virus vectors, and Group B adenovirus enadenovirus vectors, etc.

Non-viral vectors for gene transfer include naked DNA, plasmids, transposons, and mRNA. Non-limiting examples include pKK plasmid (Clonetech), pUC plasmid, pET plasmid (Novagen, Inc., Madison, Wis.), pRSET or pREP plasmid (Invitrogen, San Diego, Calif.), pMAL plasmid (New England Biolabs, Beverly, Mass.).

In the present invention, the vector can be introduced into many appropriate host cells using methods disclosed herein, cited, or otherwise known to those skilled in the art.

Appropriate expression vectors of the present invention may comprise, in addition to expression control elements such as promoters, start codons, stop codons, polyadenylation signals, or enhancers, base sequences encoding signal peptides for membrane targeting or secretion. Start and stop codons are generally considered part of the nucleotide sequence encoding the immunogenic target protein, and when the gene construct is administered, it must function in the individual and must be in-frame with the coding sequence.

In the present specification, the term "promoter" refers, as used herein, to any sequence that regulates the expression of a coding sequence such as a gene. A promoter may be, for example, constitutive, inducible, repressive, or tissue-specific. A promoter is a control sequence that is the region of the polynucleotide sequence in which the start and rate of transcription are controlled. In the present invention, non-limiting examples of the promoter include Rous sarcoma virus (RSV) LTR promoter (optionally having RSV enhancer), cytomegalovirus (CMV) promoter, SV40 promoter, dihydrofolate reductase promoter, beta-actin promoter, phosphoglycerate kinase (PGK) promoter, U6 promoter, EF1 alpha short form (EFS) promoter, human polypeptide chain elongation factor (EF1a) promoter, P5 promoter, Ubc promoter, CAG promoter, TRE promoter, UAS promoter, Ac5 promoter, polyhedrin promoter, CaMKIIa promoter, Gal1 promoter, TEF1 promoter, GDS promoter, ADH1 promoter, CaMV35S promoter, ubiquitin (Ubi) promoter, such as ubiquitin C (UbiC), H1 promoter, U6 promoter, alpha-1-antitrypsin promoter, spleen focus-forming virus (SFFV) promoter, etc.

Furthermore, in the present invention, the promoter can be coupled to an enhancer to increase transcription efficiency. In this case, non-limiting examples of the enhancer include RSV enhancer, CMV enhancer, or alpha-fetoprotein MERII enhancer, etc., but are not limited thereto. The vector of the present invention may be fused with other sequences to facilitate purification of antibodies expressed therefrom. The sequences to be fused include, for example, glutathione S-transferase (Pharmacia, USA), maltose binding protein (NEB, USA), FLAG (IBI, USA), and 6x His (hexahistidine; Quiagen, USA), etc., but are not limited thereto.

The vector of the present invention includes antibiotic resistance genes commonly used as selection markers in the art, and may include, for example, genes conferring resistance to ampicillin, gentamicin, carbenicillin, chloramphenicol, streptomycin, kanamycin, geneticin, neomycin, and tetracycline, but are not limited thereto. According to another embodiment of the present invention, it relates to host cells transfected with the expression vector provided in the present invention.

In the present specification, the term "host cell" may include individual cells or cell cultures that may be recipients or were recipients of vector(s) for incorporation of the polypeptide insert. Host cells include offspring of a single host cell, and such offspring may not necessarily be completely identical to the original parent cell due to natural, accidental, or intentional mutations (morphologically or in genomic DNA complement). Host cells include cells transfected in vivo with the polypeptide(s) of the present application.

In the present invention, the host cell may include cells of mammalian, plant, insect, fungal, or cellular origin, and for example, bacterial cells such as E. coli, Streptomyces, Salmonella typhimurium, etc.; fungal cells such as yeast cells, Pichia pastoris, etc.; insect cells such as Drosophila, Spodoptera Sf9 cells, etc.; CHO (Chinese hamster ovary cells), SP2/0 (murine myeloma), human lymphoblastoid, COS, NSO (murine myeloma), 293T, bovine melanoma cells, HT-1080, BHK (Baby Hamster Kidney cells), HEK (Human Embryonic Kidney cells), or PERC.6 (human retinal cells); or plant cells, but is not limited thereto, and any cells available as host cell lines known to those skilled in the art can be used.

In the present invention, the transfection method is any method of introducing the desired vector into the host cell, and includes any known method capable of introducing a vector into a host cell, such as CaCl2-mediated methods, electroporation, microinjection, calcium phosphate precipitation, electroporation, liposome-mediated transfection, DEAE-dextran treatment, gene bombardment, and viral-mediated transfection, etc., but is not limited thereto.

According to another embodiment, the present invention relates to a method of producing an antibody or antigen-binding fragment that specifically binds to MYCT1, comprising: culturing the host cell to produce an antibody or antigen-binding fragment; and isolating and purifying the produced antibody or antigen-binding fragment.

In the present invention, the host cell can be cultured in various media. Any commercially available medium can be used without limitation as a culture medium. All other essential supplements known to those skilled in the art may also be included at appropriate concentrations. Culture conditions, such as temperature, pH, etc., are already being used with host cells selected for expression, and this will be apparent to those skilled in the art.

In the present invention, recovery of the antibody or antigen-binding fragment thereof can be performed by removing impurities by, for example, centrifugation or ultrafiltration, and the resulting product can be purified using, for example, affinity chromatography or the like. Additional other purification techniques such as anion or cation exchange chromatography, hydrophobic interaction chromatography, hydroxyapatite chromatography, etc., can be used.

According to another embodiment of the present invention, it relates to bispecific or multispecific antibodies comprising the antibody or antigen-binding fragment provided in the present invention.

In the present specification, the term "bispecific antibody" refers to an antibody with binding or antagonistic capability against one or more targets, and means an antibody with binding or antagonistic capability against two different targets bonded together, or an antibody in which a substance with binding capability against one target and a substance with antagonistic capability against another target are bonded.

In the present specification, the term "multispecific antibody" refers to an antibody having binding specificity against at least three or more different antigens. Multispecific antibodies may include antibodies of three or more specificities, for example, tri-specific antibodies, tetra-specific antibodies, or antibodies targeting higher numbers of targets.

In the present invention, antibodies belonging to bispecific or multispecific antibodies can be classified into scFv-based antibodies, Fab-based antibodies, and IgG-based antibodies. In the case of bispecific or multispecific antibodies, two or more signals can be simultaneously inhibited or amplified, which can be more effective than the case of inhibiting/amplifying a single signal, and compared to the case where each signal is treated with a respective signal inhibitor, lower-dose administration is possible, and two or more signals in the same time and space can be inhibited/amplified.

In the present invention, methods of producing bispecific or multispecific antibodies are widely known. Traditionally, recombinant production of bispecific antibodies is based on co-expression of two or more immunoglobulin heavy chain/light chain pairs under conditions where the two or more heavy chains have different specificities.

In the present invention, in the case of bispecific or multispecific antibodies based on scFv, diabodies can be made by combining VL and VH of different scFvs in heterodimeric form, tandem scFv can be produced by linking different scFvs together, and heterodimeric miniantibodies can be produced by expressing the CH1 and CL of Fab at the termini of respective scFvs, and by replacing some amino acids of the CH3 domain, which is the homodimeric domain of Fc, to change them to 'knob into hole' form heterodimeric structures, heterodimeric scFv-type minibodies can be produced by expressing these modified CH3 domains at the termini of different scFvs.

In the present invention, in the case of bispecific or multispecific antibodies based on Fab, individual Fab' against specific antigens can be combined using disulfide bonds or mediators in heterodimeric Fab form, and by expressing different scFv against specific antigens at the termini of the heavy or light chains of specific Fab, the antigen valency can be made 2, or by placing a hinge region between Fab and scFv, it can be produced with 4 antigen-binding valencies in homodimeric form. Furthermore, by fusing different scFv against antigens at the light chain terminus and heavy chain terminus of Fab, a dual-target bibody with antigen valency of 3, by fusing different scFv at the light chain terminus and heavy chain terminus of Fab respectively, a triple-target bibody with antigen valency of 3, or by chemically conjugating three different Fab can be obtained.

In the present invention, in the case of bispecific or multispecific antibodies based on IgG, a method is known in which Trion Pharma re-hybridizes mouse and rat hybridomas to produce hybrid hybridomas, also known as quadromas, to produce bispecific antibodies. Furthermore, while the light chain portion is shared, and some amino acids of the Fc CH3 homodimeric domain are modified for different heavy chains to be produced in heterodimeric form as the so-called 'Holes and Knob' form, bispecific antibodies can be produced. In addition to heterodimeric bispecific antibodies, different scFv can be fused and expressed at the constant domains instead of the variable domains of the heavy and light chains of IgG to be produced as homodimeric (scFv)4-IgG. Furthermore, ImClone has reported producing bispecific antibodies by fusing only a single variable domain against mouse platelet-derived growth factor receptor-alpha (Platelet-derived Growth Factor Receptor-α) to the amino terminus of the light chain of IMC-1C11, a chimeric monoclonal antibody against human VEGFR-2. Furthermore, antibodies with multiple antigen-binding valencies against CD20 can be produced through the so-called 'dock and lock (DNL)' method using the dimerization and docking domain (DDD) of the protein kinase A (PKA) R subunit and the anchoring domain of PKA.

In the present invention, many diverse recombinant antibody formats have been developed, such as bispecific or multispecific antibodies with bivalency or higher, trivalency or higher, or tetravalency or higher. For example, the bispecific or multispecific antibodies with bivalency or higher, trivalency or higher, or tetravalency or higher described in International Patent Application Publication Nos. WO2001/077342, WO2009/080251, WO2009/080252, WO2009/080253, WO2009/080254, WO2010/112193, WO2010/115589, WO2010/136172, WO2010/145792, WO2010/145793, and WO2011/117330 are also included. Bispecific or multispecific antibodies with bivalency or higher, trivalency or higher, or tetravalency or higher indicate that two or more binding domains, three or more binding domains, or four or more binding domains, respectively, are present in the antibody molecule.

The bispecific or multispecific antibody according to the present invention may specifically comprise the anti-MYCT1 antibody or antigen-binding fragment in the form of a complete IgG antibody or a fragment thereof, for example, in the form of single-chain Fv, VH domain and/or VL domain, Fab, or (Fab)2. Furthermore, the antibody targeting MYCT1 and an antibody binding to a different target, for example, PD-1, PD-L1, BTLA, CTLA-4, VISTA, LAG3, TIM3, CD137(4-1BB), VISTA, CD258(LIGHT), TIGIT, CD134(OX40), CD28, CD278(ICOS), CD27, CD154(CD40L), CD357(GITR), CD30, DR3, CD226(DNAM1), CD96, CD200, CD200R, Transferrin receptor, c-Met, EGFR, HER2, KDR, PDGFRa, NRP1, MARCO, an antibody targeting one or more selected from the group consisting of the above, specifically in the form of a complete IgG antibody or a fragment thereof, for example, in the form of single-chain Fv, VH domain and/or VL domain, Fab, or (Fab)2 may be included.

Through the bispecific or multispecific antibody according to the present invention, in addition to MYCT1, additional binding specificity derived from or mediated by other targets can be obtained.

For example, the bispecific antibody according to the present invention can simultaneously target MYCT1 and one or more selected from the group consisting of PD-1, PD-L1, BTLA, CTLA-4, VISTA, LAG3, TIM3, CD137(4-1BB), VISTA, CD258(LIGHT), TIGIT, CD3, CD20, CD134(OX40), CD28, CD278(ICOS), CD27, CD154(CD40L), CD357(GITR), CD30, DR3, CD226(DNAM1), CD96, CD200, CD200R, Transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

For example, the multispecific antibody according to the present invention can simultaneously target MYCT1 and two or more selected from the group consisting of PD-1, PD-L1, BTLA, CTLA-4, VISTA, LAG3, TIM3, CD137(4-1BB), VISTA, CD258(LIGHT), TIGIT, CD3, CD20, CD134(OX40), CD28, CD278(ICOS), CD27, CD154(CD40L), CD357(GITR), CD30, DR3, CD226(DNAM1), CD96, CD200, CD200R, Transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

According to another embodiment of the present invention, it relates to immune cell engaging (immune cell engage) bispecific or multispecific antibodies comprising one or more scFv of the antigen-binding fragment provided in the present invention and scFv binding to immune cell activation antigens.

In the present invention, through the immune cell engaging bispecific or multispecific antibody, temporary cytolytic synapses between cytotoxic T cells or NK cells and cancer target cells are induced to release cytotoxins.

In the present invention, the T cell activation antigen may be CD3, TCRα, TCRβ, TCRγ, TCRξ, HVEM, LIGHT, CD40, 4-1BB, OX40, DR3, GITR, CD30, TIM1, SLAM, CD2, or CD226, but is not limited thereto.

In the present invention, the NK cell activation antigen may be NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160, but is not limited thereto.

Other immune cell engagers are specifically described in U.S. Patent Application Publication US 2017/0368169, and may be incorporated herein by reference.

Specifically, the immune cell engaging bispecific or multispecific antibody comprises serial scFv and can bind to antigens and surface antigens on cancer cells as follows. The surface antigen on the cancer cell is MYCT1 targeted by the antibody according to the present invention.

In the present invention, the immune cell engaging bispecific or multispecific antibody may, for example, comprise a structure of VL(MYCT1)-VH(MYCT1)-VH(CD3 or CD16A)-VL(CD3 or CD16A), VH(MYCT1)-VL(MYCT1)-VH(CD3 or CD16A)-VL(CD3 or CD16A), VH(CD3 or CD16A)-VL(CD3 or CD16A)-VH(MYCT1)-VL(MYCT1), or VH(CD3 or CD16A)-VL(CD3 or CD16A)-VL(MYCT1)-VH(MYCT1).

In the present invention, a linker may be further included between the heavy chain variable region and the light chain variable region. Here, the linker may be a peptide linker and may have a length of about 10-25 aa. For example, hydrophilic amino acids such as glycine and/or serine may be included, but is not limited thereto.

Specifically, in the present invention, the linker may include, for example, (GS)n, (GGS)n, (GSGGS)n, or (GnS)m (where n and m are each 1 to 10), and preferably may include GGGGS, and preferably may be GGGGSGGGGSGGGGS, but is not limited thereto.

Examples of the immune cell engaging bispecific or multispecific antibody in the present invention include blinatumomab (Amgen), which binds to CD3 and CD19; solitomab (Amgen), which binds to CD3 and EpCAM; MEDI 565 (MedImmune, Amgen), which binds to CD3 and CEA; and BAY2010112 (Bayer, Amgen), which binds to CD3 and PSMA. Exemplary DARTs include MGD006 (Macrogenics), which binds to CD3 and CD123; and MGD007 (Macrogenics), which binds to CD3 and gpA33. Exemplary TandAbs may include AFM11 (Affimed Therapeutics), which binds to CD3 and CD19; and AFM13 (Affimed Therapeutics), which binds to CD30 and CD16A.

### Immunoconjugate

According to another embodiment of the present invention, the present invention relates to an immunoconjugate in which an antibody or antigen-binding fragment provided by the present invention is combined with a cytotoxic agent.

In the present invention, the cytotoxic agent may be one or more selected from the group consisting of a chemotherapeutic agent or chemotherapy drug, a growth inhibitor, a toxin (for example, a protein toxin, a bacterium, fungus, plant or animal-derived enzymatically active toxin or a fragment thereof) and a radioisotope.

In one embodiment, the immunoconjugate provided by the present invention may be an antibody-drug conjugate (ADC) in which the antibody or antigen-binding fragment is bound to a drug.

In the present specification, the term "antibody-drug conjugate (Antibody-Drug Conjugate, ADC)" refers to a form in which a drug and an antibody are chemically linked while not reducing the biological activity of the antibody and the drug. In the present invention, the antibody-drug conjugate refers to a form in which a drug is bound to an amino acid residue at the N-terminus of the heavy chain and/or light chain of an antibody, specifically a form in which a drug is bound to the N-terminus, α-amine group of the heavy chain and/or light chain of an antibody.

In the present invention, the antibody or antigen-binding fragment may be bound to a drug through a linker. The linker is a site connecting the antibody and the drug, which is cleavable under intracellular conditions, that is, allows the drug to be released from the antibody in an intracellular environment, and reflecting the long half-life of the antibody, the antibody is stable during systemic circulation, and the binding of the linker and drug should not affect the stability of the antibody and drug kinetics.

In the present invention, the linker may include, for example, a cleavable linker or a non-cleavable linker. In the case of a cleavable linker, it can be cleaved by intracellular peptidases or protease enzymes such as lysosomal or endosomal proteases, similar to a peptide linker, and in the case of a non-cleavable linker, for example, a thioether linker can release the drug after the antibody is non-selectively degraded by intracellular hydrolysis.

In the present invention, the cleavable linker may include a peptide linker. The peptide linker has a length of at least 2 or more amino acids. For example, a dipeptide of Val-Cit, Val-Ala, or Val-Cit or Phe-Leu or Gly-Phe-Leu-Gly may be included. Examples of linkers are specifically described in International Patent Application Publication No. WO2004/010957, and can be incorporated by reference in the present invention.

In the present invention, the antibody-drug conjugate forms an ADC-antigen complex after the antibody region of the ADC binds to the antigen of the target cancer cell, and then is internalized into the cancer cell through an endosome-lysosome pathway. In this case, the intracellular release of the cytotoxic drug is regulated by the internal environment of the endosome/lysosome.

In the present invention, the cleavable linker is pH-sensitive, and may be sensitive to hydrolysis at a specific pH value. Generally, pH-sensitive linkers are indicative of being hydrolyzed under acidic conditions. For example, an acid-labile linker that can be hydrolyzed in a lysosome, such as hydrazone, semicarbazone, thiosemicarbazone, cis-aconitic amide, orthoesters, acetals, ketals, and the like.

In the present invention, the linker may be cleaved under reducing conditions, and for example, a disulfide linker may be applicable to this. A variety of disulfide bonds can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene). These disulfide linkers can be degraded by thiol-disulfide exchange with intracellular glutathione.

In the present invention, the drug and/or drug-linker may be randomly conjugated through lysine of the antibody, or conjugated through cysteine exposed when the disulfide-bonded chain is reduced. In some cases, a linker-drug may be conjugated through cysteine present in a genetically engineered tag, for example, a peptide or protein. The genetically engineered tag, for example, a peptide or protein may include an amino acid motif that can be recognized by, for example, isoprenoid transferase. The peptide or protein has a deletion at the carboxy terminus of the peptide or protein, or has an addition through a covalent bond of a spacer unit to the carboxy (C) terminus of the peptide or protein.

In the present invention, the peptide or protein may be directly covalently bonded to the amino acid motif or covalently bonded to a spacer unit to be connected to the amino acid motif. The amino acid spacer unit is composed of 1 to 20 amino acids, among which the glycine (Glycine) unit is preferred.

In the present invention, the linker may include a beta-glucuronide linker that is recognized and hydrolyzed by beta-glucuronidase (β-glucuronidase), which is present in multiple copies in the lysosome or is overexpressed in some tumor cells. Unlike peptide linkers, it has greater hydrophilicity and has the advantage of being able to increase the solubility of the antibody-drug complex when combined with a drug having high hydrophobic properties. In this regard, a beta-glucuronide linker disclosed in International Patent Application Publication No. WO2015/182984, for example, a beta-glucuronide linker comprising a self-immolative group can be used, and the above document is incorporated by reference.

In the present invention, the linker may be, for example, a non-cleavable linker, and the drug is released through only one step of antibody hydrolysis in cells, for example, producing an amino acid-linker-drug complex. This type of linker may be a thioether group or maleimidocaproyl group, and can maintain stability in blood.

In the present invention, a linker-drug may be conjugated via cysteine residues exposed upon reduction of disulfide bonds in the antibody or antigen-binding fragment, either by random conjugation or by introducing an antibody-terminal-binding peptide having the sequence GGGGGGGCVIM.

In the present invention, the drug is a formulation exhibiting a pharmacological effect, which can be bound to an antibody, and specifically may be a chemotherapeutic agent, a toxin, microRNA (miRNA), siRNA, shRNA or a radioisotope. The chemotherapeutic agent may be, for example, a cytotoxic agent or an immunosuppressive agent. Specifically, it may include a chemotherapeutic agent that can function as a microtubule inhibitor, a mitosis inhibitor, a topoisomerase inhibitor, or a DNA intercalator. Additionally, it may include a drug for the treatment of cancer, inflammation or immune diseases. For example, it may include an immunomodulatory compound, an anticancer agent, an antiviral agent, an antibacterial agent, an antifungal agent, an antiparasitic agent, or a combination thereof.

In the present invention, the anticancer agent may be, for example, a maytansinoid, auristatin (including MMAE, MMAF), aminopterin, actinomycin, bleomycin, talisomycin, camptothecin, N8-acetyl spermidine, 1-(2-chloroethyl)-1,2-dimethyl sulfonyl hydrazide, esperamycin, etoposide, 6-mercaptopurine, dolastatin, trichothecene, calicheamicin, taxol (taxol), taxane, paclitaxel (paclitaxel), docetaxel (docetaxel), methotrexate, vincristine, vinblastine, doxorubicin, melphalan, mitomycin A, mitomycin C, chlorambucil, duocamycin, L-asparaginase (L-asparaginase), mercaptopurine (mercaptopurine), thioguanine (thioguanine), hydroxyurea (hydroxyurea), cytarabine (cytarabine), cyclophosphamide (cyclophosphamide), ifosfamide (ifosfamide), nitrosourea (nitrosourea), cisplatin (cisplatin), carboplatin (carboplatin), mitomycin (mitomycin), dacarbazine (dacarbazine), procarbazine (procarbazine), topotecan (topotecan), nitrogen mustard (nitrogen mustard), cytoxan (cytoxan), etoposide (etoposide), 5-fluorouracil (5-fluorouracil), CNU (bischloroethylnitrosourea), irinotecan (irinotecan), camptothecin (camptothecin), bleomycin (bleomycin), idarubicin (idarubicin), daunorubicin (daunorubicin), dactinomycin (dactinomycin), plicamycin (plicamycin), mitoxantrone (mitoxantrone), asparaginase (asparaginase), vinorelbine (vinorelbine), chlorambucil (chlorambucil), melphalan (melphalan), carmustine (carmustine), lomustine (lomustine), busulfan (busulfan), treosulfan (treosulfan), decarbazine (decarbazine), etoposide (etoposide), teniposide (teniposide), topotecan (topotecan), 9-aminocamptothecin (9-aminocamptothecin), crisnatol (crisnatol), mitomycin C (mitomycin C), trimetrexate (trimetrexate), mycophenolic acid (mycophenolic acid), tiazofurin (tiazofurin), ribavirin (ribavirin), EICAR (5-ethynyl-1-beta-D-ribofuranosylimidazole-4-carboxamide), hydroxyurea (hydroxyurea), deferoxamine (deferoxamine), floxuridine (floxuridine), doxifluridine (doxifluridine), raltitrexed (raltitrexed), cytarabine (cytarabine(ara C)), cytosine arabinoside (cytosine arabinoside), fludarabine (fludarabine), tamoxifen (tamoxifen), raloxifene (raloxifene), megestrol (megestrol), goserelin (goserelin), leuprolide acetate (leuprolide acetate), flutamide (flutamide), bicalutamide (bicalutamide), EB1089, CB1093, KH1060, verteporfin (verteporfin), phthalocyanine (phthalocyanine), photosensitizer Pe4 (photosensitizer Pe4), demethoxy-hypocrellin A (demethoxy-hypocrellin A), Interferon-α (Interferon-α), Interferon-γ (Interferon-γ), tumor necrosis factor (tumor necrosis factor), Gemcitabine (Gemcitabine), velcade (velcade), revamid (revamid), thalamid (thalamid), lovastatin (lovastatin), 1-methyl-4-phenylpyridiniumion (1-methyl-4-phenylpyridiniumion), staurosporine (staurosporine), actinomycin D (actinomycin D), dactinomycin (dactinomycin), bleomycin A2 (bleomycin A2), bleomycin B2 (bleomycin B2), peplomycin (peplomycin), epirubicin (epirubicin), pirarubicin (pirarubicin), zorubicin (zorubicin), mitoxantrone (mitoxantrone), verapamil (verapamil) and thapsigargin (thapsigargin), nucleic acid degrading enzymes and toxins derived from bacteria, plants or animals, which is selected from the group consisting of one or more, but is not limited thereto.

In the present invention, the anti-inflammatory agent may include a steroid formulation that binds to a glucocorticoid receptor to reduce inflammation or swelling, a non-steroidal anti-inflammatory drug (NSAIDs) that alleviates pain in response to cyclooxygenase (COX) which synthesizes inflammation-producing prostaglandins, or an immunospecific anti-inflammatory agent (ImSAIDs) that changes the activation and transport of inflammatory cells, but is not limited thereto.

In the present invention, the immune disease treatment agent may include azathioprine, chlorambucil, cyclophosphamide, cyclosporine, mycophenolate, azathioprine (azathioprine), or methotrexate (methotrexate), but is not limited thereto.

In the present invention, in some cases, the drug may include one or more nucleophilic groups selected from the group consisting of amine, thiol, hydroxyl, hydrazide, oxime, hydrazine, thiosemicarbazone, hydrazine carboxylate, and arylhydrazide groups that can react to form covalent bonds with electrophilic groups on the linker and linker reagent, but is not limited thereto.

### Chimeric Antigen Receptor (CAR)

According to another embodiment of the present invention, the present invention relates to a chimeric antigen receptor (chimeric antigen receptor; CAR) comprising an antigen-binding fragment provided by the present invention.

In the present specification, the term "chimeric antigen receptor" or "CAR" means an engineered receptor comprising an extracellular antigen-binding domain and an intracellular signaling domain. The most common type of CAR is a single-chain variable fragment (scFv) derived from a monoclonal antibody fused to the transmembrane and intracellular domain of a T cell co-receptor such as a CD3ζ chain, but the present invention described herein is not limited to these domains.

As used in the present specification, "chimeric antigen receptor" or "CAR" means any receptor engineered to express any intracellular signaling molecule and an extracellular antigen-binding domain fused or linked to them.

In the present invention, the binding domain may comprise an antigen-binding fragment provided by the present invention, preferably scFv.

The chimeric antigen receptor of the present invention may further comprise at least one of a hinge region (or spacer) and a signaling domain.

In the present invention, the hinge region is a part connecting the antigen-binding domain and the transmembrane domain and is also called a "spacer", and has the purpose of extending the antigen-binding domain from a T cell membrane or NK cell membrane.

In the present invention, the hinge region may be obtained from any suitable sequence from any genus including human or a part thereof, or commonly used in the art such as all or part of CD8, D28, 4-1BB, OX40, CD3 zeta (ζ) chain, T cell receptor α or β chain, CD28, CD3ε, CD45, CD4, CD5, CD8, CD9, CD16, CD22, CD33, CD37, CD64, CD80, CD86, CD134, CD137, ICOS, CD154, functional derivatives thereof or combinations thereof, which may include a hinge region of a human protein, but is not limited thereto.

Furthermore, in the present invention, the hinge region may include one selected from immunoglobulin (e.g., IgG1, IgG2, IgG3, IgG4, and IgD) without being limited to immunoglobulin, but is not limited thereto.

In the present invention, the signaling domain means a part of a chimeric antigen receptor found or engineered to be found inside a T cell. In the present invention, the signaling domain may or may not include a transmembrane domain that serves to fix the chimeric antigen receptor to the plasma membrane of a T cell.

In the present invention, the transmembrane domain and the signaling domain may be derived from the same protein (e.g., CD3ζ), or the transmembrane domain and the signaling domain may be derived from different proteins (e.g., the transmembrane domain of CD28 and the intracellular signaling domain of the CD3ζ molecule, or vice versa).

In the present invention, the transmembrane domain comprises a hydrophobic polypeptide spanning a cell membrane. In particular, the transmembrane domain may span from one side of the cell membrane (extracellular) to the other side of the cell membrane (intracellular or cytoplasm).

In the present invention, the transmembrane domain may be in the form of an alpha helix or a beta barrel, or a combination thereof. Furthermore, in the present invention, the transmembrane domain may comprise a multimeric protein comprising multiple transmembrane fragments, each having an alpha-helical, beta sheet, or combinations thereof.

In the present invention, the transmembrane domain may include, for example, all or part of T cell receptor α or β chain, CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof or combinations thereof, but is not limited thereto.

In the present invention, the transmembrane domain, which is artificially designed, may be a polypeptide containing predominantly hydrophobic residues such as leucine and valine. In one embodiment of the present invention, a triplet of phenylalanine, tryptophan and valine may be found at each end of a synthetic membranespanning domain.

In the present invention, at least one costimulatory domain may be further included between the transmembrane domain and the signaling domain. In the present invention, the costimulatory domain is a site where costimulatory signals are transmitted, and CAR-T cells or CAR-NK cells recognizing a specific antigen bound with the antigen-binding domain can induce an immune response, help self-proliferation, and serve to transmit signals to increase the time of persistence in the body.

In the present invention, the costimulatory domain is the 4-1BB (CD137); OX40; CD27; CD28; CD30; CD40; PD-1; CD2; CD7; CD258; natural killer group 2 member C (NKG2C); natural killer group 2 member (NKG2D); B7-H3; CD83; ICAM-1; LFA-1(CD11a/CD18) or a ligand binding to ICOS; active fragments thereof; functional derivatives thereof; or a functional signaling domain derived from a polypeptide comprising a combination thereof, but is not limited thereto.

In the present invention, the signaling domain may comprise a polypeptide that provides activation of immune cells to stimulate or activate at least some aspects of an immune cell signaling pathway.

In the present invention, the signaling domain may comprise a functional signaling domain derived from a polypeptide comprising all or part of CD3 zeta (ζ), common FcR gamma (FcER1G), Fc gamma RIIIa, FcR beta (Fc epsilon RIβ), CD3 gamma, CD3 delta, CD3 epsilon, CD79a, CD79b, DNAX-activated protein 10 (DAP10), DNAX-activated protein 12 (DAP12), active fragments thereof, functional derivatives thereof or combinations thereof, but is not limited thereto, and such signaling domains are known in the art.

Furthermore, the chimeric antigen receptor of the present invention may further comprise a signal peptide (signal peptide).

In the present invention, the "signal peptide" comprises a peptide sequence, which is a kind of peptide of any secreted or transmembrane protein, and directs the transport of the chimeric antigen receptor of the present invention to the cell membrane and cell surface, and can provide correct positioning of the chimeric antigen receptor of the present invention. In particular, in the present invention, the signal peptide supports the chimeric antigen receptor of the present invention on the cell membrane, at which point the extracellular portion of the chimeric antigen receptor is displayed on the cell surface, the transmembrane portion spans the plasma membrane, and the active domain is located in the cytoplasmic portion, or inside the cell.

In the present invention, the signal peptide may be used without limitation as long as it performs the above function. For example, it may be an n-terminal CD8α signal peptide (n-terminal CD8α signal peptide), but is not limited thereto.

According to another embodiment of the present invention, the present invention relates to a nucleic acid molecule encoding the chimeric antigen receptor according to the present invention.

In the present invention, the nucleic acid molecule encoding the chimeric antigen receptor can be easily prepared from the amino acid sequence of the chimeric antigen receptor specified by any conventional method. The base sequence encoding the amino acid sequence can be obtained for the amino acid sequence of each domain from the aforementioned NCBI RefSeq ID or the accession number of GenBank, and the nucleic acid of the present disclosure can be produced using standard molecular biological and/or chemical procedures. For example, a polynucleotide based on a base sequence can be synthesized, and the nucleic acid molecule of the present disclosure can be prepared by combining DNA fragments obtained from a cDNA library using polymerase chain reaction (PCR).

The nucleic acid molecule of the present invention may be part of a gene or an expression or cloning cassette, but is not limited thereto.

According to another embodiment of the present invention, the present invention relates to an expression cassette (cassette) comprising the nucleic acid molecule provided by the present invention.

The expression cassette of the present invention may comprise a promoter capable of controlling the expression of the nucleic acid molecule of the present invention, enhancers, polyadenylation signals, transcription termination factors, or expression control sequences such as an internal ribosome entry site (IRES).

In the present invention, the promoter may include, for example, an SFFV promoter, elongation factor 1α (elongation factor 1a; EF 1a) promoter, or CAG (chicken beta-actin promoter with CMV enhancer) promoter, but is not limited thereto.

The expression cassette of the present invention further comprises a surrogate marker useful for monitoring the expression thereof, such as a gene sequence encoding a cell surface marker (for example, a truncated cell surface marker), which can be used to confirm transduction or manipulation of cells expressing the receptor.

Exemplary marker genes in the present invention may include base sequences encoding truncated epidermal growth factor receptor (truncated epidermal growth factor receptor, EGFRt), prostate-specific membrane antigen (prostate-specific membrane antigen, PSMA), CD34, nerve growth factor receptor (nerve growth factor receptor, NGFR), or modified forms thereof, but is not limited thereto.

Furthermore, the marker gene in the present invention may include a fluorescent protein, such as green fluorescent protein (green fluorescent protein, GFP), enhanced green fluorescent protein (enhanced green fluorescent protein, EGFP), for example, super-fold GFP (super-fold GFP, sfGFP), red fluorescent protein (red fluorescent protein, RFP), such as tdTomato, mCherry, mStrawberry, AsRed2, DsRed or DsRed2, cyan fluorescent protein (cyan fluorescent protein, CFP), blue green fluorescent protein (blue green fluorescent protein, BFP), enhanced blue fluorescent protein (enhanced blue fluorescent protein, EBFP), and yellow fluorescent protein (yellow fluorescent protein, YFP), and base sequences encoding species variants, monomeric variants, and codon-optimized and/or enhanced variants of fluorescent proteins, but is not limited thereto.

Furthermore, the marker gene in the present invention may be an enzyme, such as luciferase, lacZ gene derived from E. coli, alkaline phosphatase, secreted embryonic alkaline phosphatase (secreted embryonic alkaline phosphatase, SEAP), chloramphenicol acetyl transferase (chloramphenicol acetyl transferase, CAT), or include them. Exemplary luminescent reporter genes may include base sequences encoding luciferase (luciferase, luc), β-galactosidase, chloramphenicol acetyltransferase (CAT), β-glucuronidase (β-glucuronidase, GUS), or variants thereof, but is not limited thereto.

Furthermore, the marker gene in the present invention may be a gene encoding a polypeptide that confers resistance to an exogenous agent or drug, such as, puromycin resistance gene, hygromycin resistance gene, blasticidin resistance gene, neomycin resistance gene, geneticin resistance gene, or zeocin resistance gene, or modified forms thereof, but is not limited thereto.

Furthermore, in the present invention, one end of the marker gene and the polynucleotide of the present invention may be connected by a polynucleotide encoding a cleavable peptide.

In the present invention, the cleavable peptide corresponds to a self-cleaving site, and may be, for example, P2A, F2A, E2A, T2A peptide, or a combination thereof, but is not limited thereto.

According to another embodiment of the present invention, the present invention relates to a recombinant expression vector comprising the expression cassette provided by the present invention.

In the present invention, the description regarding the recombinant expression vector overlaps with that described in the antibody or antigen-binding fragment, and therefore the description is omitted hereinafter.

According to another embodiment of the present invention, the present invention relates to an immune effector cell transduced with the recombinant expression vector provided by the present invention.

In the present invention, the immune effector cell may be an allogeneic or autologous immune cell. Furthermore, in the present invention, the immune effector cell may be derived from, for example, T cells, dendritic cells, killer dendritic cells, mast cells, NK cells, NKT cells, macrophages, monocytes, B cells, and stem cells, but is not limited thereto.

In the present specification, the term "natural killer cells (Natural Killer Cells; NK cells)" is defined as large granular lymphocytes (LGL), and constitutes three types of cells differentiated from common lymphoid progenitor-derived B and T lymphocytes. NK cells are known to differentiate and mature in the bone marrow, lymph nodes, spleen, tonsils, and thymus and enter the circulation. In the present invention, the NK cells may include any type of NK cell without limitation, such as cultured NK cells, for example, primary NK cells (primary NK cells), NK cells from cultured NK cell lines, or NK cells obtained from mammals, but is not limited thereto. When NK cells are obtained from mammals, NK cells can be obtained from a plurality of sources including, but not limited to, blood, bone marrow, lymph nodes, thymus, or other tissue or body fluid. NK cells may be enriched or purified. NK cells may preferably be human NK cells (for example, can be isolated from humans). NK cell lines are available from, for example, ATCC (American Type Culture Collection), and include, for example, NK-92 cells (ATCC CRL-2407), NK92MI cells (ATCC CRL-2408), or derivatives thereof.

According to another embodiment of the present invention, the present invention relates to a polypeptide expressed from the immune effector cell provided by the present invention.

### Therapeutic Uses

According to another embodiment of the present invention, the present invention relates to an antibody or antigen-binding fragment provided by the present invention; or a pharmaceutical composition for the prevention, improvement or treatment of MYCT1-related diseases comprising the antibody or antigen-binding fragment.

The antibody or antigen-binding fragment of the present invention exhibits excellent affinity and binding force to MYCT1. Specifically, by specifically binding to tumor endothelial cells expressing MYCT1 and reprogramming the tumor microenvironment (TME), it can be usefully applied to the treatment of diseases related to MYCT1 overexpression, for example, cancer treatment, particularly the treatment of solid tumors.

The disease related to MYCT1 may be a disease related to the expression or overexpression of MYCT1, for example, it may be cancer. Accordingly, the MYCT1-related disease is preferably cancer or a tumor, but is not limited thereto.

In the present specification, the term "cancer" represents or refers to a physiological condition characterized by uncontrolled cell growth typically in a mammal.

The cancer or carcinoma that can be treated with the composition of the present invention is not particularly limited, and includes both solid tumors and hematological malignancies. Examples of such cancers include skin cancers such as melanoma, liver cancer, hepatocellular carcinoma, hepatic carcinoma, gastric cancer, breast cancer, lung cancer, ovarian cancer, bronchial cancer, nasopharyngeal cancer, laryngeal cancer, pancreatic cancer, bladder cancer, colorectal cancer, colon cancer, pancreatic cancer, cervical cancer, brain cancer, prostate cancer, bone cancer, skin cancer, thyroid cancer, parathyroid cancer, kidney cancer, esophageal cancer, biliary cancer, testicular cancer, rectal cancer, head and neck cancer, cervical cancer, ureteral cancer, osteosarcoma, neuroblastoma, fibrosarcoma, rhabdomyosarcoma, astrocytoma, neuroblastoma, and glioma, but is not limited thereto.

More preferably, the cancer is characterized by the expression of MYCT1 protein and may be breast cancer, pancreatic cancer, prostate cancer, lung cancer, thyroid cancer, gastric cancer, ovarian cancer, colorectal cancer, liver cancer, gallbladder cancer, kidney cancer, cervical cancer, or bladder cancer, but is not limited thereto. The cancer may be a primary cancer or a metastatic cancer.

In the present invention, the MYCT1-related disease may be an angiogenesis-related disease. The angiogenesis-related disease is rheumatoid arthritis, osteoarthritis, septic arthritis, psoriasis, corneal ulcer, agerelated macular degeneration, diabetic retinopathy, proliferative vitreoretinopathy, immature retinopathy, ophthalmic inflammation, keratoconus, Sjogren's syndrome, myopia, ophthalmic tumors, corneal transplant rejection, abnormal wound healing, bone disease, proteinuria, abdominal aortic aneurysm disease, degenerative cartilage loss due to traumatic joint injury, demyelinating disease of the nervous system, cirrhosis, glomerulonephritis, immature rupture of the fetal membrane, inflammatory bowel disease, periodontal disease, atherosclerosis, restenosis, central nervous system inflammatory disease, Alzheimer's disease, and skin aging, which may be selected from one or more of the group consisting thereof, but is not limited thereto.

In the specification, the term "angiogenesis" is used to include all aspects of the maintenance and development of blood vessels. Accordingly, angiogenesis includes the maintenance and control of existing vascular structures and microvasculature, as well as the formation of new capillaries leading to neovascularization. Angiogenesis is a complex process that includes a series of sequential steps comprising endothelial cell-mediated degradation of the vascular basement membrane and interstitial matrix, migration of endothelial cells, proliferation of endothelial cells, and formation of capillary loops by endothelial cells. Angiogenesis includes growth and/or development of new blood vessels (also referred to as neovascularization), expansion of microvasculature, excessive or prolonged blood vessel growth, and maintenance of existing vascular structures. MYCT1 is known to be involved in the regulation of angiogenesis (see non-patent literature).

In the present specification, the term "angiogenesis-related disease" is used to mean a specific pathological process in humans in which angiogenesis is abnormally prolonged. This further includes vascular angiogenic diseases and conditions such as diseases and disorders related to, caused by or associated with angiogenesis. Non-limiting examples of such diseases include various forms of cancer and metastasis characterized by angiogenesis/neovascularization. The antibodies and antigen-binding fragments thereof disclosed herein can be used to treat angiogenesis-related diseases by binding to MYCT1 and inhibiting angiogenesis.

In the present invention, the term "prevention" may be included without limitation if it is any act capable of blocking, suppressing, or delaying symptoms caused by MYCT1-related diseases using the composition of the present invention.

In the present invention, the terms "improvement" and "treatment" may be included without limitation if it is any act that enables or benefits the symptoms caused by MYCT1-related diseases to improve using the composition of the present invention.

The pharmaceutical composition of the present invention may be formulated in the form of an oral formulation such as powders, granules, capsules, tablets, aqueous suspensions, an external formulation, suppositories, and sterile injection solutions according to conventional methods, but is not limited thereto. Preferably, the pharmaceutical composition may be formulated for intratracheal administration or inhalation administration; or may be formulated for use as an injection, but is not limited thereto.

The pharmaceutical composition of the present invention may comprise a pharmaceutically acceptable carrier. The pharmaceutically acceptable carrier may include binders, glidants, disintegrants, excipients, solubilizers, dispersants, stabilizers, suspending agents, colorants, flavoring agents, etc. for oral administration, and in the case of injections, buffers, preservatives, pain-relieving agents, solubilizers, isotonic agents, stabilizing agents, etc. may be mixed and used, and in the case of local administration, bases, excipients, lubricants, preservatives, etc. may be used. The formulation of the pharmaceutical composition of the present invention may be prepared in various ways by mixing with the pharmaceutically acceptable carrier as described above. For example, for oral administration, it can be prepared in the form of tablets, troches, capsules, Elixir, suspension, syrup, wafers, etc., and in the case of injections, it can be prepared in the form of unit dose ampules or multiple dose forms. In addition, it can be formulated as a solution, suspension, tablet, capsule, sustained-release formulation, etc.

Examples of carriers, excipients and diluents suitable for the formulation of the present invention are lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxy benzoate, propylhydroxybenzoate, talc, magnesium stearate, or mineral oil, etc., which may be used. Additionally, fillers, anti-caking agents, lubricants, wetting agents, flavoring agents, emulsifying agents, preservatives, etc. may be further included.

The administration route of the pharmaceutical composition of the present invention is not limited to, but includes oral, intravenous, intramuscular, intraarterial, intrabone, intrathecal, intracardiac, transdermal, subcutaneous, intraperitoneal, intranasal, enteral, topical, sublingual or rectal. It may be administered orally or parenterally, and is preferably orally administered, but is not limited thereto.

The non-parenteral administration of the present invention includes subcutaneous, intradermal, intravenous, intramuscular, intra-articular, intra-synovial, intrasternal, intrathecal, intralesional, and intracranial injection or infusion techniques. The pharmaceutical composition of the present invention may also be administered in the form of a suppository for rectal administration.

The pharmaceutical composition of the present invention may vary depending on various factors including the activity of the specific compound used, age, body weight, general health, gender, diet, administration time, administration route, excretion rate, drug combination, and the severity of the specific disease to be prevented or treated, and the dosage of the pharmaceutical composition may vary depending on the patient's condition, body weight, severity of disease, form of medication, administration route, and duration, but can be appropriately selected by a person skilled in the art, and can be administered at 0.0001 to 50 mg/kg or 0.001 to 50 mg/kg per day. The administration may be once per day or divided into multiple times. The dosage does not limit the scope of the present invention in any way. The pharmaceutical composition according to the present invention can be formulated as pills, sugar-coated pills, capsules, liquid solutions, gels, syrups, slurries, suspensions.

According to another specific example of the present invention, the present invention relates to a method for preventing, improving, or treating MYCT1-related diseases, comprising the step of administering to a subject a therapeutically effective amount of an antibody or antigen-binding fragment provided by the present invention; or a bispecific or multispecific antibody comprising the antibody or antigen-binding fragment.

In the present invention, the subject is a subject in which a MYCT1-related disease (particularly a disease caused by overexpression of MYCT1) has occurred or is suspected to occur, and the subject suspected of disease onset includes humans, primates, cattle, horses, sheep, pigs, chickens, turkeys, quails, cats, dogs, mice, rats, rabbits, or guinea pigs in which the disease may have occurred or occur, but the subject capable of treatment with the effective substance provided by the present invention is included without limitation.

In the present specification, the term "pharmaceutically effective amount" is an amount sufficient to stop or alleviate the physiological effects caused by MYCT1-related diseases in a subject or patient. An appropriate effective amount can be determined by the treating physician within the scope of correct medical judgment, and can be administered in one or several doses. However, for the purposes of the present invention, the specific therapeutically effective amount for a particular patient is the type and degree of response to be achieved, whether different formulations are used in some cases, including the type and degree of response to be achieved, a composition comprising a specific active ingredient, the patient's age, body weight, general health status, gender and diet, administration time, administration route, frequency of administration, and the secretion rate of the composition comprising the active ingredient, treatment period, and various factors including drugs used together or used simultaneously with the specific composition, and similar factors well known in the medical field, it is desirable to apply differently.

The total effective amount of the composition of the present invention may be administered to a patient as a single dose, or may be administered by a fractionated treatment protocol (fractionated treatment protocol) in which the patient is treated over a long period of time with multiple doses. The composition of the present invention may vary the content of the active ingredient depending on the severity of the disease. Specifically, a preferred total dose of the composition of the present invention may be about 0.0001 mg to 500 mg per kg of patient body weight per day. However, the dose of the composition is determined by considering various factors such as the administration route of the pharmaceutical composition and the number of treatments, as well as the patient's age, body weight, health status, gender, severity of the disease, diet, and excretion rate, so an effective dosage for the patient is determined, and therefore, when considering this point, those with ordinary knowledge in the field will be able to determine an appropriate effective dosage according to the specific use of the composition of the present invention. The pharmaceutical composition according to the present invention is not particularly limited in its formulation, administration route, and administration method as long as it shows the effect of the present invention.

### Diagnostic Uses

According to another embodiment of the present invention, the present invention relates to a diagnostic composition for MYCT1-related diseases comprising an antibody or antigen-binding fragment provided by the present invention; or the antibody or antigen-binding fragment.

In the present invention, by detecting MYCT1 by the antibody or antigen-binding fragment, it can be used as a diagnostic marker for evaluating the progression of angiogenesis-related diseases, for example, cancer or diseases characterized by neovascularization, and the prognosis before and after treatment.

The antibody or antigen-binding fragment of the present invention can be used in any known assay method such as competitive binding assay, direct and indirect sandwich assay, and immunoprecipitation assay.

The antibody or antigen-binding fragment of the present invention may be labeled with a label that performs the following functions: (i) provides a detectable signal; (ii) reacts with a second label to modify a detectable signal provided by, for example, Fluorescence Resonance Energy Transfer (FRET) of a first or second label; (iii) affects motility by charge, hydrophobicity, shape, or other physical parameters, such as electrophoretic motility, or (iv) provides a capture moiety such as affinity, antibody/antigen or ion complex. Suitable as labels are fluorescent labels, luminescent labels, chromophoric labels, radioactive labels, isotope labels, isobaric labels, enzyme labels, particle labels, especially metal particle labels, magnetic particle labels, macromolecular particle labels, biotin, receptor ligands or cell adhesion proteins or organic small molecules such as binding molecules such as lectins, labels-sequences containing detectable nucleic acids and/or amino acid residues by use of binders, and the like.

In the present invention, the specific type of the label is not particularly limited, but may be, for example, an enzyme using hydrogen peroxide to oxidize a dye precursor, for example, HRP, lactoperoxidase or microperoxidase, biotin/avidin, spin labels, bacteriophage labels, stable free radicals, radioactive isotopes such as 32P, 14C, 125I, 3H, and 131I, fluorophores such as rare earth chelates or fluorescein and derivatives thereof, rhodamine and derivatives thereof, dansyl, umbelliferone, luciferase, such as firefly luciferase and bacterial luciferase (U.S. Patent No. 4,737,456), luciferin, 2,3-dihydropterazine dione, horseradish peroxidase (HRP), alkaline phosphatase, β-galactosidase, glucoamylase, lysozyme, saccharide oxidase, for example, glucose oxidase, galactose oxidase, and glucose-6-phosphate dehydrogenase, heterocyclic oxidase, for example, uric acid oxidase, and xanthine oxidase, but are not limited to these.

According to another embodiment of the present invention, the present invention relates to a diagnostic kit for MYCT1-related diseases comprising the diagnostic composition of the present invention.

In the present invention, the "kit" refers to a tool capable of evaluating the expression level of the biomarker by labeling a probe or antibody that specifically binds to the biomarker component with a detectable label. Regarding the probe or antibody, the detectable substance is not only directly labeled by the reaction with the substrate, but also includes an indirect label conjugated with a colorimetric label by reactivity with other reagents that are directly labeled. It may include a colorimetric substrate solution that will react with the label, a washing solution, and other solutions, and can be manufactured including the reagent components used. In the present invention, the kit may be a kit comprising essential elements required for performing RT-PCR, and in addition to specific primer pairs for marker genes, it may include test tubes, reaction buffers, deoxynucleotides (dNTPs), Taq-polymerase, reverse transcriptase, DNase, RNase inhibitor, sterile water, etc. Furthermore, the kit may be a kit for detecting genes for cancer diagnosis, which comprises essential elements required for performing a DNA chip. The DNA chip kit includes a substrate to which cDNA corresponding to genes or fragments thereof is attached as probes, and the substrate may include cDNA corresponding to quantitative control genes or fragments thereof. The kit of the present invention is not limited if it is known in the art.

In the present invention, the kit may be an RT-PCR kit, a DNA chip kit, an ELISA kit, a protein chip kit, a rapid kit, or an MRM (Multiple reaction monitoring) kit.

The kit of the present invention may further comprise one or more different constituent compositions, solutions, or devices suitable for the analysis method. For example, in the present invention, the kit may further comprise essential elements required for performing reverse transcription polymerase reaction. The reverse transcription polymerase reaction kit comprises a specific primer pair for genes encoding the marker protein. Primers are nucleotides with sequences specific to the nucleic acid sequence of the gene and may have a length of about 7 bp to 50 bp, more preferably about 10 bp to 30 bp in length. It may also include primers specific to the nucleic acid sequence of the control gene. Otherwise, the reverse transcription polymerase reaction kit may include test tubes or other appropriate containers, reaction buffers (with various pH and magnesium concentrations), deoxynucleotides (dNTPs), enzymes such as Taq-polymerase and reverse transcriptase, DNase, RNase inhibitor, DEPC-water, sterile water, etc.

Furthermore, the diagnostic kit of the present invention may comprise essential elements required for performing DNA chips. The DNA chip kit may include a substrate to which cDNA or oligonucleotide corresponding to genes or fragments thereof is attached, and reagents, formulations, enzymes, etc. for preparing fluorescent labeled probes. The substrate may also include cDNA or oligonucleotides corresponding to control genes or fragments thereof.

Furthermore, the diagnostic kit of the present invention may comprise essential elements required for performing ELISA. The ELISA kit includes antibodies specific for the protein. The antibodies are antibodies with high specificity and affinity for the marker protein and little cross-reactivity with other proteins, and are monoclonal antibodies, polyclonal antibodies, or recombinant antibodies. Furthermore, the ELISA kit may include antibodies specific for control proteins. Otherwise, the ELISA kit may include reagents capable of detecting bound antibodies, for example, labeled secondary antibodies, chromophores, enzymes (e.g. conjugated with antibodies) and substrates thereof, or other substances capable of binding to antibodies.

In the present invention, the immobilizer for the antigen-antibody binding reaction may be a nitrocellulose membrane, PVDF membrane, well plate synthesized from polyvinyl resin or polystyrene resin, glass slide glass, etc., but is not limited thereto.

In the present invention, the wash solution preferably comprises a phosphate buffer solution, NaCl, and Tween 20, and more preferably a buffer solution (PBST) composed of 0.02 M phosphate buffer solution, 0.13 M NaCl, and 0.05% Tween 20. The wash solution is added to the immobilizer in an appropriate amount after the antigen-antibody binding reaction and then the secondary antibody is reacted with the antigen-antibody complex, and then washed 3 to 6 times. The reaction stop solution is preferably a sulfuric acid solution (H₂SO₄).

According to another specific example of the present invention, the present invention relates to a method of providing information for diagnosis of MYCT1-related diseases, comprising the step of contacting an antibody or antigen-binding fragment provided by the present invention with a biological sample isolated from a desired subject; or a bispecific or multispecific antibody comprising the antibody or antigen-binding fragment.

In the present invention, the subject is a subject in which a MYCT1-related disease (particularly a disease caused by overexpression of MYCT1) has occurred or is suspected to occur, and the subject suspected of disease onset includes humans, primates, cattle, horses, sheep, pigs, chickens, turkeys, quails, cats, dogs, mice, rats, rabbits, or guinea pigs in which the disease may have occurred or occur, but the subject in which diagnosis of the disease is possible with the effective substance provided by the present invention is included without limitation.

In the present invention, the biological sample may include whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid, but is not limited thereto.

The information providing method of the present invention may include measuring the expression level of MYCT1 protein in the biological sample using an antibody or antigen-binding fragment provided by the present invention.

In the present invention, the measurement of the expression level of the protein may be performed by Western blotting, ELISA (enzyme linked immunosorbent assay), competitive binding assay, or similar methods.

The information providing method of the present invention may include the step of predicting that a MYCT1-related disease has occurred or is likely to occur when the expression level of MYCT1 protein measured for the biological sample is increased compared to a control group. Here, the control group may be the expression level of MYCT1 in a healthy normal control group, but is not limited thereto.

Terms used in the examples are used for illustrative purposes only and should not be construed as limiting. Singular expressions include plural expressions unless the context clearly indicates otherwise. In the present specification, terms such as "comprise" or "have" are intended to specify that the features, numbers, steps, operations, components, parts, or combinations thereof described in the specification exist, and do not preclude the existence or possibility of addition of one or more other features, numbers, steps, operations, components, parts, or combinations thereof in advance.

Unless otherwise defined, all terms used herein, including technical or scientific terms, have the same meaning as generally understood by a person of ordinary skill in the technical field to which the embodiments belong. Terms as defined in commonly used dictionaries should be interpreted as having meanings consistent with their meanings in the context of the related technology, and unless explicitly defined in the present application, are not interpreted in an idealistic or excessively formal sense.

In describing the components of the embodiments, terms such as first, second, A, B, (a), (b), etc. may be used. These terms are only for distinguishing the components from other components, and the nature, sequence, or order of the components is not limited by the terms. When a component is described as being "connected," "coupled," or "linked" to another component, the component may be directly connected or connected to the other component, but it should be understood that another component may be "connected," "coupled," or "linked" between each component.

### Combination Therapy

According to another embodiment of the present invention, the present invention relates to a combination therapy in which a pharmaceutical composition comprising an antibody or antigen-binding fragment provided by the present invention; an immunoconjugate; a chimeric antigen receptor; or an immune effector cell expressing a chimeric antigen receptor as an active ingredient is administered in combination with at least one additional therapeutic agent or therapeutic procedure.

The at least one additional therapeutic agent or therapeutic procedure may be selected from one or more of chemotherapy, targeted anti-cancer therapy, oncolytic drug, cytotoxic agent, immune-based therapy, cytokine, surgical procedure, radiation procedure, vaccine, or cell therapy, but is not limited thereto.

The at least one therapeutic agent may be an immuno-anticancer agent.

The at least one therapeutic agent may be a PD-1 inhibitor, for example, a PD-1 antibody. Specifically, the PD-1 inhibitor may be selected from PDR001, Nivolumab, Pembrolizumab, Pidilizumab, MEDI0680, REGN2810, TSR-042, PF-06801591, or AMP-224, but is not limited thereto.

The at least one therapeutic agent may be a PD-L1 inhibitor, for example, a PD-L1 antibody. Specifically, it may be selected from FAZ053, Atezolizumab, Avelumab, Durvalumab, or BMS-936559, but is not limited thereto.

In the present invention, the "combination" should be understood as representing simultaneous, separate, or sequential administration. When the administration is sequential or separate, the interval of the secondary component administration should be such that the beneficial effects of the combination are not lost.

### Uses for Enhancing Sensitivity to Immuno-Anticancer Agents

According to another embodiment of the present invention, the present invention relates to uses for enhancing the sensitivity of immuno-anticancer agents in various cancer types of the antibody or antigen-binding fragment provided by the present invention.

In the present specification, the term "sensitivity" means whether a specific drug (e.g., an immuno-anticancer agent) is effective against cancer in individual patients.

When mentioning sensitivity in the present specification, the term "resistance" used means that the drug does not respond sensitively and the efficacy of the drug does not act.

For example, the specific drug is mainly an immuno-anticancer agent, and these anti-cancer agents may or may not be effective depending on the type of cancer. Additionally, even in the case of cancer types that are recognized as effective, it is known that there are cases where the effect is shown and cases where the effect is not shown depending on individual patients. Such whether the anti-cancer agent is effective against the cancer of individual patients is called anti-cancer drug sensitivity. Accordingly, if before the start of treatment according to the present invention, it is possible to predict patients expected to benefit (responders) and patients not expected to benefit (non-responders), highly effective and safe chemotherapy can be realized. The present invention provides a method of enhancing sensitivity to immuno-anticancer agents in an individual by administering a composition comprising a therapeutically effective amount of an antibody or antigen-binding fragment of the present invention to a subject with resistance to immuno-anticancer agents. Hereinafter, examples will be described in detail with reference to the attached drawings. However, various changes may be made to the examples, and the scope of the patent application is not limited or restricted by these examples. All changes, equivalents, or substitutes for the examples should be understood to be included in the scope of rights.

Furthermore, in the description with reference to the attached drawings, regardless of the reference numerals, the same components are given the same reference numerals, and redundant descriptions thereof are omitted. In describing the examples, when it is determined that detailed descriptions of related known technologies may unnecessarily obscure the gist of the examples, the detailed descriptions are omitted.

The present invention can make various transformations and can have various embodiments. Hereinafter, specific embodiments will be illustrated in the drawings and described in detail in the detailed description. However, this is not intended to limit the present invention to specific embodiments, and should be understood to include all transformations, equivalents, or substitutes included in the spirit and technical scope of the present invention. In describing the present invention, when it is determined that detailed descriptions of related known technologies may obscure the gist of the present invention, the detailed descriptions are omitted.

### EXAMPLES

### Example 1. Phage Display Library Screening

### 1-1. Antigen Preparation

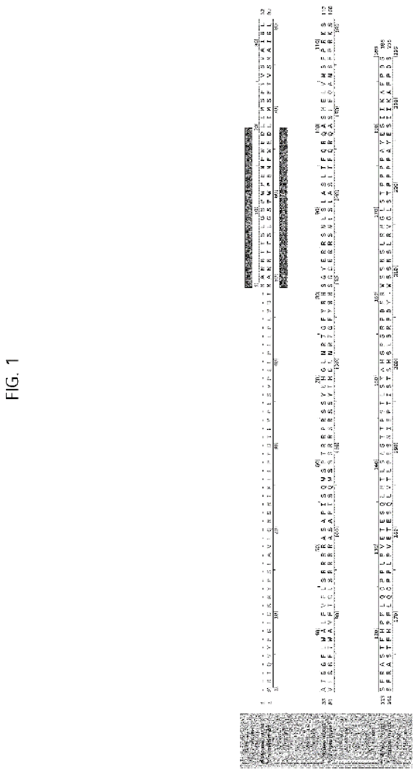

The antigens used in the phage display library screening for production of anti-MYCT1 antibodies were synthesized by WuXi Biologics. For human MYCT1, the antigen included amino acid sequences 1 to 235 (SEQ ID NO: 1), and for mouse MYCT1, included amino acid sequences 1 to 188 (SEQ ID NO: 2) and the overlapping sequence of the mouse transmembrane domain (SEQ ID NO: 3). For human MYCT1, amino acid sequences 49 to 67, and for mouse MYCT1, amino acid sequences 1 to 19 were used, and biotin was conjugated for screening. The results of amino acid sequence comparison between human and mouse MYCT1 are presented in FIG. 1.

### 1-2. Antibody Selection Through Antigen Preparation and Phage Library Screening

Using two types of libraries provided by WuXi Biologics-a human synthetic antibody library (Synthetic Fab) and a human naïve antibody library (Naïve scFv)-three rounds of panning were performed. In each round, enrichment titer was confirmed through polyclonal phage ELISA. As the rounds increased, phage enrichment against the target antigen was confirmed, and titer increased. (Results not shown).

### 1-3. Mono Phage Selection with Binding Specificity to MYCT1

To detect phage binding to MYCT1, monoclonal phage ELISA (Enzyme-Linked Immunosorbent Assay) was performed. Specifically, MYCT1 (positive) and negative antigens were suspended in DPBS and 100 µL/well was added to immuno-plates to coat the antigen. The supernatant was removed and a blocking solution (4% Skim milk) was added for blocking. The supernatant was removed, and 100 µL of poly-phage (or mono-phage) was added to the antigen-coated immuno-plates to induce binding. The supernatant was removed and 200 µL/well PBST was added three times for washing. 100 µL/well anti-M13-HRP was added to the immuno-plate and reacted. The supernatant was removed and 200 µL/well PBST was added three times for washing. 100 µL/well TMB peroxidase substrate (or o-Phenylenediamine dihydrochloride) was added to the immuno-plate and reacted. After 10 minutes, 50 µL/well stop solution (Sulfuric acid added) was added to stop the reaction. Absorbance was measured at wavelength 450 nm (or 490 nm) using an ELISA device (plate reader). A total of 87 antibodies from the scFv naïve library and 29 antibodies from the Fab synthetic library were selected.

### 1-4. Phage Selection with Binding Specificity to Cells Expressing MYCT1

The cells used for cell binding force analysis were four types: WT MCEC (Mouse cardiac endothelial cell line), mMOE-FLAG MCEC (mouseMYCT1-FLAG tagged overexpressed Mouse cardiac endothelial cell line), WT iMLEC (Mouse lung endothelial cell line), and iMLEC mKO hMOE long FLAG (MYCT1 knockout mouse lung endothelial cell line [CSII-MCS-IRES2-bsr.FLAG-humanMYCT1]). 200,000 cells were placed in a 96-well plate and phage was serially diluted twofold in 1% BSA/1X PBS, reacted at 4°C for 1 hour, and antic-Myc-RPE antibody was diluted 1:800 in 1% BSA/1X PBS and dark-reacted at 4°C for 30 minutes. After resuspending in 100 µL PBS, FACS analysis was performed to select 23 types of antibodies binding to human overexpressing cell lines.

The CDR sequences of antibodies selected in Example 1-3 are shown in Tables 1 to 8 below, and the sequences of the heavy chain variable region and light chain variable region are summarized as Tables 9 to 22 below.

**Table 1[JY1].**

| HCDR1 | SEQ ID NO | HCDR1 | SEQ ID NO |
|---|---|---|---|
| GDSVSSNNVAWD | 4 | GFTFSSSAMH | 24 |
| GDSVSNNNGAWN | 5 | GFTFSSYAMH | 25 |
| GDSVSSNSAAWN | 6 | GFTFSSYAMS | 26 |
| GDSVSSNSVTWH | 7 | GFTFSSYGMH | 27 |
| GDSVTSNSAAWN | 8 | GFTFSSYWMN | 28 |
| GFPFSRNAMS | 9 | GFTFSTYAMS | 29 |
| GFPFSRYAMS | 10 | GFTFSTYGMH | 30 |
| GFPFSRYGMS | 11 | GGTFSRYGIS | 31 |
| GFPFSSNAMS | 12 | GGTFSSYAIS | 32 |
| GFSFSNYGIH | 13 | GYDFSTNWIA | 33 |
| GFSFSSYSMN | 14 | GYIFTSYSMH | 34 |
| GFTFRTYVMH | 15 | GYRFTNYWIG | 35 |
| GFTFSNYWMH | 16 | GYTFGSHYMH | 36 |
| GFTFSNYWMS | 17 | GYTFTGYGIH | 37 |
| GFTFSRNAMS | 18 | GYTFTKYYMH | 38 |
| GFTFSRNYMS | 19 | GYTFTNYAIH | 39 |
| GFTFSRYAMS | 20 | GYTFTNYYMH | 40 |
| GFTFSSHAMS | 21 | GYTFTSYGIH | 41 |
| GFTFSSHYMS | 22 | GYTFTSYYMH | 42 |
| GFTFSSNAMS | 23 | RFTFNSYAMH | 43 |

**Table 2.**

| HCDR2 | SEQ ID NO | HCDR2 | SEQ ID NO |
|---|---|---|---|
| AISGSGGSTYYADSVKG | 44 | RTYYRSKWYNDYAVSVKS | 65 |
| AISGSGSSTYYADSVKG | 45 | RTYYRSKWYTDYAVSVKG | 66 |
| AISGSSSEKYYADSVKG | 46 | RTYYRSQWHHEYAVSVK | 67 |
| AISSDGSNKYYADSVKG | 47 | RTYYRSRWYNDYAVSVKS | 68 |
| FIRYDGNSKYYADSVKG | 48 | SISGSGGSTYYADSVKG | 69 |
| GIIPIFGTANYAQKFQG | 49 | SISQSGSTIYYADSVKG | 70 |
| GIIPILGTANYAQKFQG | 50 | TISGSGGSTYYADSVKG | 71 |
| GISYDGRNKYYADSVKG | 51 | VIHPGGSEIKSSPSFQG | 72 |
| IINPSGGSANY AQKFQ | 52 | VISGSGGNTYYADSVKG | 73 |
| IINPSGGSANY AQKFQG | 53 | VISGSGGSTYYADSVKG | 74 |
| IINPSGGSTSFAQKFQG | 54 | VISPNGDRTRYAQKFKGR | 75 |
| IINPSGGSTSYAQKFKG | 55 | VISYDGRNKYYADSVKG | 76 |
| IINPSGGSTSY AQKFQG | 56 | VISYDGSNKYYADSVKG | 77 |
| IINPSGGSTSYAQKFQGR | 57 | VISYDVSNKYYADSVKG | 78 |
| IINPSGGSTTYAQKFQG | 58 | VTSYDGRYRYYADSVRG | 79 |
| IINTRAGNTAYAQRFQG | 59 | VVSSDGNKKYYADSVKG | 80 |
| IISASSGNTGYAQKFQG | 60 | WINGDNGNTKHSQKFQG | 81 |
| IIYPGDSKTRYSPSFQG | 61 | WINPGSGGTNY AQKFQG | 82 |
| NIKQDASEKYYVDSVKG | 62 | WINPSSGNTKY AQKFQG | 83 |
| NIKQDGSGQYYMDSVKG | 63 | WINPYTGNTNY AQKFQG | 84 |
| RAYYRSKWYYNYAVSVKS | 64 | YVSRDSRTIHYADSVKG | 85 |

**Table 3.**

| HCDR3 | SEQ ID NO | HCDR3 | SEQ ID NO |
|---|---|---|---|
| AELDY | 86 | GPSIYKYGMDV | 137 |
| AHSSSLDI | 87 | GRRYRAFDV | 138 |
| AQSSSWLYGMDV | 88 | GRYYGDI | 139 |
| ARYPGYFDV | 89 | GRYYRGFDY | 140 |
| ASGGVPIPYYSYYGMDV | 90 | GSRRVAFDY | 141 |
| AVYYDSSGGMDV | 91 | GSYSRFDY | 142 |
| DAGTTGMDV | 92 | GWAMDA | 143 |
| DASYGMDV | 93 | GWAMDV | 144 |
| DFDI | 94 | GYSASFDY | 145 |
| DFDY | 95 | HHGMDV | 146 |
| DFDL | 96 | IQHYYDSSGRFRYYYYGMDV | 147 |
| DGIVGGASGARYYYYYMDV | 97 | KARRPGGHFDY | 148 |
| DGSSGNPPVYGMDV | 98 | LGGGSGNYGMDV | 149 |
| DKANGMDV | 99 | LLRTGYFDL | 150 |
| DKSWFGELRKYYYYGMDV | 100 | LPGYGAYERIDPNDDY | 151 |
| DLAAARSGHGRRNYYYGMDV | 101 | LSSGWYMAFDY | 152 |
| DLHEAYGMDV | 102 | MGPGEYYGASNDYYYNGMDV | 153 |
| DLVGYIDY | 103 | MVRGVYGMDV | 154 |
| DLVYGSGSYGMDV | 104 | PHPVRGVIGEYFQH | 155 |
| DMDV | 105 | RAFDI | 156 |
| DNGADTAIDY | 106 | RNTDY | 157 |
| DPLSVAGPFDY | 107 | RVLDY | 158 |
| DRAPFSYYYDSSGLFQH | 108 | RWGDY | 159 |
| DRGNGMDV | 109 | SLRSGFDS | 160 |
| DRGYGMDV | 110 | SLRSSWLFDY | 161 |
| DRKWGYFDPYYYYGMDV | 111 | SRRSGFDY | 162 |
| DRRSSSLDY | 112 | STAAADMDV | 163 |
| DVAAAGDY | 113 | TKGQQLAFDY | 164 |
| DYHYYMDV | 114 | TQRSGYFDY | 165 |
| DYYGSLDV | 115 | TRPGIGLDP | 166 |
| EEISVVRGVKIDY | 116 | TVAGTFDY | 167 |
| EFRGRGMDVW | 117 | VGGGRDSTDY | 168 |
| EGIAARHYYGMDV | 118 | VGGGRDSTDY | 169 |
| EGSSYGMDV | 119 | VGRRRSNYYYYMDV | 170 |
| EMAESTWAFYAYYYDMDV | 120 | VGRVFDI | 171 |
| EPQNSGSFEY | 121 | VISRTNGMDV | 172 |
| EPRDCSGGSCGGMDV | 122 | VLRPGYFDY | 173 |
| ESKQQLVRGYYYGMDV | 123 | VLRRGYFDV | 174 |
| EVAAAPRYGMDV | 124 | VLRYGYFDY | 175 |
| EWRGYGMDV | 125 | VPGYVPLDV | 176 |
| EYGSGSRGYGMDV | 126 | VRRPGAFDY | 177 |
| FGSGAYGNGAFDY | 127 | VRRYGYFDY | 178 |
| GARNSDAFDI | 128 | VRSSGLDY | 179 |
| GGAGSGHMDV | 129 | VRYAGYFDV | 180 |
| GGAGSGHMNV | 130 | VRYRGYFDY | 181 |
| GGGIAAFY | 131 | VRYSGYFDY | 182 |
| GGIAAAGTRYNWFDP | 132 | VSGPSSGWYRGDFDY | 183 |
| GGPKLRGAVGVMDV | 133 | VSRRSPFDP | 184 |
| GGYGGKSRGTYYYYGMDV | 134 | WSYVFYI | 185 |
| GIDY | 135 | YYDYRMDV | 186 |
| GKVDY | 136 | VLRRGYFDY | 187 |

**Table 4.**

| LCDR1 | SEQ ID NO | LCDR1 | SEQ ID NO |
|---|---|---|---|
| ASSTGAVTNGYYPN | 188 | SGSDSNIGRYFVS | 227 |
| ASSTGAVTSGYYPN | 189 | SGSNSNIGSYSVN | 228 |
| CGSSSNIGSNPVN | 190 | SGSRSNIGNYLVH | 229 |
| GGNNIGSKNVH | 191 | SGSRSNIGRYVVN | 230 |
| GSSSNIGSNKVN | 192 | SGSSFNIGAGYDVH | 231 |
| KSSQSLLYSSNNKTYFA | 193 | SGSSNIGSNTVH | 232 |
| KSSQSVLYICSNKNFLA | 194 | SGSSSNIGKNYVS | 233 |
| KSSQSVLYRANNKNYLA | 195 | SGSSSNIGKYPVN | 234 |
| KSSQSVLYSSNNKNDLA | 196 | SGSSSNIGSFHVN | 235 |
| KSSQSVLYSSNNKNYLA | 197 | SGSSSNIGSHYVS | 236 |
| RARQGISNYLA | 198 | SGSSSNIGSNPVN | 237 |
| RASQDISNWLA | 199 | SGSSSNIGSNTVH | 238 |
| RASQGISSWLA | 200 | SGSSSNIGSNTVN | 239 |
| RASQSIRNFLN | 201 | SGSSSNIGSNYVH | 240 |
| RASQSISNDLA | 202 | SGSSSNIGSYTVN | 241 |
| RASQSISSWVA | 203 | SGSSSNIGTNSVN | 242 |
| RASQSISSYLA | 204 | SGSSSNIGTYTVN | 243 |
| RASQSLSSWLA | 205 | SGTRPNIGGYTVN | 244 |
| RASQSVGNYLA | 206 | SGTSSDVGGYNYVS | 245 |
| RASQSVGSNLA | 207 | TGASGDVGGYNYVS | 246 |
| RASQSVSNYLA | 208 | TGGSSNIGAGYDVH | 247 |
| RASQSVSSNLA | 209 | TGNSNNVGNQGAA | 248 |
| RASQSVSSYLA | 210 | TGNSNNVGNYGAA | 249 |
| RASQSVSTNLA | 211 | TGRSSNLGAGHDVH | 250 |
| RASQSVSTYLA | 212 | TGSSSNIGAGYDVH | 251 |
| RFSQSLVHLDGNTYLN | 213 | TGSSSNIGAGYNVY | 252 |
| RSSQSLAHSDGNTYLN | 214 | TGSSSNIGANYGVH | 253 |
| RSSQSLISGGGDTYLH | 215 | TGTSRDVGGYNYVS | 254 |
| RSSQSLLHSNGKNYLD | 216 | TGTSSDIGAYKFVS | 255 |
| RSSQSLLHSNGNNYLD | 217 | TGTSSDIGGYNFVS | 256 |
| RSSQSLLHSNGYNYLD | 218 | TGTSSDVGGDKYVF | 257 |
| RSSQSLLHTDGNTYLN | 219 | TGTSSDVGGDKYVS | 258 |
| RSSQSLLYTDGNTYLN | 220 | TGTSSDVGGYDYVS | 259 |
| RSSQSLVHRDGNTYLN | 221 | TGTSSDVGGYNFVS | 260 |
| RSSQSLVHSDGNTYLN | 222 | TGTSSDVGGYNSVA | 261 |
| RSSQSLVHSDGNTYLN | 223 | TGTSSDVGGYNYVS | 262 |
| RSSQSLVHSNGKTYLD | 224 | TGTSSDVGNYNYVS | 263 |
| SGDSNNIGKYGAA | 225 | TGTSSDVGSYNYIS | 264 |
| SGGRSNIGSNIVN | 226 | | |

**Table 5.**

| LCDR2 | SEQ ID NO | LCDR2 | SEQ ID NO |
|---|---|---|---|
| AASNRAT | 265 | GNSNRPS | 293 |
| AASSLRS | 266 | GNTNRPS | 294 |
| AASSRAT | 267 | GVSERPS | 295 |
| DASNRAT | 268 | KASSLES | 296 |
| DASTRAT | 269 | KASSLQS | 297 |
| DNDKRPS | 270 | KASTLQS | 298 |
| DNDQRPS | 271 | KVSKRVS | 299 |
| DNNKRPS | 272 | KVSNRAS | 300 |
| DTDKRPS | 273 | KVSNRDS | 301 |
| DVNDRPS | 274 | LGSNRAS | 302 |
| DVNKRPS | 275 | NNNQRPS | 303 |
| DVRNRPS | 276 | NNSQRP | 304 |
| DVSKRPS | 277 | RDINRPS | 305 |
| DVSNRPS | 278 | RDSQRPS | 306 |
| DVTNRPS | 279 | RNNNRPS | 307 |
| ENNKRPS | 280 | RSNQRSS | 308 |
| EVNNRPS | 281 | SDNQRPS | 309 |
| EVSKRPS | 282 | SNNQRPS | 310 |
| EVSNRPS | 283 | SNNQRSS | 311 |
| EVSRRPS | 284 | SNSQRPS | 312 |
| EVTKRPS | 285 | SNTQRPS | 313 |
| GASNRAT | 286 | STSNKHS | 314 |
| GASSRAT | 287 | STSNKHSW | 315 |
| GASTLQS | 288 | TDNQRPS | 316 |
| GASTRAT | 289 | TNNKRPS | 317 |
| GKNQRPS | 290 | TNNQRPA | 318 |
| GNNNRPS | 291 | WASTRES | 319 |
| GNNRRPS | 292 | WAYTRES | 320 |

**Table 6.**

| LCDR3 | SEQ ID NO | LCDR3 | SEQ ID NO |
|---|---|---|---|
| AAWDDRLNGVV | 321 | QQYNSFPPT | 367 |
| AAWDDSLNAVV | 322 | QQYNSFYT | 368 |
| AAWDDSLNGPR | 323 | QQYNTSPT | 369 |
| AAWDDSLNGPV | 324 | QQYSKWPIT | 370 |
| AAWDDSLNGVV | 325 | QQYSNSPLT | 371 |
| AAWDDSLNVV | 326 | QQYSSSPVT | 372 |
| AAWDSSLGVV | 327 | QQYYSPLLT | 373 |
| AAWDSSLRAWV | 328 | QQYYSSPIT | 374 |
| AAYDTSLGVV | 329 | QQYYSSPLT | 375 |
| ASWDDSLKGPV | 330 | QQYYSSPPT | 376 |
| ASWDDSLNGPA | 331 | QQYYSSPVT | 377 |
| ASWDDSLSVV | 332 | QQYYSTPLT | 378 |
| ATWDANLSGVV | 333 | QQYYSTPT | 379 |
| ATWDDSLNGLV | 334 | QQYYTTPPT | 380 |
| ATWDDSLNGVV | 335 | QSFDSSLSAVV | 381 |
| ATWDGSLNGVV | 336 | QSYDNSLSAHVV | 382 |
| ATWDSSQSGGI | 337 | QSYDSSLKLV | 383 |
| GTWDSSLSAVV | 338 | QSYDSSLSGPSVV | 384 |
| KQYSKWPLT | 339 | QSYDSSLSGPVV | 385 |
| LLYYGGAQV | 340 | QSYDSSLSGYVV | 386 |
| LQGTHRPPT | 341 | QSYDSSLSVV | 387 |
| LTWDSNLSAAV | 342 | QSYDSSPHVV | 388 |
| MQALQTPLT | 343 | QVWDSSTFV | 389 |
| MQALQTPWT | 344 | RQATQFPLT | 390 |
| MQGSHWPPIT | 345 | SAWDSSLSAVV | 391 |
| MQGTHWPPIT | 346 | SSFAGTSYV | 392 |
| MQGTHWPWT | 347 | SSFTRSRTWV | 393 |
| MQGTLWPPIT | 348 | SSFTSSRTLV | 394 |
| MQGTQRPPT | 349 | SSFTTSRTLV | 395 |
| QHYGKSPIT | 350 | SSYAGNKLV | 396 |
| QHYYTSPLT | 351 | SSYAGSHVV | 397 |
| QKYNNAPLA | 352 | SSYAGSNNWVV | 398 |
| QQADSAPLT | 353 | SSYRRSNIVV | 399 |
| QQANSAPLT | 354 | SSYRRSSVLV | 400 |
| QQLNSAPIT | 355 | SSYTRSTTLV | 401 |
| QQRGSWPLT | 356 | SSYTSRRTPYV | 402 |
| QQRSNWPPT | 357 | SSYTSSRTLV | 403 |
| QQSHSVPYT | 358 | SSYTSSSTGV | 404 |
| QQYGNWPLT | 359 | SSYTSSSTLV | 405 |
| QQYGRSPPT | 360 | SSYTSSSTPVV | 406 |
| QQYGSSPIT | 361 | SSYTSSSTVGVV | 407 |
| QQYGSSPLT | 362 | SSYTSSSTVV | 408 |
| QQYGTSPLT | 363 | SSYTSSTTLV | 409 |
| QQYNNWPLT | 364 | STWDSNLRGYV | 410 |
| QQYNRSPIT | 365 | TSFRSSNTLV | 411 |
| QQYNRSPLT | 366 | | |

**Table 7.**

| No. | Type | Source | Clone | HCDR1 SEQ ID NO | HCDR2 SEQ ID NO | HCDR3 SEQ ID NO | LCDR1 SEQ ID NO | LCDR2 SEQ ID NO | LCDR3 SEQ ID NO |
|---|---|---|---|---|---|---|---|---|---|
| 1 | scFv | Human Naive | P1R21-A1 | 13 | 76 | 105 | 222 | 301 | 346 |
| 2 | scFv | Human Naive | P1R21-B3 | 40 | 53 | 160 | 262 | 278 | 404 |
| 3 | scFv | Human Naive | P1R21-E3 | 42 | 56 | 113 | 262 | 283 | 407 |
| 4 | scFv | Human Naive | P1R21-E4 | 26 | 44 | 147 | 201 | 266 | 358 |
| 5 | scFv | Human Naive | P1R21-F4 | 25 | 77 | 110 | 251 | 293 | 388 |
| 6 | scFv | Human Naive | P1R21-H11 | 6 | 68 | 143 | 230 | 304 | 325 |
| 7 | scFv | Human Naive | P1R22-G9 | 25 | 77 | 109 | 252 | 293 | 382 |
| 8 | scFv | Human Naive | P1R31-A10 | 42 | 56 | 112 | 262 | 283 | 409 |
| 9 | scFv | Human Naive | P1R31-B8 | 40 | 52 | 160 | 245 | 279 | 401 |
| 10 | scFv | Human Naive | P1R31-C8 | 42 | 56 | 98 | 233 | 272 | 338 |
| 11 | scFv | Human Naive | P1R31-E1 | 42 | 56 | 95 | 237 | 310 | 324 |
| 12 | scFv | Human Naive | P1R31-E10 | 25 | 77 | 114 | 246 | 275 | 403 |
| 13 | scFv | Human Naive | P1R31-F7 | 25 | 77 | 126 | 251 | 293 | 386 |
| 14 | scFv | Human Naive | P1R31-G8 | 30 | 48 | 130 | 194 | 320 | 378 |
| 15 | scFv | Human Naive | P1R31-H11 | 25 | 77 | 128 | 249 | 307 | 328 |
| 16 | scFv | Human Naive | P1R32-A10 | 33 | 72 | 91 | 205 | 296 | 368 |
| 17 | scFv | Human Naive | P1R32-A6 | 42 | 56 | 156 | 222 | 301 | 346 |
| 18 | scFv | Human Naive | P1R32-C12 | 16 | 63 | 131 | 262 | 282 | 397 |
| 19 | scFv | Human Naive | P1R32-C8 | 25 | 77 | 125 | 251 | 293 | 384 |
| 20 | scFv | Human Naive | P1R32-E9 | 42 | 56 | 118 | 264 | 277 | 392 |
| 21 | scFv | Human Naive | P1R32-F10 | 25 | 77 | 117 | 251 | 293 | 387 |
| 22 | scFv | Human Naive | P1R32-H11 | 42 | 56 | 95 | 239 | 310 | 324 |
| 23 | Fab | Human Synthetic | P2R31-H3 | 20 | 74 | 138 | 200 | 297 | 354 |

**Table 8.**

| No. | Type | Source | Clone | HCDR1 SEQ ID NO | HCDR2 SEQ ID NO | HCDR3 SEQ ID NO | LCDR1 SEQ ID NO | LCDR2 SEQ ID NO | LCDR3 SEQ ID NO |
|---|---|---|---|---|---|---|---|---|---|
| 24 | scFv | Human Naive | P1R21-A7 | 6 | 65 | 123 | 193 | 319 | 373 |
| 25 | scFv | | P1R21-B12 | 42 | 56 | 95 | 239 | 310 | 322 |
| 26 | scFv | | P1R21-B6 | 42 | 59 | 90 | 254 | 279 | 395 |
| 27 | scFv | | P1R21-B9 | 42 | 56 | 94 | 239 | 310 | 325 |
| 28 | scFv | | P1R21-C12 | 42 | 56 | 88 | 261 | 276 | 393 |
| 29 | scFv | | P1R21-D11 | 8 | 66 | 116 | 189 | 315 | 340 |
| 30 | scFv | | P1R21-D9 | 42 | 56 | 133 | 260 | 295 | 394 |
| 31 | scFv | | P1R21-E9 | 42 | 56 | 87 | 241 | 290 | 334 |
| 32 | scFv | | P1R21-F11 | 24 | 79 | 137 | 262 | 283 | 405 |
| 33 | scFv | | P1R21-F5 | 7 | 64 | 186 | 227 | 273 | 342 |
| 34 | scFv | | P1R21-H10 | 30 | 48 | 129 | 197 | 319 | 378 |
| 35 | scFv | | P1R21-H2 | 25 | 77 | 99 | 251 | 293 | 387 |
| 36 | scFv | | P1R22-A1 | 25 | 77 | 164 | 203 | 296 | 369 |
| 37 | scFv | | P1R22-A3 | 42 | 56 | 163 | 250 | 294 | 383 |
| 38 | scFv | | P1R22-B10 | 34 | 54 | 146 | 229 | 303 | 333 |
| 39 | scFv | | P1R22-B2 | 39 | 81 | 127 | 244 | 312 | 335 |
| 40 | scFv | | P1R22-B4 | 5 | 67 | 144 | 228 | 310 | 321 |
| 41 | scFv | | P1R22-C10 | 26 | 44 | 150 | 235 | 309 | 330 |
| 42 | scFv | | P1R22-C11 | 42 | 56 | 104 | 247 | 293 | 385 |
| 43 | scFv | | P1R22-C2 | 27 | 77 | 172 | 262 | 278 | 408 |
| 44 | scFv | | P1R22-C6 | 42 | 56 | 101 | 195 | 319 | 379 |
| 45 | scFv | | P1R22-D3 | 26 | 44 | 161 | 239 | 310 | 324 |
| 46 | scFv | | P1R22-E1 | 42 | 56 | 97 | 197 | 319 | 375 |
| 47 | scFv | | P1R22-E2 | 43 | 80 | 149 | 243 | 317 | 323 |
| 48 | scFv | | P1R22-F11 | 27 | 51 | 152 | 210 | 268 | 357 |
| 49 | scFv | | P1R22-G10 | 25 | 78 | 119 | 231 | 293 | 387 |
| 50 | scFv | | P1R22-G5 | 15 | 47 | 107 | 215 | 301 | 341 |
| 51 | scFv | | P1R22-H1 | 42 | 56 | 102 | 258 | 274 | 402 |
| 52 | scFv | | P1R22-H8 | 35 | 61 | 151 | 214 | 301 | 347 |
| 53 | scFv | | P1R31-A4 | 42 | 56 | 95 | 239 | 310 | 325 |
| 54 | scFv | | P1R31-B1 | 8 | 66 | 116 | 188 | 314 | 340 |
| 55 | scFv | | P1R31-B11 | 14 | 85 | 153 | 220 | 299 | 349 |
| 56 | scFv | | P1R31-B2 | 39 | 81 | 127 | 244 | 313 | 335 |
| 57 | scFv | | P1R31-B4 | 42 | 57 | 132 | 191 | 305 | 389 |
| 58 | scFv | | P1R31-B9 | 36 | 75 | 121 | 236 | 280 | 337 |
| 59 | scFv | | P1R31-C11 | 25 | 77 | 168 | 221 | 301 | 345 |

**Table 9.**

| No. | Type | Source | Clone | HCDR1 SEQ ID NO | HCDR2 SEQ ID NO | HCDR3 SEQ ID NO | LCDR1 SEQ ID NO | LCDR2 SEQ ID NO | LCDR3 SEQ ID NO |
|---|---|---|---|---|---|---|---|---|---|
| 60 | scFv | Human Naive | P1R31-C2 | 31 | 56 | 148 | 197 | 319 | 378 |
| 61 | scFv | | P1R31-D11 | 42 | 56 | 106 | 256 | 285 | 396 |
| 62 | scFv | | P1R31-D6 | 26 | 44 | 167 | 255 | 284 | 398 |
| 63 | scFv | | P1R31-D9 | 42 | 56 | 95 | 190 | 310 | 324 |
| 64 | scFv | | P1R31-F1 | 6 | 65 | 124 | 197 | 319 | 373 |
| 65 | scFv | | P1R31-F4 | 17 | 62 | 120 | 196 | 319 | 380 |
| 66 | scFv | | P1R31-F5 | 4 | 65 | 166 | 226 | 318 | 336 |
| 67 | scFv | | P1R31-F8 | 42 | 57 | 96 | 239 | 310 | 325 |
| 68 | scFv | | P1R31-G6 | 25 | 77 | 154 | 217 | 302 | 343 |
| 69 | scFv | | P1R31-H4 | 42 | 56 | 115 | 263 | 281 | 411 |
| 70 | scFv | | P1R32-A9 | 32 | 49 | 108 | 225 | 307 | 410 |
| 71 | scFv | | P1R32-B2 | 42 | 56 | 95 | 242 | 308 | 331 |
| 72 | scFv | | P1R32-B5 | 26 | 44 | 150 | 234 | 316 | 326 |
| 73 | scFv | | P1R32-C1 | 42 | 56 | 95 | 192 | 311 | 322 |
| 74 | scFv | | P1R32-C11 | 26 | 44 | 155 | 235 | 309 | 330 |
| 75 | scFv | | P1R32-D4 | 25 | 77 | 168 | 213 | 301 | 345 |
| 76 | scFv | | P1R32-D7 | 24 | 79 | 137 | 262 | 283 | 406 |
| 77 | scFv | | P1R32-E4 | 42 | 56 | 100 | 248 | 307 | 391 |
| 78 | scFv | | P1R32-E6 | 26 | 44 | 183 | 197 | 319 | 378 |
| 79 | scFv | | P1R32-E8 | 25 | 77 | 93 | 251 | 293 | 387 |
| 80 | scFv | | P1R32-F1 | 42 | 55 | 103 | 259 | 283 | 400 |
| 81 | scFv | | P1R32-F7 | 42 | 56 | 92 | 262 | 278 | 399 |
| 82 | scFv | | P1R32-F9 | 26 | 44 | 170 | 198 | 288 | 352 |
| 83 | scFv | | P1R32-G10 | 25 | 77 | 165 | 251 | 291 | 387 |
| 84 | scFv | | P1R32-G2 | 42 | 56 | 102 | 257 | 274 | 402 |
| 85 | scFv | | P1R32-H2 | 25 | 77 | 111 | 253 | 292 | 381 |
| 86 | scFv | | P1R32-H5 | 38 | 58 | 86 | 219 | 301 | 348 |
| 87 | scFv | | P1R32-H6 | 42 | 56 | 122 | 262 | 283 | 405 |
| 88 | scFv | | P1R32-H9 | 42 | 56 | 134 | 209 | 289 | 364 |

**Table 10.**

| No. | Type | Source | Clone | HCDR1 SEQ ID NO | HCDR2 SEQ ID NO | HCDR3 SEQ ID NO | LCDR1 SEQ ID NO | LCDR2 SEQ ID NO | LCDR3 SEQ ID NO |
|---|---|---|---|---|---|---|---|---|---|
| 89 | Fab | Human Synthetic | P2R21-E2 | 32 | 50 | 162 | 216 | 300 | 344 |
| 90 | Fab | | P2R22-E6 | 20 | 69 | 141 | 211 | 269 | 356 |
| 91 | Fab | | P2R31-A2 | 12 | 73 | 140 | 199 | 297 | 353 |
| 92 | Fab | | P2R31-A3 | 28 | 70 | 145 | 218 | 300 | 390 |
| 93 | Fab | | P2R31-B4 | 21 | 74 | 135 | 210 | 267 | 370 |
| 94 | Fab | | P2R31-B7 | 20 | 45 | 177 | 210 | 289 | 363 |
| 95 | Fab | | P2R31-B9 | 28 | 46 | 184 | 204 | 287 | 366 |
| 96 | Fab | | P2R31-C5 | 20 | 45 | 175 | 209 | 265 | 350 |
| 97 | Fab | | P2R31-C8 | 26 | 74 | 178 | 209 | 286 | 377 |
| 98 | Fab | | P2R31-C9 | 21 | 74 | 187 | 209 | 268 | 374 |
| 99 | Fab | | P2R31-D4 | 19 | 44 | 142 | 200 | 297 | 355 |
| 100 | Fab | | P2R31-E2 | 23 | 74 | 174 | 211 | 268 | 351 |
| 101 | Fab | | P2R31-F10 | 10 | 74 | 89 | 209 | 289 | 372 |
| 102 | Fab | | P2R31-G10 | 37 | 60 | 139 | 206 | 286 | 339 |
| 103 | Fab | | P2R31-G7 | 41 | 83 | 157 | 232 | 306 | 329 |
| 104 | Fab | | P2R32-A1 | 18 | 74 | 178 | 209 | 268 | 372 |
| 105 | Fab | | P2R32-A2 | 32 | 49 | 179 | 224 | 300 | 344 |
| 106 | Fab | | P2R32-A7 | 20 | 74 | 176 | 210 | 287 | 359 |
| 107 | Fab | | P2R32-B7 | 26 | 74 | 178 | 209 | 287 | 361 |
| 108 | Fab | | P2R32-D1 | 22 | 71 | 171 | 210 | 286 | 360 |
| 109 | Fab | | P2R32-D11 | 11 | 44 | 159 | 208 | 286 | 371 |
| 110 | Fab | | P2R32-D12 | 20 | 44 | 185 | 212 | 287 | 365 |
| 111 | Fab | | P2R32-D5 | 41 | 84 | 136 | 238 | 271 | 332 |
| 112 | Fab | | P2R32-E11 | 20 | 44 | 173 | 202 | 298 | 367 |
| 113 | Fab | | P2R32-F1 | 20 | 74 | 182 | 209 | 286 | 376 |
| 114 | Fab | | P2R32-F11 | 9 | 74 | 180 | 207 | 286 | 362 |
| 115 | Fab | | P2R32-G8 | 41 | 82 | 158 | 240 | 270 | 327 |
| 116 | Fab | | P2R32-G9 | 29 | 44 | 181 | 209 | 286 | 376 |

**Table 11.**

| No. | Source | Clone | VH SEQ ID NO | VL SEQ ID NO | No. | Source | Clone | VH SEQ ID NO | VL SEQ ID NO |
|---|---|---|---|---|---|---|---|---|---|
| 1 | Human Naïve | P1R21-A1 | 412 | 528 | 13 | Human Naïve | P1R31-F7 | 424 | 540 |
| 2 | Human Naïve | P1R21-B3 | 413 | 529 | 14 | Human Naïve | P1R31-G8 | 425 | 541 |
| 3 | Human Naïve | P1R21-E3 | 414 | 530 | 15 | Human Naïve | P1R31-H11 | 426 | 542 |
| 4 | Human Naïve | P1R21-E4 | 415 | 531 | 16 | Human Naïve | P1R32-A10 | 427 | 543 |
| 5 | Human Naïve | P1R21-F4 | 416 | 532 | 17 | Human Naïve | P1R32-A6 | 428 | 544 |
| 6 | Human Naïve | P1R21-H11 | 417 | 533 | 18 | Human Naïve | P1R32-C12 | 429 | 545 |
| 7 | Human Naïve | P1R22-G9 | 418 | 534 | 19 | Human Naïve | P1R32-C8 | 430 | 546 |
| 8 | Human Naïve | P1R31-A10 | 419 | 535 | 20 | Human Naïve | P1R32-E9 | 431 | 547 |
| 9 | Human Naïve | P1R31-B8 | 420 | 536 | 21 | Human Naïve | P1R32-F10 | 432 | 548 |
| 10 | Human Naïve | P1R31-C8 | 421 | 537 | 22 | Human Naïve | P1R32-H11 | 433 | 549 |
| 11 | Human Naïve | P1R31-E1 | 422 | 538 | 23 | Human Synthetic | P2R31-H3 | 434 | 550 |
| 12 | Human Naïve | P1R31-E10 | 423 | 539 | | | | | |

**Table 12.**

| No. | Source | Clone | VH SEQ ID NO | VL SEQ ID NO | No. | Source | Clone | VH SEQ ID NO | VL SEQ ID NO |
|---|---|---|---|---|---|---|---|---|---|
| 24 | Human Naïve | P1R21-A7 | 435 | 551 | 48 | Human Naïve | P1R22-F11 | 459 | 575 |
| 25 | Human Naïve | P1R21-B12 | 436 | 552 | 49 | Human Naïve | P1R22-G10 | 460 | 576 |
| 26 | Human Naïve | P1R21-B6 | 437 | 553 | 50 | Human Naïve | P1R22-G5 | 461 | 577 |
| 27 | Human Naïve | P1R21-B9 | 438 | 554 | 51 | Human Naïve | P1R22-H1 | 462 | 578 |
| 28 | Human Naïve | P1R21-C12 | 439 | 555 | 52 | Human Naïve | P1R22-H8 | 463 | 579 |
| 29 | Human Naïve | P1R21-D11 | 440 | 556 | 53 | Human Naïve | P1R31-A4 | 464 | 580 |
| 30 | Human Naïve | P1R21-D9 | 441 | 557 | 54 | Human Naïve | P1R31-B1 | 465 | 581 |
| 31 | Human Naïve | P1R21-E9 | 442 | 558 | 55 | Human Naïve | P1R31-B11 | 466 | 582 |
| 32 | Human Naïve | P1R21-F11 | 443 | 559 | 56 | Human Naïve | P1R31-B2 | 467 | 583 |
| 33 | Human Naïve | P1R21-F5 | 444 | 560 | 57 | Human Naïve | P1R31-B4 | 468 | 584 |
| 34 | Human Naïve | P1R21-H10 | 445 | 561 | 58 | Human Naïve | P1R31-B9 | 469 | 585 |
| 35 | Human Naïve | P1R21-H2 | 446 | 562 | 59 | Human Naïve | P1R31-C11 | 470 | 586 |
| 36 | Human Naïve | P1R22-A1 | 447 | 563 | 60 | Human Naïve | P1R31-C2 | 471 | 587 |
| 37 | Human Naïve | P1R22-A3 | 448 | 564 | 61 | Human Naïve | P1R31-D11 | 472 | 588 |
| 38 | Human Naïve | P1R22-B10 | 449 | 565 | 62 | Human Naïve | P1R31-D6 | 473 | 589 |
| 39 | Human Naïve | P1R22-B2 | 450 | 566 | 63 | Human Naïve | P1R31-D9 | 474 | 590 |
| 40 | Human Naïve | P1R22-B4 | 451 | 567 | 64 | Human Naïve | P1R31-F1 | 475 | 591 |
| 41 | Human Naïve | P1R22-C10 | 452 | 568 | 65 | Human Naïve | P1R31-F4 | 476 | 592 |
| 42 | Human Naïve | P1R22-C11 | 453 | 569 | 66 | Human Naïve | P1R31-F5 | 477 | 593 |
| 43 | Human Naïve | P1R22-C2 | 454 | 570 | 67 | Human Naïve | P1R31-F8 | 478 | 594 |
| 44 | Human Naïve | P1R22-C6 | 455 | 571 | 68 | Human Naïve | P1R31-G6 | 479 | 595 |
| 45 | Human Naïve | P1R22-D3 | 456 | 572 | 69 | Human Naïve | P1R31-H4 | 480 | 596 |
| 46 | Human Naïve | P1R22-E1 | 457 | 573 | 70 | Human Naïve | P1R32-A9 | 481 | 597 |
| 47 | Human Naïve | P1R22-E2 | 458 | 574 | | | | | |

**Table 13.**

| No. | Source | Clone | VH SEQ ID NO | VL SEQ ID NO | No. | Source | Clone | VH SEQ ID NO | VL SEQ ID NO |
|---|---|---|---|---|---|---|---|---|---|
| 71 | Human Naïve | P1R32-B2 | 482 | 598 | 94 | Human Synthetic | P2R31-B7 | 505 | 621 |
| 72 | Human Naïve | P1R32-B5 | 483 | 599 | 95 | Human Synthetic | P2R31-B9 | 506 | 622 |
| 73 | Human Naïve | P1R32-C1 | 484 | 600 | 96 | Human Synthetic | P2R31-C5 | 507 | 623 |
| 74 | Human Naïve | P1R32-C11 | 485 | 601 | 97 | Human Synthetic | P2R31-C8 | 508 | 624 |
| 75 | Human Naïve | P1R32-D4 | 486 | 602 | 98 | Human Synthetic | P2R31-C9 | 509 | 625 |
| 76 | Human Naïve | P1R32-D7 | 487 | 603 | 99 | Human Synthetic | P2R31-D4 | 510 | 626 |
| 77 | Human Naïve | P1R32-E4 | 488 | 604 | 100 | Human Synthetic | P2R31-E2 | 511 | 627 |
| 78 | Human Naïve | P1R32-E6 | 489 | 605 | 101 | Human Synthetic | P2R31-F10 | 512 | 628 |
| 79 | Human Naïve | P1R32-E8 | 490 | 606 | 102 | Human Synthetic | P2R31-G10 | 513 | 629 |
| 80 | Human Naïve | P1R32-F1 | 491 | 607 | 103 | Human Synthetic | P2R31-G7 | 514 | 630 |
| 81 | Human Naïve | P1R32-F7 | 492 | 608 | 104 | Human Synthetic | P2R32-A1 | 515 | 631 |
| 82 | Human Naïve | P1R32-F9 | 493 | 609 | 105 | Human Synthetic | P2R32-A2 | 516 | 632 |
| 83 | Human Naïve | P1R32-G10 | 494 | 610 | 106 | Human Synthetic | P2R32-A7 | 517 | 633 |
| 84 | Human Naïve | P1R32-G2 | 495 | 611 | 107 | Human Synthetic | P2R32-B7 | 518 | 634 |
| 85 | Human Naïve | P1R32-H2 | 496 | 612 | 108 | Human Synthetic | P2R32-D1 | 519 | 635 |
| 86 | Human Naïve | P1R32-H5 | 497 | 613 | 109 | Human Synthetic | P2R32-D11 | 520 | 636 |
| 87 | Human Naïve | P1R32-H6 | 498 | 614 | 110 | Human Synthetic | P2R32-D12 | 521 | 637 |
| 88 | Human Naïve | P1R32-H9 | 499 | 615 | 111 | Human Synthetic | P2R32-D5 | 522 | 638 |
| 89 | Human Synthetic | P2R21-E2 | 500 | 616 | 112 | Human Synthetic | P2R32-E11 | 523 | 639 |
| 90 | Human Synthetic | P2R22-E6 | 501 | 617 | 113 | Human Synthetic | P2R32-F1 | 524 | 640 |
| 91 | Human Synthetic | P2R31-A2 | 502 | 618 | 114 | Human Synthetic | P2R32-F11 | 525 | 641 |
| 92 | Human Synthetic | P2R31-A3 | 503 | 619 | 115 | Human Synthetic | P2R32-G8 | 526 | 642 |
| 93 | Human Synthetic | P2R31-B4 | 504 | 620 | 116 | Human Synthetic | P2R32-G9 | 527 | 643 |

**Table 14.**

| Name | SEQ ID NO | Sequence |
|---|---|---|
| P1R21-A1_VH | 412 | EVQLVETGGGVVQPGRSLRLSCAAAGFSFSNYGIHWVRQAPGKGLEW VAVISYDGRNKYYADSVKGRFTLSRDNSKNTLYLQMNSLGPEDTAMY YCAKDMDVWGQGTTVTVSS |
| P1R21-B3_VH | 413 | EVQLVQSGAEVKKPGASVKVSCKASGYTFTNYYMHWVRQAPGQGLE WMGIINPSGGSANYAQKFQGRVTMTRDTSTSTVYMELTRLRSQDTAV YYCARSLRSGFDSWGQGTLVTVSS |
| P1R21-E3_VH | 414 | EVQLVQSGAEVKKPGASVKVSCKASGYTFTSYYMHWVRQAPGQGLE WMGIINPSGGSTSYAQKFQGRVTMTRDTSTSTVYMELSSLRSEDTAVY YCARDVAAAGDYWGQGTLVTVSS |
| P1R21-E4_VH | 415 | EVQLVESGGGLVQPGGSLRLSCAASGFTFSSYAMSWVRQAPGKGLEW VSAISGSGGSTYYADSVKGRFTISRDNAKNSLYLQMNSLRAEDTAVYY CARIQHYYDSSGRFRYYYYGMDVWGQGTTVTVSS |
| P1R21-F4_VH | 416 | EVQLVESGGGVVQPGRSLRLSCAASGFTFSSYAMHWVRQAPGKGLEW VAVISYDGSNKYYADSVKGRFTISRDNSKNTLYLQMNSLRAEDTAVY YCARDRGYGMDVWGQGTLVTVSS |
| P1R21-H11_VH | 417 | QVQLQQSGPGLVKSSQTLSLTCAISGDSVSSNSAAWNWIRQSPSRGLE WLGRTYYRSRWYNDYAVSVKSRITINPDTSKNQFSLQLNSVTPEDTAV YYCASGWAMDAWGRGTTVTVSS |
| P1R22-G9_VH | 418 | EVQLLESGGGVVQPGRSLRLSCAASGFTFSSYAMHWVRQAPGKGLEW VAVISYDGSNKYYADSVKGRFTISRDNSKNTLYLQMNSLRAEDTAVY YCARDRGNGMDVWGQGTTVTVSS |
| P1R31-A10_VH | 419 | EVQLVQSGAEVKKPGASVKVSCKASGYTFTSYYMHWVRQAPGQGLE WMGIINPSGGSTSYAQKFQGRVTMTRDTSTSTVYMELSSLRSEDTAVY YCARDRRSSSLDYWGQGTLVTVSS |
| P1R31-B8_VH | 420 | EVQLVQSGAEVKKPGASVKVSCKASGYTFTNYYMHWVRQAPGQGLE WMGIINPSGGSANYAQKFQGRVTMTRDTSTSTVYMELTRLRSQDTAV YYCARSLRSGFDSWGQGTLVTVSS |
| P1R31-C8_VH | 421 | EVQLVQSGAEVKKPGASVKVSCKASGYTFTSYYMHWVRQAPGQGLE WMGIINPSGGSTSYAQKFQGRVTMTRDTSTSTVYMELSSLRSEDTAVY YCARDGSSGNPPVYGMDVWGQGTTVTVSS |
| P1R31-E1_VH | 422 | QVQLQQSGAEVKKPGASVKVSCKASGYTFTSYYMHWVRQAPGQGLE WMGIINPSGGSTSYAQKFQGRVTMTRDTSTSTVYMELSSLRSEDTAVY YCARDFDYWGQGTLVTVSS |
| P1R31-E10_VH | 423 | EVQLVESGGGVVQPGRSLRLSCAASGFTFSSYAMHWVRQAPGKGLEW VAVISYDGSNKYYADSVKGRFTISRDNSKNTLYLQMNSLRAEDTAVY YCARDYHYYMDVWGKGTTVTVSS |
| P1R31-F7_VH | 424 | EVQLVETGGGVVQPGRSLRLSCAASGFTFSSYAMHWVRQAPGKGLEW VAVISYDGSNKYYADSVKGRFTISRDNSKNTLYLQMNSLRAEDTAVY YCAREYGSGSRGYGMDVWGQGTTVTVSS |
| P1R31-G8_VH | 425 | EVQLVESGGGVVQPGGSLRLSCAASGFTFSTYGMHWVRQAPGKGLEW VAFIRYDGNSKYYADSVKGRFTISRDNSKNTLYLQMNSLRAEDTAVYY CAKGGAGSGHMNVWGQGTTVTVSS |
| P1R31-H11_VH | 426 | QVQLVESGGGVVQPGRSLRLSCAASGFTFSSYAMHWVRQAPGKGLEW VAVISYDGSNKYYADSVKGRFTISRDNSKNTLYLQMNSLRAEDTAVY YCASGARNSDAFDIWGQGTTVTVSS |
| P1R32-A10_VH | 427 | EVQLVQSGAEVKKSGDSLKISCKGSGYDFSTNWIAWVRQMPGKGLEL MGVIHPGGSEIKSSPSFQGQVTFSADTSSSTAYLQWSSLKASDTAMYYC ARAVYYDSSGGMDVWGQGTLVTVSS |
| P1R32-A6_VH | 428 | QVQLVQSGAEVKKPGASVKVSCKASGYTFTSYYMHWVRQAPGQGLE WMGIINPSGGSTSYAQKFQGRVTMTRDTSTSTVYMELSSLRSEDTAVY YCARRAFDIWGQGTTVTVSS |
| P1R32-C12_VH | 429 | EVQLVETGGGLVQPGGSLRLSCAASGFTFSNYWMHWVRQAPGKGLE WVANIKQDGSGQYYMDSVKGRFTISRDNAQNSLFLQMDSLRAEDTAI YYCARGGGIAAFYWGQGTLVTVSS |
| P1R32-C8_VH | 430 | EVQLLESGGGVVQPGRSLRLSCAASGFTFSSYAMHWVRQAPGKGLEW VAVISYDGSNKYYADSVKGRFTISRDNSKNTLYLQMNSLRAEDTAVY YCAREWRGYGMDVWGQGTTVTVSS |

**Table 15.**

| Name | SEQ ID NO | Sequence |
|---|---|---|
| P1R21-E9_VH | 442 | EVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARAHSSSLDIWGQGTTVTVSS |
| P1R21-F11_VH | 443 | EVQLVESGGGVVQPGRSLRLSCAASGFTFSSSAMHWVRQAPGKGLEWV AVTSYDGRYRYYADSVRGRFTISRDNSKNTLFMQMNSLRAEDTAVYYC ARGPSIYKYGMDVWGQGTTVTVSS |
| P1R21-F5_VH | 444 | QVQLQQSGPGLVKPSQTLSLTCAISGDSVSSNSVTWHWIRQSPSRGLEWL GRAYYRSKWYYNYAVSVKSRVTINPDTSKNQFSLQLNSVTPEDTAVYY CARYYDYRMDVWGQGTTVTVSS |
| P1R21-H10_VH | 445 | EVQLVESGGGVVQPGGSLRLSCAASGFTFSTYGMHWVRQAPGKGLEWV AFIRYDGNSKYYADSVKGRFTISRDNSKNTLYLQMNSLRAEDTAVYYCA KGGAGSGHMDVWGQGTTVTVSS |
| P1R21-H2_VH | 446 | EVQLLESGGGVVQPGRSLRLSCAASGFTFSSYAMHWVRQAPGKGLEWV AVISYDGSNKYYADSVKGRFTISRDNSKNTLYLQMNSLRAEDTAVYYCA RDKANGMDVWGQGTTVTVSS |
| P1R22-A1_VH | 447 | EVQLLESGGGVVQPGRSLRLSCAASGFTFSSYAMHWVRQAPGKGLEWV AVISYDGSNKYYADSVKGRFTISRDNSKNTLYLQMNSLRAEDTAVYYCA RTKGQQLAFDYWGQGTLVTVSS |
| P1R22-A3_VH | 448 | QVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARSTAAADMDVWGQGTTVTVSS |
| P1R22-B10_VH | 449 | EVQLVQSGAEVKKPGASVKVSCKASGYIFTSYSMHWVRQAPGQGLEW MGIINPSGGSTSFAQKFQGRVTMTRDTSTSTVYMELSSLRSEDTAVYYCA RHHGMDVWGQGTTVTVSS |
| P1R22-B2_VH | 450 | QVQLVQSGAEVKKPGASVKVSCRASGYTFTNYAIHWVRQAPGQRLEW MGWINGDNGNTKHSQKFQGRVTITRDTSATTAYMELSSLRSEDTAVYY CAVFGSGAYGNGAFDYWGQGTLVTVSS |
| P1R22-B4_VH | 451 | QVQLQQSGPGVVKPSQTLSLTCAISGDSVSNNNGAWNWIRQSPSRGLEW LGRTYYRSQWHHEYAVSVKSRITINPDTSGNQFSLQLNSVTPEDTAVYY CARGWAMDVWGQGTLVTVSS |
| P1R22-C10_VH | 452 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYCA KLLRTGYFDLWGRGTLVTVSS |
| P1R22-C11_VH | 453 | QVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARDLVYGSGSYGMDVWGQGTMVTVSS |
| P1R22-C2_VH | 454 | QVQLVESGGGVVQPGRSLRLSCAASGFTFSSYGMHWVRQAPGKGLEWV AVISYDGSNKYYADSVKGRFTISRDNSKNTLYLQMNSLRAEDTAVYYCA KVISRTNGMDVWGQGTTVTVSS |
| P1R22-C6_VH | 455 | QVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARDLAAARSGHGRRNYYYGMDVWGQGTTVTVSS |
| P1R22-D3_VH | 456 | EVQLVETGGGLVQPGGSLRLSCAASGFTFSSYAMSWVRQAPGKGLEWV SAISGSGGSTYYADSVKGRFTISRDNSKNTLYLQMNSLRAEDTAVYYCA KSLRSSWLFDYWGQGTLVTVSS |
| P1R22-E1_VH | 457 | QVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARDGIVGGASGARYYYYYMDVWGKGTTVTVSS |
| P1R22-E2_VH | 458 | QVQLVQSGGGVVQPGRSLRLSCAASRFTFNSYAMHWVRQAPGKGLEW VAVVSSDGNKKYYADSVKGRFTISRDNSKNTLYLQMNSLRAEDTAVYY CAKLGGGSGNYGMDVWGQGTLVTVSS |
| P1R22-F11_VH | 459 | QVQLVQSGGGVVQPGRSLRLSCAASGFTFSSYGMHWVRQAPGKGLEW VAGISYDGRNKYYADSVKGRFTISRDNSKNTLYLQMNSLRAEDTAVYY CATLSSGWYMAFDYWGQGTLVTVSS |
| P1R22-G10_VH | 460 | EVQLVESGGGVNQPGRSLRLSCAASGFTFSSYAMHWVRQAPGKGLEWM AVISYDVSNKYYADSVKGRFTISRDNSKNTLYLQMNSLRAEDTAVYYCA REGSSYGMDVWGQGTTVTVSS |
| P1R22-G5_VH | 461 | EVQLVESGGGVVQPGRSLRLSCAASGFTFRTYVMHWVRQTPGKGLEWV AAISSDGSNKYYADSVKGRFTISRDNSKNTLYVQMNSLRGEDTAVYYCA RDPLSVAGPFDYWGQGTLVTVSS |
| P1R22-H1_VH | 462 | EVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARDLHEAYGMDVWGQGTTVTVSS |

**Table 16.**

| Name | SEQ ID NO | Sequence |
|---|---|---|
| P1R22-H8_VH | 463 | EVQLVQSGAEVKKPGESLKISCKGSGYRFTNYWIGWVRQMPGKGLEWMG IIYPGDSKTRYSPSFQGQVTISADKSISTTYLQWSSLKASDTAIYYCARLPGY GAYERIDPNDDYWGQGTLVTVSS |
| P1R31-A4_VH | 464 | QVQLQQSGAEVKKPGASVKVSCKASGYTFTSYYMHWVRQAPGQGLEWM GIINPSGGSTSYAQKFQGRVTMTRDTSTSTVYMELSSLRSEDTAVYYCARD FDYWGQGTLVTVSS |
| P1R31-B1_VH | 465 | QVQLQQSGPGLVKPSQTLSLTCAISGDSVTSNSAAWNWIRQSPSRGLEWL GRTYYRSKWYTDYAVSVKGRITINPDISKDQFSLQLNSVTPEDTAVYYCAR EEISVVRGVKIDYWGQGTLVTVSS |
| P1R31-B11_V H | 466 | QVQLVESGGGLVQPGGSLRLSCAASGFSFSSYSMNWVRQAPGKGLEWVS YVSRDSRTIHYADSVKGRFSISRDNAKNSLYLQMNSLRDDDTAVYYCVK MGPGEYYGASNDYYYNGMDVWGQGTTVTVSS |
| P1R31-B2_VH | 467 | QVQLVQSGAEVKKPGASVKVSCRASGYTFTNYAIHWVRQAPGQRLEWM GWINGDNGNTKHSQKFQGRVTITRDTSATTAYMELSSLRSEDTAVYYCAV FGSGAYGNGAFDYWGQGTLVTVSS |
| P1R31-B4_VH | 468 | QVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARG GIAAAGTRYNWFDPWGQGTLVTVSS |
| P1R31-B9_VH | 469 | EVQLVQSGAEVKNPGASVKVSCKASGYTFGSHYMHAVRQAPGQGLEWV GVISPNGDRTRYAQKFKGRVTMTRDTSTNTVYMELSSLGSEDTAVYYCVR EPQNSGSFEYWGRGTLVTVSS |
| P1R31-C11_V H | 470 | EVQLVETGGGVVQPGRSLRLSCAASGFTFSSYAMHWVRQAPGKGLEWVA VISYDGSNKYYADSVKGRFTISRDNSKNTLYLQMNSLRAEDTAVYYCARV GGGRDSTDYWGQGTLVTVSS |
| P1R31-C2_VH | 471 | EVQLVQSGAEVKKPGSSVKVSCKASGGTFSRYGISWVRQAPGQGLEWMG IINPSGGSTSYAQKFQGRVTMTRDTSTSTVYMELSSLRSEDTAVYYCARKA RRPGGHFDYWGQGTLVTVSS |
| P1R31-D11_V H | 472 | EVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD NGADTAIDYWGQGTLVTVSS |
| P1R31-D6_VH | 473 | EVQLLESGGGLVQPGGSLRLSCAASGFTFSSYAMSWVRQAPGKGLEWVS AISGSGGSTYYADSVKGRFTISRDNSKNTLYLQMNSLRAEDTAVYYCAKT VAGTFDYWGQGTLVTVSS |
| P1R31-D9_VH | 474 | EVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD FDYWGQGTLVTVSS |
| P1R31-F1_VH | 475 | QVQLQQSGPGLVKPSQTLSLTCAISGDSVSSNSAAWNWIRQSPSRGLEWLG RTYYRSKWYNDYAVSVKSRITINPDTSKNQFSLQLNSVTPEDTAVYYCAR EVAAAPRYGMDVWGQGTTVTVSS |
| P1R31-F4_VH | 476 | EVQLVETGGGLVQPGESLRLSCAASGFTFSNYWMSWVRQAPGKGLEWVA NIKQDASEKYYVDSVKGRFTISRDNAKNSLYLQMNSLRAADTAVYYCAR EMAESTWAFYAYYYDMDVWGQGTTVTVSS |
| P1R31-F5_VH | 477 | QVQLQQSGPGLVKPSQTLSLTCAISGDSVSSNNVAWDWIRQSPSRGLEWL GRTYYRSKWYNDYAVSVKSRITINPDTSKNQFSLQLNSVTPEDTAVYYCA RTRPGIGLDPWGQGTLVTVSS |
| P1R31-F8_VH | 478 | QVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD FDLWGRGTLVTVSS |
| P1R31-G6_VH | 479 | EVQLVESGGGVVQPGRSLRLSCAASGFTFSSYAMHWVRQAPGKGLEWVA VISYDGSNKYYADSVKGRFTISRDNSKNTLYLQMNSLRAEDTAVYYCAR MVRGVYGMDVWGQGTTVTVSS |
| P1R31-H4_VH | 480 | QVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD YYGSLDVWGQGTTVTVSS |
| P1R32-A9_VH | 481 | EVQLVQSGAEVKKPGSSVKVSCKASGGTFSSYAISWVRQAPGQGLEWMG GIIPIFGTANYAQKFQGRVTITADESTSTAYMELSSLRSEDTAVYYCARDRA PFSYYYDSSGLFQHWGQGTLVTVSS |
| P1R32-B2_VH | 482 | QVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD FDYWGQGTLVTVSS |
| P1R32-B5_VH | 483 | EVQLVESGGGLVQPGGSLRLSCAASGFTFSSYAMSWVRQAPGKGLEWVS AISGSGGSTYYADSVKGRFTISRDNSKNTLYLQMNSLRAEDTAVYYCAKL LRTGYFDLWGRGTLVTVSS |
| P1R32-C1_VH | 484 | QVQLQQSGAEVKKPGASVKVSCKASGYTFTSYYMHWVRQAPGQGLEWM GIINPSGGSTSYAQKFQGRVTMTRDTSTSTVYMELSSLRSEDTAVYYCARD FDYWGQGTLVTVSS |
| P1R32-C11_V H | 485 | QVQLVQSGGGLVQPGGSLRLSCAASGFTFSSYAMSWVRQAPGKGLEWVS AISGSGGSTYYADSVKGRFTISRDNSKNTLYLQMNSLRAEDTAVYYCAIPH PVRGVIGEYFQHWGQGTLVTVSS |
| P1R32-D4_VH | 486 | EVQLVETGGGVVQPGRSLRLSCAASGFTFSSYAMHWVRQAPGKGLEWVA VISYDGSNKYYADSVKGRFTISRDNSKNTLYLQMNSLRAEDTAVYYCARV GGGRDSTDYWGQGTLVTVSS |
| P1R32-D7_VH | 487 | EVQLLESGGGVVQPGRSLRLSCAASGFTFSSSAMHWVRQAPGKGLEWVA VTSYDGRYRYYADSVRGRFTISRDNSKNTLFMQMNSLRAEDTAVYYCAR GPSIYKYGMDVWGQGTTVTVSS |
| P1R32-E4_VH | 488 | EVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD KSWFGELRKYYYYYGMDVWGQGTTVTVSS |
| P1R32-E6_VH | 489 | EVQLVESGGGLVQPGGSLRLSCAASGFTFSSYAMSWVRQAPGKGLEWVS AISGSGGSTYYADSVKGRFTISRDNSKNTLYLQMNSLRAEDTAVYYCAKV SGPSSGWYRGDFDYWGQGTLVTVSS |
| P1R32-E8_VH | 490 | EVQLVESGGGVVQPGRSLRLSCAASGFTFSSYAMHWVRQAPGKGLEWVA VISYDGSNKYYADSVKGRFTISRDNSKNTLYLQMNSLRAEDTAVYYCARD ASYGMDVWGQGTTVTVSS |
| P1R32-F1_VH | 491 | EVQLVQSGAEVKKPGASVKVSCKASGYTFTSYYMHWVRQAPGQGLEWM GIINPSGGSTSYAQKFKGRVTMTRDTSTSTVYMQLRSLRSEDTAVYYCAR DLVGYIDYWGQGTLVTVSS |
| P1R32-F7_VH | 492 | QVQLVQSGAEVKKPGASVKVSCKASGYTFTSYYMHWVRQAPGQGLEWM GIINPSGGSTSYAQKFQGRVTMTRDTSTSTVYMELSSLRSEDTAVYYCARD AGTTGMDVWGQGTLVTVSS |

**Table 17.**

| Name | SEQ ID NO | Sequence |
|---|---|---|
| P1R32-F9_VH | 493 | QVQLVETGGGLVQPGGSLRLSCAASGFTFSSYAMSWVRQAPGKGLEWV SAISGSGGSTYYADSVKGRFTISRDNSKNTLYLQMNSLRAEDTAVYYCV LVGRRRSNYYYYMDVWGKGTTVTVSS |
| P1R32-G10_VH | 494 | EVQLVESGGGVVQPGRSLRLSCAASGFTFSSYAMHWVRQAPGKGLEWV AVISYDGSNKYYADSVKGRFTISRDNSKNTLYLQMNSLRAEDTAVYYC ARTQRSGYFDYWGQGTLVTVSS |
| P1R32-G2_VH | 495 | EVQLVQSGAEVNKPGASLKVSCKASGYTFTSYYMHWVRQAPGQGLEW MGIINPSGGSTSYAQKFQGRVTMTRDTSTSTVYMELSSLRSEDTAVYYC ARDLHEAYGMDVWCQGTTVTVSS |
| P1R32-H2_VH | 496 | EVQLLESGGGVVQPGRSLRLSCAASGFTFSSYAMHWVRQAPGKGLEWV AVISYDGSNKYYADSVKGRFTISRDNSKNTLYLQMNSLRAEDTAVYYC ARDRKWGYFDPYYYYGMDVWGQGTTVTVSS |
| P1R32-H5_VH | 497 | QVQLVESGAEVRKPGASVKVSCKASGYTFTKYYMHWIRQAPGQGLEW MGIINPSGGSTTYAQKFQGRVTMTRDTSTSTVYMELSSLRSEDTAVYYC SRAELDYWGQGTLVTVSS |
| P1R32-H6_VH | 498 | EVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC AREPRDCSGGSCGGMDVWGQGTTVTVSS |
| P1R32-H9_VH | 499 | QVQLVQSGAEVKKPGASVKVSCKASGYTFTSYYMHWVRQAPGQGLEW MGIINPSGGSTSYAQKFQGRVTMTRDTSTSTVYMELSSLRSEDTAVYYC ARGGYGGKSRGTYYYYGMDVWGQGTTVTVSS |
| P2R21-E2_VH | 500 | QVQLVQSGAEEVKKPGSSVKVSCKASGGTFSSYAISWVRQAPGQGLEW MGGIIPILGTANYAQKFQGRVTITADESTSTAYMELSSLRSEDTAVYYCA RSRRSGFDYWGQGTLVTVSS |
| P2R22-E6_VH | 501 | EVQLLESGGGGLVQPGGSLRLSCAASGFTFSRYAMSWVRQAPGKGLEW VSSISGSGGSTYYADSVKGRFTISRDNSKNTLYLQMNSLRAEDTAVYYC ARGSRRVAFDYWGQSTLVTVSS |
| P2R31-A2_VH | 502 | EVQLLESGGGLVQPGGSLRLSCAASGFPFSSNAMSWVRQAPGKGLEW VSVISGSGGNTYYADSVKGRFTISRDNSKNTLYLQMNSLRAEDTAVYYC ARGRYYRGFDYWGQGTLVTVSS |
| P2R31-A3_VH | 503 | EVQLVESGGGGLVQPGGSLRLSCAASGFTFSSYWMNWVRQAPGKGLE WVSSISQSGSTIYYADSVKGRFTISRDNAKNSLYLQMNSLRAEDTAMYY CARGYSASFDYWGQGTLVTVSS |
| P2R31-B4_VH | 504 | EVQLLESGGGGLVQPGGSLRLSCAASGFTFSSHAMSWVRQAPGKGLEW VSVISGSGGSTYYADSVKGRFTISRDNSKNTLYLQMNSLRAEDTAVYYC AKGIDYWGQGTLVTVSS |
| P2R31-B7_VH | 505 | EVQLLESGGGGLVQPGGSLRLSCAASGFTFSRYAMSWVRQAPGKGLEW VSAISGSGSSTYYADSVKGRFTISRDNSKNTLYLQMNSLRAEDTAVYYC ARVRRPGAFDYWGQGTLVTVSS |
| P2R31-B9_VH | 506 | EVQLVESGGGGLVQPGGSLRLSCAASGFTFSSYWMNWVRQAPGKGLE WVSAISGSSSEKYYADSVKGRFTISRDNAKNSLYLQMNSLRAEDTAVYY CARVSRRSPFDPWGQGTLVTVSS |
| P2R31-C5_VH | 507 | EVQLLESGGGGLVQPGGSLRLSCAASGFTFSRYAMSWVRQAPGKGLEW VSAISGSGSSTYYADSVKGRFTISRDNSKNTLYLQMNSLRAEDTAMYYC TRVLRYGYFDYWGQGTLVTVSS |
| P2R31-C8_VH | 508 | EVQLLESGGGGLVQPGGSLRLSCAASGFTFSSYAMSWVRQAPGKGLEW VSVISGSGGSTYYADSVKGRFTISRDNSKNTLYLQMNSLRAEDTALYYC ARVRRYGYFDYWGQGTLVTVSS |
| P2R31-C9_VH | 509 | EVQLLESGGGGLVQPGGSLRLSCAASGFTFSSHAMSWVRQAPGKGLEW VSVISGSGGSTYYADSVKGRFTISRDNSKNTLYLQMNSLRAEDTALYYC ARVLRRGYFDYWGQGTLVTVSS |
| P2R31-D4_VH | 510 | EVQLLESGGGGLVQPGGSLRLSCAASGFTFSRNYMSWVRQAPGKGLEW VSAISGSGGSTYYADSVKGRFTISRDNSKNTLYLQMNSLRAEDTAVYYC ARGSYSRFDYWGQGTLVTVSS |
| P2R31-E2_VH | 511 | EVQLLESGGGGLVQPGGSLRLSCAASGFTFSSNAMSWVRQAPGKGLEW VSVISGSGGSTYYADSVKGRFTISRDNSKNTLYLQMNSLRAEDTALYYC ARVLRRGYFDVWGQGTLVTVSS |
| P2R31-F10_VH | 512 | EVQLLESGGGGLVQPGGSLRLSCAASGFPFSRYAMSWVRQAPGKGLEW VSVISGSGGSTYYADSVKGRFTISRDNSKNTLYLQMNSLRAEDTALYYC ARARYPGYFDVWGQGTLVTVSS |
| P2R31-G10_VH | 513 | QVQLVQSGAEVKKPGASVKVSCKASGYTFTGYGIHWVRQAPGQGLEW MGIISASSGNTGYAQKFQGRVTMTRDTSTSTAYMELSSLRSDDTAMYYC VRGRYYGDIWGQGTLVTVSS |
| P2R31-G7_VH | 514 | QVQLVQSGAEVKKPGASVKVSCKASGYTFTSYGIHWVRQAPGQGLEW MGWINPSSGNTKYAQKFQGRVTMTRDTSTSTAYMELSSLRSDDTAVYY CARRNTDYWGQGTLVTVSS |
| P2R32-A1_VH | 515 | EVQLLESGGGGLVQPGGSLRLSCAASGFTFSRNAMSWVRQAPGKGLEW VSVISGSGGSTYYADSVKGRFTISRDNSKNTLYLQMNSLRAEDTAVYYC ATVRRYGYFDYWGQGTLVTVSS |
| P2R32-A2_VH | 516 | QVQLVQSGAEEVKKPGSSVKVSCKASGGTFSSYAISWVRQAPGQGLEW MGGIIPIFGTANYAQKFQGRVTITADESTSTAYMELSSLRSEDTAMYYCA RVRSSGLDYWGQGTLVTVSS |
| P2R32-A7_VH | 517 | EVQLLESGGGGLVQPGGSLRLSCAASGFTFSRYAMSWVRQAPGKGLEW VSVISGSGGSTYYADSVKGRFTISRDNSKNTLYLQMNSLRAEDTAVYYC ARVPGYVPLDVWGQGTLVTVSS |
| P2R32-B7_VH | 518 | EVQLLESGGGGLVQPGGSLRLSCAASGFTFSSYAMSWVRQAPGKGLEW VSVISGSGGSTYYADSVKGRFTISRDNSKNTLYLQMNSLRAEDTAVYYC VRVRRYGYFDYWGQGTLVTVSS |
| P2R32-D1_VH | 519 | EVQLLESGGGGLVQPGGSLRLSCAASGFTFSSHYMSWVRQAPGKGLEW VSTISGSGGSTYYADSVKGRFTISRDNSKNTLYLQMNSLRAEDTALYYC ARVGRVFDIWGQGTLVTVSS |

**Table 18.**

| Name | SEQ ID NO | Sequence |
|---|---|---|
| P2R32-D12_VH | 521 | EVQLLESGGGGLVQPGGSLRLSCAASGFTFSRYAMSWVRQAPGKGLEW VSAISGSGGSTYYADSVKGRFTISRDNSKNTLYLQMNSLRAEDTALYYC ARWSYVFYIWGQGTLVTVSS |
| P2R32-D5_VH | 522 | QVQLVQSGAEVKKPGASVKVSCKASGYTFTSYGIHWVRQAPGQGLEW MGWINPYTGNTNYAQKFQGRVTMTRDTSTSTAYMELSSLRSDDTAVYY CARGKVDYWGQGTLVTVSS |
| P2R32-E11_VH | 523 | EVQLLESGGGGLVQPGGSLRLSCAASGFTFSRYAMSWVRQAPGKGLEW VSAISGSGGSTYYADSVKGRFTISRDNSKNTLYLQMNSLRAEDTAMYYC ARVLRPGYFDYWGQGTLVTVSS |
| P2R32-F1_VH | 524 | EVQLLESGGGGLVQPGGSLRLSCAASGFTFSRYAMSWVRQAPGKGLEW VSVISGSGGSTYYADSVKGRFTISRDNSKNTLYLQMNSLRAEDTAVYYC ARVRYSGYFDYWGQGTLVTVSS |
| P2R32-F11_VH | 525 | EVQLLESGGGGLVQPGGSLRLSCAASGFPFSRNAMSWVRQAPGKGLEW VSVISGSGGSTYYADSVKGRFTISRDNSKNTLYLQMNSLRAEDTALYYC ARVRYAGYFDVWGQGTLVTVSS |
| P2R32-G8_VH | 526 | QVQLVQSGAEVKKPGASVKVSCKASGYTFTSYGIHWVRQAPGQGLEW MGWINPGSGGTNYAQKFQGRVTMTRDTSTSTAYMELSSLRSDDTAVYY CARRVLDYWGQGTLVTVSS |
| P2R32-G9_VH | 527 | EVQLVESGGRGLVKPGGSLRLSCAASGFTFSTYAMSWVRQAPGKGLEW VSAISGSGGSTYYADSVKGRFTISRDNSKNTLYLQMNSLRAEDTALYYC ATVRYRGYFDYWGQGTLVTVSS |
| P1R21-A1_VL | 528 | DIVMTQSPLSLSVTPGEPASISCRSSQSLVHSDGNTYLNWFQQRPGQSPR RLIYKVSNRDSGVPDRFSGSGSGTDFTLQISRVEAEDVGVYYCMQGTHW PPITFGQGTRLEIK |
| P1R21-B3_VL | 529 | QSALTQPASVSGSPGQSITISCTGTSSDVGGYNYVSWYQQHPGKAPKLMI YDVSNRPSGVSNRFSGSKSGNTASLTISGLQAEDEADYYCSSYTSSSTGV FGTGTKVTVL |
| P1R21-E3_VL | 530 | QSALTQPASVSGSPGQSITISCTGTSSDVGGYNYVSWYQQHPGKAPKLMI YEVSNRPSGVSNRFSGSKSGNTASLTISGLQAEDEADYYCSSYTSSSTVG VVFGGGTQLTVL |
| P1R21-E4_VL | 531 | AIRMTQSPSSLFASVGDRVTITCRASQSIRNFLNWYQQKPGKAPNILIFAA SSLRSGVPSRFSGGGSGTDFTLTISSLQPEDFATYYCQQSHSVPYTFGQGT KVDIK |
| P1R21-F4_VL | 532 | QSVLTQPPSVSGAPGQRVTISCTGSSSNIGAGYDVHWYQQLPGTAPKLLI YGNSNRPSGVPDRFSGSKSGTSASLAITGLQAEDEADYYCQSYDSSPHVV FGGGTELTVL |
| P1R21-H11_VL | 533 | QPVLTQPPSASGTPGQSVTMSCSGSRSNIGRYVVNWYQHLPGTAPKLLIF NNSQRPSGVPDRFSGSKSGTSASLTISGLQSDDETDYYCAAWDDSLNGV VFGGGTKVTVL |
| P1R22-G9_VL | 534 | QSVLTQPPSVSGAPGQRVTISCTGSSSNIGAGYNVYWYQQLPGTAPKLLI YGNSNRPSGVPDRFSGSKSGTSASLAITGLQAEDEADYYCQSYDNSLSA HVVFGGGTKVTVL |
| P1R31-A10_VL | 535 | QSALTQPASVSGSPGQSITISCTGTSSDVGGYNYVSWYQQHPGKAPKLMI SEVSNRPSGVSNRFSGSKSGNTASLTISGLQAEDEADYYCSSYTSSTTLVF GGGTQLTVL |
| P1R31-B8_VL | 536 | QSALTQPASVSGSPGQSITISCSGTSSDVGGYNYVSWYQQHPGKVPKLLI YDVTNRPSGVSNRFSGSKSGNTASLTISGLQAEDEADYYCSSYTRSTTLV FGGGTQLTVL |
| P1R31-C8_VL | 537 | QSVLTQPPSVYAAPGQKVTISCSGSSSNIGKNYVSWYQQLPGTAPKLLIY DNNKRPSGIPDRFSGSKSGTSATLGITGLQTGDEADYYCGTWDSSLSAVV FGGGTKVTVL |
| P1R31-E1_VL | 538 | QSVLTQPPSASGTPGQRVTISCSGSSSNIGSNPVNWYQQLPGTAPKLLIYS NNQRPSGVPDRFSGSKSGTSASLAISGLQSEDEADYYCAAWDDSLNGPV FGGGTKVTVL |
| P1R31-E10_VL | 539 | QSALTQPASVSGSPGQSITISCTGASGDVGGYNYVSWYQQYPGKAPKLII YDVNKRPSGVSNRFSGSKSDNTASLTISGLQAEDEADYYCSSYTSSRTLV FGGGTKVTVL |
| P1R31-F7_VL | 540 | QSVVTQPPSVSGAPGQRVTISCTGSSSNIGAGYDVHWYQQLPGTAPKLLI YGNSNRPSGVPDRFSGSKSGTSASLAITGLQAEDEADYYCQSYDSSLSGY VVFGGGTQLTVL |
| P1R31-G8_VL | 541 | DIVMTQSPDSLAVSMGERLSINCKSSQSVLYICSNKNFLAWYQQKPGQPP KMLIYWAYTRESGVPDRFSGSGSGTDFTLIISILQAEDVAVYYCQQYYST PLTFGGGTKVEII |
| P1R31-H11_VL | 542 | QAVVTQPPSVSKGLRQTATLTCTGNSNNVGNYGAAWLQQHQGHPPKLL SYRNNNRPSGISERLSASRSGNTASLTITGLQPEDEADYYCAAWDSSLRA WVFGGGTQLTVL |
| P1R32-A10_VL | 543 | AIQLTQSPSTRFASVGDRVTITCRASQSLSSWLAWYQQKPGKAPKLLIYK ASSLESGVPSRFSGSGSGTEFTLTISSLQPDDFATYYCQQYNSFYTFGQGT KVEIK |
| P1R32-A6_VL | 544 | DVVMTQSPLSLPVTLGQPASISCRSSQSLVHSDGNTYLNWFQQRPGQSPR RLIYKVSNRDSGVPDRFSGSGSGTDFTLKISRVEAEDVGVYYCMQGTHW PPITFGQGTKLEIK |
| P1R32-C12_VL | 545 | QSALTQPPSASGSPGQSVTISCTGTSSDVGGYNYVSWYQQHPGKAPKLM IYEVSKRPSGVPDRFSGSKSGNTASLTVSGLQAEDEADYYCSSYAGSHV VFGGGTQLTVL |
| P1R32-C8_VL | 546 | QSVLTQPPSVSGAPGQRVTISCTGSSSNIGAGYDVHWYQQLPGTAPKLLI YGNSNRPSGVPDRFSGSKSGTSASLAITGLQAEDEADYYCQSYDSSLSGP SVVFGGGTKLTVL |
| P1R32-E9_VL | 547 | QSALIQPASVSGFPGQSITVSCTGTSSDVGSYNYISWYQQHPGKAPKLMI YDVSKRPSGVSNRFSGSKSGNTASLTISGLQAEDEADYYCSSFAGTSYVL GTGTKVTVL |
| P1R32-F10_VL | 548 | QSVVTQPPSVSGAPGQRVTISCTGSSSNIGAGYDVHWYQQLPGTAPKLLI YGNSNRPSGVPDRFSGSKSGTSASLAITGLQAEDEADYYCQSYDSSLSVV FGGGTQLTVL |
| P1R32-H11_VL | 549 | QAVLTQPPSASGTPGQRVTISCSGSSSNIGSNTVNWYQQLPGTAPKLLIYS NNQRPSGVPDRFSGSKSGTSASLAISGLQSEDEADYYCAAWDDSLNGPV FGGGTQLTAL |

**Table 19.**

| Name | SEQ ID NO | Sequence |
|---|---|---|
| P2R31-H3_VL | 550 | DIQMTQSPSTLSASVGDRVTITCRASQGISSWLAWYQQKPGKAPKLLIYK ASSLQSGVPSRFSGSGSGTEFTLTISSLQPEDFATYYCQQANSAPLTFGQG TKVEIK |
| P1R21-A7_VL | 551 | DIVMTQSPDSLAVSLGERATINCKSSQSLLYSSNNKTYFAWYQQRPGQPP KLLIYWASTRESGVPDRFSGSGSTTDFTLTITSLQAEDVAIYYCQQYYSPL LTFGGGTKVEIK |
| P1R21-B12_VL | 552 | SYELTQPPSASGTPGQRVTISCSGSSSNIGSNTVNWYQQLPGTAPKLLIYS NNQRPSGVPDRFSGSKSGTSASLAISGLQSEDEADYYCAAWDDSLNAVV FGGGTQLTAL |
| P1R21-B6_VL | 553 | QSALTQPASVSGSPGQSITISCTGTSRDVGGYNYVSWYQQHPGKAPKLII YDVTNRPSGVSDRFSASKSANTASLTLSGLLPEDEADYYCSSFTTSRTLV FGGGTKVTVL |
| P1R21-B9_VL | 554 | QSVLTQPPSASGTPGQRVTISCSGSSSNIGSNTVNWYQQLPGTAPKLLIYS NNQRPSGVPDRFSGSKSGTSASLAISGLQSEDEADYYCAAWDDSLNGVV FGGGTQLTAL |
| P1R21-C12_VL | 555 | QSALTQPASVSGSAGQSITISCTGTSSDVGGYNSVAWYQQHPGKAPKLM IFDVRNRPSGVSYRFFGSKSGNTASLTISGLQSDDEADYYCSSFTRSRTW VFGGGTKVTVL |
| P1R21-D11_VL | 556 | QTVVTQEPSLTVSPGGTVTLTCASSTGAVTSGYYPNWFQQKPGQAPRAL IYSTSNKHSWTPARFSGSLLGGKAALTLSGVQPEDEAEYYCLLYYGGAQ VFGGGTQLTVL |
| P1R21-D9_VL | 557 | QSALTQPASVSESPGQSITISCTGTSSDVGGYNFVSWYQQHPGKAPKLMI FGVSERPSGVSNRFSGSKSGNTASLIISGLQAEDEADYYCSSFTSSRTLVF GGGTQLTVL |
| P1R21-E9_VL | 558 | QAGLTQPPSASGTPGQRVTISCSGSSSNIGSYTVNWYQQLPGTAPKLLIFG KNQRPSGVPDRFSGSKSGTSASLSISGLQSEDEADYYCATWDDSLNGLVF GGGTKLTVL |
| P1R21-F11_VL | 559 | QSALTQPASVSGSPGQSITISCTGTSSDVGGYNYVSWYQQHPGKAPKLMI YEVSNRPSGVSNRFSGSKSGNTASLTISGLQAEDEADYYCSSYTSSSTLVF GGGTQLTVL |
| P1R21-F5_VL | 560 | QSVVTQPPSMSAAPGQKVTIPCSGSDSNIGRYFVSWYQHVPGTAPKLLIF DTDKRPSGVPDRFSASKSGTSATLGIAGLQSGDEADYYCLTWDSNLSAA VFGGGTKVTVL |
| P1R21-H10_VL | 561 | DIVMTQSPDSLAVSLGERATINCKSSQSVLYSSNNKNYLAWYQQKPGQP PKLLIYWASTRESGVPDRFSGSGSGTDFTLTISSLQAEDVAVYYCQQYYS TPLTFGQGTKVDIK |
| P1R21-H2_VL | 562 | QSVLTQPPSVSGAPGQRVTISCTGSSSNIGAGYDVHWYQQLPGTAPKLLI YGNSNRPSGVPDRFSGSKSGTSASLAITGLQAEDEADYYCQSYDSSLSVV FGGGTKLTVL |
| P1R22-A1_VL | 563 | AIRMTQSPSTLSASVGDRVTITCRASQSISSWVAWYQQKPGKAPKLLIYK ASSLESGVPSRFSGSGSGTDFTLTISSLQPDDFATYYCQQYNTSPTFGGGT KVEIK |
| P1R22-A3_VL | 564 | QSVVTQPPSVSGAPGQRVTISCTGRSSNLGAGHDVHWYQQVPGTAPKLL IYGNTNRPSGVPERFSGSKSGTSASLVISGLQAEDEADYYCQSYDSSLKL VFGGGTQLTVL |
| P1R22-B10_VL | 565 | QPVLTQPPSASGTPGLTVTISCSGSRSNIGNYLVHWYQQVPGTAPKLLIY NNNQRPSGVPDRFSGSKSGTSASLAISGLRSEDEASYYCATWDANLSGV VFGGGTQLTVL |
| P1R22-B2_VL | 566 | QAGLTQPPSASGTPGQRVTISCSGTRPNIGGYTVNWYRQLPGTAPRLLIY SNSQRPSGVPDRFSGSKSGTSASLAISGLQSEDEADYYCATWDDSLNGV VFGGGTQLTVL |
| P1R22-B4_VL | 567 | QAVLTQPPSASGTPGQRVTISCSGSNSNIGSYSVNWYQQLPGTAPKLLIHS NNQRPSGVPDRFSGSKSGTSASLAISGLQSEDEADYYCAAWDDRLNGVV FGGGTKLTVL |
| P1R22-C10_VL | 568 | QPVLTQPPSASGTPGQSVTLSCSGSSSNIGSFHVNWYQQFPGMAPKLLIY SDNQRPSGVPDRFSGSKSGTSASLAISGLRSEDEADYYCASWDDSLKGPV FGGGTELTVL |
| P1R22-C11_VL | 569 | QAVLTQPPSVSGAPGQRVTISCTGGSSNIGAGYDVHWYQQLPGTAPKLLI YGNSNRPSGVPDRFSGSKSGTSASLAITGLQAEDEADYYCQSYDSSLSGP VVFGGGTKLTVL |
| P1R22-C2_VL | 570 | QSALTQPASVSGSPGQSITISCTGTSSDVGGYNYVSWYQQHPGKAPKLMI YDVSNRPSGVSNRFSGSKSGNTASLTISGLQAEDEADYYCSSYTSSSTVV FGGGTKLTVL |
| P1R22-C6_VL | 571 | DIVMTQSPDSLAVSLGERATINCKSSQSVLYRANNKNYLAWYQQRPGQP PRLLTYWASTRESGVPDRFSGSGSGTDFTLTISSLQAEDVAVYYCQQYYS TPTFGQGTKVEIK |
| P1R22-D3_VL | 572 | QPVLTQPPSASGTPGQRVTISCSGSSSNIGSNTVNWYQQLPGTAPKLLIYS NNQRPSGVPDRFSGSKSGTSASLAISGLQSEDEADYYCAAWDDSLNGPV FGGGTQLTVL |
| P1R22-E1_VL | 573 | DIVMTQSPDSLAVSLGERATINCKSSQSVLYSSNNKNYLAWYQQKPGQP PKLLIHWASTRESGVPDRFSGSGSGTDFTLTISSLQAEDVAVYYCQQYYS SPLTFGGGTKVDIK |
| P1R22-E2_VL | 574 | QPVLTQPPSTSGTPGQRVTISCSGSSSNIGTYTVNWYRQLPGTAPKLLIYT NNKRPSGVPDRFSGSKSGTSASLAISGLQSEDEADYYCAAWDDSLNGPR FGGGTQLTVL |
| P1R22-F11_VL | 575 | EIVLTQSPATLSLSPGERATLSCRASQSVSSYLAWYQQKPGQAPRLLIYD ASNRATGIPARFSGSGSGTDFTLTISSLEPEDFAVYYCQQRSNWPPTFGQG TKLEIK |
| P1R22-G10_VL | 576 | QSVVSQPPSVSGAPGQRVIISCSGSSFNIGAGYDVHWCQQLTATHSKLLM YGNSNRPSGVPDRFFGSKSATSASLAITGLQAEDEADYYCQSYDSSLSVV FGGGTKVDVL |
| P1R22-G5_VL | 577 | DVVMTQSPLSLSVTLGQPASISCRSSQSLISGGGDTYLHWFQQRPGQPPR RLIYKVSNRDSGVPDRFSGSGSGTDFTLKISRVEADDVGVYYCLQGTHRP PTFGGGTKLEIK |

**Table 20.**

| Name | SEQ ID NO | Sequence |
|---|---|---|
| P1R22-H1_VL | 578 | QSALTQPASVSGSPGQSITISCTGTSSDVGGDKYVSWYQQHPGKAPKLMI YDVNDRPSGVSDRFSGSKSGNTASLTISGVQPEDEADYYCSSYTSRRTPY VFGTGTKLTVL |
| P1R22-H8_VL | 579 | DIVMTQTPLSLPVTLGQPASISCRSSQSLAHSDGNTYLNWFQQRPGQSPR RLIYKVSNRDSGVPDRFSGSGSGTDFTLKISRVEAEDVGVYYCMQGTHW PWTFGQGTKLEIK |
| P1R31-A4_VL | 580 | RPVLIQPPSASGTPGQRVTISCSGSSSNIGSNTVNWYQQLPGTAPKLLIYS NNQRPSGVPDRFSGSKSGTSASLAISGLQSEDEADYYCAAWDDSLNGVV FGGGTKVTVL |
| P1R31-B1_VL | 581 | QTVVTQEPSLTVSPGGTVTLTCASSTGAVTNGYYPNWFQQKPGQAPRAL IYSTSNKHSWTPARFSGSLLGGKAALTLSGVQPEDEAEYYCLLYYGGAQ VFGGGTKVTVL |
| P1R31-B11_VL | 582 | DIVMTQSPLTLPVTLGQPASISCRSSQSLLYTDGNTYLNWFQHRPGQSPR RLIYKVSKRVSGVPDRFSGSGSGTDFTLKISRVEAEDVGVYYCMQGTQR PPTFGQGTRLEIK |
| P1R31-B2_VL | 583 | QAGLTQPPSASGTPGQRVTISCSGTRPNIGGYTVNWYRQLPGTTPTLLIYS NTQRPSGVPDRFSGSKSGTSASLAISGLQSEDEADYYCATWDDSLNGVV FGGGTKVTVL |
| P1R31-B4_VL | 584 | QPVLTQPLSVSVALGQTARITCGGNNIGSKNVHWYQQKPAQAPVLVIYR DINRPSGIPERFSGSNSGNTATLTISRAQAGDEADYYCQVWDSSTFVFGT GTKVTVL |
| P1R31-B9_VL | 585 | QSVLTQPPSVSAVPGQKVAISCSGSSSNIGSHYVSWYQQFPGTAPKLLIYE NNKRPSGIPDRFSASKSGTSATLGITGLQTGDEADYYCATWDSSQSGGIF GGGTKVTVL |
| P1R31-C11_VL | 586 | DVVMTQSPLSLPVTLGQPASISCRSSQSLVHRDGNTYLNWFQQRPGQSP RRLIYKVSNRDSGVPDRFSGSGSGTDFTLKISRVEAEDVGVYYCMQGSH WPPITFGQGTRLEIK |
| P1R31-C2_VL | 587 | DIVMTQSPDSLAVSLGERATINCKSSQSVLYSSNNKNYLAWYQQKPGQP PKLLIYWASTRESGVPDRFSGSGSGTDFTLTISSLQAEDVAVYYCQQYYS TPLTFGGGTKVEIK |
| P1R31-D11_VL | 588 | QAGLTQPPSASGSPGQSVTISCTGTSSDIGGYNFVSWYQQHPGKAPKLIIS EVTKRPSGVPDRFSGSKSGNTASLTVSGLQAEDEADYYCSSYAGNKLVF GGGTKVTVL |
| P1R31-D6_VL | 589 | QSALTQPPSASGSPGQSVTISCTGTSSDIGAYKFVSWYQQHPGKAPKLIIS EVSRRPSGVPDRFAASKSGNTASLTVSGLQPEDEAYYYCSSYAGSNNWV VFGGGTQLTVL |
| P1R31-D9_VL | 590 | HPVLTQPPSVSGTPGQRVTISCCGSSSNIGSNPVNWYQQLPGTAPKLLIYS NNQRPSGVHDRFSGSKSGTSASLAISGLQSEDEADYYCAAWDDSLNGPV FGGGTQLTAL |
| P1R31-F1_VL | 591 | DIVMTQSPDSLAVSLGERATINCKSSQSVLYSSNNKNYLAWYQQKPGQP PKLLISWASTRESGVPDRFSGSGSGTDFTLTISSLQAEDVAVYYCQQYYS PLLTFGGGTKLEIK |
| P1R31-F4_VL | 592 | DIVMTQSPDSLAVSLGERATINCKSSQSVLYSSNNKNDLAWYQQKPGQP PKLLIYWASTRESGVPDRFSGSGSGTDFTLTISSLQAEDVALYYCQQYYT TPPTFGQGTRLEIK |
| P1R31-F5_VL | 593 | QAGLTQPPSASGTPGQRVTISCSGGRSNIGSNIVNWFQQLPGTAPKLVMY TNNQRPAGVPDRFSGSKSGTSASLAIRGLQSEDEADYYCATWDGSLNGV VFGGGTKLTVL |
| P1R31-F8_VL | 594 | QSVLTQPPSASGTPGQRVTISCSGSSSNIGSNTVNWYQQLPGTAPKLLIYS NNQRPSGVPDRFSGSKSGTSASLAISGLQSEDEADYYCAAWDDSLNGVV FGGGTQLTAL |
| P1R31-G6_VL | 595 | DIVMTQSPLSLPVTPGEPASISCRSSQSLLHSNGNNYLDWYLQKPGQSPQ LLIYLGSNRASGVPDRFSGSGSGTDFTLKISRVEAEDVGVYYCMQALQTP LTFGGGTKVEIK |
| P1R31-H4_VL | 596 | QSALTQPASVSGSPGQSITISCTGTSSDVGNYNYVSWYQQHPGKVPKLMI YEVNNRPSGVSNRFSGSKSGNTASLTISGLQAEDEADYYCTSFRSSNTLV FGGGTQLTVL |
| P1R32-A9_VL | 597 | SYELTQPPSVSKDLRQTATLTCSGDSNNIGKYGAAWLQQHPGHPPKLLL YRNNNRPSGISERFSAARSGNTASLTITGLQPDDEADYYCSTWDSNLRGY VFGPGTKVTVL |
| P1R32-B2_VL | 598 | QPVLTQPPSVSGTPGQRVTISCSGSSSNIGTNSVNWYQQLPGTAPKLLIYR SNQRSSGVPERFSASKSGTSASLAISGLQSEDEADYYCASWDDSLNGPAF GGGTKVTVL |
| P1R32-B5_VL | 599 | QPVLTQPPSTSGTPGQRVTISCSGSSSNIGKYPVNWYQQLPGMAPKLLMY TDNQRPSGVPDRFSGSKSGTSASLAISGLQSEDEGDYYCAAWDDSLNVV FGGGTKVTVL |
| P1R32-C1_VL | 600 | QPVLTQPPSASGTPGQRVTISFSGSSSNIGSNKVNWYQQLPGTAPKLLIYS NNQRSSGVPDRFSGSKSGTSASLAISGLQSEDEADYYCAAWDDSLNAVV FGGGTQLTAL |
| P1R32-C11_VL | 601 | QSVLTQPPSASGTPGQSVTLSCSGSSSNIGSFHVNWYQQFPGMAPKLLIY SDNQRPSGVPDRFSGSKSGTSASLAISGLRSEDEADYYCASWDDSLKGPV FGGGTQLTVL |
| P1R32-D4_VL | 602 | DVVMTQSPLSLPVTLGQPASISCRFSQSLVHLDGNTYLNWFQQRPGQSPR RLIYKVSNRDSGVPDRFSGSGSGTDFTLKISRVEAEDVGVYYCMQGSHW PPITFGQGTRLEIK |
| P1R32-D7_VL | 603 | QSALTQPASVSGSPGQSITISCTGTSSDVGGYNYVSWYQQHPGKAPKLMI YEVSNRPSGVSNRFSGSKSGNTASLTISGLQAEDEADYYCSSYTSSSTPV VFGGGTQLTVL |
| P1R32-E4_VL | 604 | QAGLTQPPSVSKGLRQTATLTCTGNSNNVGNQGAAWLQQHQGHPPKLL SYRNNNRPSGISERFSASRSGNTASLTITGLQPEDEADYYCSAWDSSLSA VVFGGGTQLTVL |
| P1R32-E6_VL | 605 | DIVMTQSPDSLAVSLGERATINCKSSQSVLYSSNNKNYLAWYQQKPGQP PKLLIYWASTRESGVPDRFSGSGSGTDFTLTISSLQAEDVAVYYCQQYYS TPLTFGGGTKVEIK |

**Table 21.**

| Name | SEQ ID NO | Sequence |
|---|---|---|
| P1R32-E8_VL | 606 | QSVLTQPPSVSGAPGQRVTISCTGSSSNIGAGYDVHWYQQLPGTAPKLLI YGNSNRPSGVPDRFSGSKSGTSASLAITGLQAEDEADYYCQSYDSSLSVV FGGGTKVTVL |
| P1R32-F1_VL | 607 | QSALTQPASVSGSPGQSITISCTGTSSDVGGYDYVSWYQQHPGKAPKLMI YEVSNRPSGVSDRFSGSKSGNTASLTISGLQAEDEGDYYCSSYRRSSVLV FGGGTKVTVL |
| P1R32-F7_VL | 608 | QSALTQPASVSGSPGQSSTMSCTGTSSDVGGYNYVSWYQQHPGKAPKLI IYDVSNRPSGVSNRFSGSKSGNTASLTISGLQAEDEADYYCSSYRRSNIVV FGGGTKVTVL |
| P1R32-F9_VL | 609 | DIVMTQSPSSLSASVGDRVTITCRARQGISNYLAWYQQKPGKVPKLLISG ASTLQSGVPSRFSGSGSGTEFTLTISSLQPEDVATYYCQKYNNAPLAFGG GTKLEIK |
| P1R32-G10_VL | 610 | QSVVTQPPSVSGAPGQRVTISCTGSSSNIGAGYDVHWYQQLPGTAPKLLI YGNNNRPSGVPDRFSGSKSGTSASLAITGLQAEDEADYYCQSYDSSLSV VFGGGTKLTVL |
| P1R32-G2_VL | 611 | QYSLTHPASVSGSSGQSITISCTGTSSDVGGDKYVFWWQQHPAKKPNLM IYDVNDRPSGVSDRFFGSKYGNTASLTIFGVQPEDEADYYCSSYTSRRTP YVFGTGTKLTVL |
| P1R32-H2_VL | 612 | QSVLTQPPSVSGAPGQRVTISCTGSSSNIGANYGVHWFQQFPGTAPKLLI YGNNRRPSGVPDRFSASKSGTSASLAITGLQADDEAEYHCQSFDSSLSAV VFGGGTKLTVL |
| P1R32-H5_VL | 613 | EIVLTQSPLSLPVTLGQPASISCRSSQSLLHTDGNTYLNWFQQRPGQSPRR LIYKVSNRDSGVPDRFSGSGSGTDFTLKISRVEAEDVGIYYCMQGTLWPP ITFGQGTRLEIK |
| P1R32-H6_VL | 614 | QSALTQPASVSGSPGQSITISCTGTSSDVGGYNYVSWYQQHPGKAPKLMI YEVSNRPSGVSNRFSGSKSGNTASLTISGLQAEDEADYYCSSYTSSSTLVF GGGTKVTVL |
| P1R32-H9_VL | 615 | SPMMTQSPATLSVSPGERATLSCRASQSVSSNLAWYQQKPGQAPRLLIY GASTRATGIPARFSGSGSGTEFTLTISSLQSEDFAVYYCQQYNNWPLTFG GGTKLEIK |
| P2R21-E2_VL | 616 | DIVMTQSPLSLPVTPGEPASISCRSSQSLLHSNGKNYLDWYLQKPGQSPQ LLIYKVSNRASGVPDRFSGSGSGTDFTLKISRVEAEDVGVYYCMQALQT PWTFGQGTKVEIK |
| P2R22-E6_VL | 617 | EIVLTQSPATLSLSPGERATLSCRASQSVSTNLAWYQQKPGQAPRLLIYD ASTRATGIPARFSGSGSGTDFTLTISSLEPEDFAVYYCQQRGSWPLTFGQG TKVEIK |
| P2R31-A2_VL | 618 | DIQMTQSPSTLSASVGDRVTITCRASQDISNWLAWYQQKPGKAPKLLIY KASSLQSGVPSRFSGSGSGTEFTLTISSLQPEDFATYYCQQADSAPLTFGQ GTKVEIK |
| P2R31-A3_VL | 619 | DIVMTQSPLSLPVTPGEPASISCRSSQSLLHSNGYNYLDWYLQKPGQSPQ LLIYKVSNRASGVPDRFSGSGSGTDFTLKISRVEAEDVGVYYCRQATQFP LTFGQGTKVEIK |
| P2R31-B4_VL | 620 | EIVLTQSPATLSLSPGERATLSCRASQSVSSYLAWYQQKPGQAPRLLIYA ASSRATGIPARFSGSGSGTDFTLTISSLEPEDFAVYYCQQYSKWPITFGQG TKVEIK |
| P2R31-B7_VL | 621 | EIVLTQSPATLSLSPGERATLSCRASQSVSSYLAWYQQKPGQAPRLLIYG ASTRATGIPARFSGSGSGTDFTLTISSLEPEDFAVYYCQQYGTSPLTFGQG TKVEIK |
| P2R31-B9_VL | 622 | EIVLTQSPATLSLSPGERATLSCRASQSISSYLAWYQQKPGQAPRLLIYGA SSRATGIPARFSGSGSGTDFTLTISSLEPEDFAVYYCQQYNRSPLTFGQGT KVEIK |
| P2R31-C5_VL | 623 | EIVLTQSPATLSLSPGERATLSCRASQSVSSNLAWYQQKPGQAPRLLIYA ASNRATGIPARFSGSGSGTDFTLTISSLEPEDFAVYYCQHYGKSPITFGQG TKVEIK |
| P2R31-C8_VL | 624 | EIVLTQSPATLSLSPGERATLSCRASQSVSSNLAWYQQKPGQAPRLLIYG ASNRATGIPARFSGSGSGTDFTLTISSLEPEDFAVYYCQQYYSSPVTFGQG TKVEIK |
| P2R31-C9_VL | 625 | EIVLTQSPATLSLSPGERATLSCRASQSVSSNLAWYQQKPGQAPRLLIYD ASNRATGIPARFSGSGSGTDFTLTISSLEPEDFAVYYCQQYSSPITFGQG TKVEIK |
| P2R31-D4_VL | 626 | DIQMTQSPSTLSASVGDRVTITCRASQGISSWLAWYQQKPGKAPKLLIYK ASSLQSGVPSRFSGSGSGTEFTLTISSLQPEDFATYYCQQLNSAPITFGQGT KVEIK |
| P2R31-E2_VL | 627 | EIVLTQSPATLSLSPGERATLSCRASQSVSTNLAWYQQKPGQAPRLLIYD ASNRATGIPARFSGSGSGTDFTLTISSLEPEDFAVYYCQHYYTSPLTFGQG TKVEIK |
| P2R31-F10_VL | 628 | EIVLTQSPATLSLSPGERATLSCRASQSVSSNLAWYQQKPGQAPRLLIYG ASTRATDIPARFSGSGSGTDFTLTISSLEPEDFAVYYCQQYSSSPVTFGQG TKVEIK |
| P2R31-G10_VL | 629 | EIVLTQSPATLSLSPGERATLSCRASQSVGNYLAWYQQKPGQAPRLLIYG ASNRATGIPARFSGSGSGTDFTLTISSLEPEDFAVYYCKQYSKWPLTFGQ GTKVEIK |
| P2R31-G7_VL | 630 | QSVLTQPPSSVSGAPGQRVTISCSGSSNIGSNTVHWYQQLPGTAPKLLIYR DSQRPSGVPDRFSGSKSGTSASLAITGLQSEDEADYYCAAYDTSLGVVFG GGTKLTVL |
| P2R32-A1_VL | 631 | EIVLTQSPATLSLSPGERATLSCRASQSVSSNLAWYQQKPGQAPRLLIYD ASNRATGIPARFSGSGSGTDFTLTISSLEPEDFAVYYCQQYSSSPVTFGQG TKVEIK |
| P2R32-A2_VL | 632 | DIVMTQSPLSLPVTPGEPASISCRSSQSLVHSNGKTYLDWYLQKPGQSPQ LLIYKVSNRASGVPDRFSGSGSGTDFTLKISRVEAEDVGVYYCMQALQT PWTFGQGTKVEIK |
| P2R32-A7_VL | 633 | EIVLTQSPATLSLSPGERATLSCRASQSVSSYLAWYQQKPGQAPRLLIYG ASSRATGIPARFSGSGSGTDFTLTISSLEPEDFAVYYCQQYGNWPLTFGQ GTKVEIK |

**Table 22.**

| Name | SEQ ID NO | Sequence |
|---|---|---|
| P2R32-B7_VL | 634 | EIVLTQSPATLSLSPGERATLSCRASQSVSSNLAWYQQKPGQAPRLLIYGA SSRATGIPARFSGSGSGTDFTLTISRLEPEDFAVYYCQQYGSSPITFGQGTK VEIK |
| P2R32-D1_VL | 635 | EIVLTQSPATLSLSPGERATLSCRASQSVSSYLAWYQQKPGQAPRLLIYGA SNRATGIPARFSGSGSGTDFTLTISSLEPEDFAVYYCQQYGRSPPTFGQGT KVEIK |
| P2R32-D11_VL | 636 | EIVLTQSPATLSLSPGERATLSCRASQSVSNYLAWYQQKPGQAPRLLIYG ASNRATGIPARFSGSGSGTDFTLTISSLEPEDFAVYYCQQYSNSPLTFGQG TKVEIK |
| P2R32-D12_VL | 637 | EIVLTQSPATLSLSPGERATLSCRASQSVSTYLAWYQQKPGQAPRLLIYG ASSRATGIPARFSGSGSGTDFTLTISSLEPEDFAVYYCQQYNRSPITFGQGT KVEIK |
| P2R32-D5_VL | 638 | QSVLTQPPSSVSGAPGQRVTISCSGSSSNIGSNTVHWYQQLPGTAPKLLIY DNDQRPSGVPDRFSGSKSGTSASLAITGLQSEDEADYYCASWDDSLSVVF GGGTKLTVL |
| P2R32-E11_VL | 639 | DIQMTQSPSTLSASVGDRVTITCRASQSISNDLAWYQQKPGKAPKLLIYK ASTLQSGVPSRFSGSGSGTEFTLTISSLQPEDFATYYWQQYNSFPPTFGQG TKVEIK |
| P2R32-F1_VL | 640 | EIVLTQSPATLSLSPGERATLSCRASQSVSSNLAWYQQKPGQAPRLLIYGA SNRATGIPARFSGSGSGTDFTLTISSLEPEDFAVYYCQQYYSSPPTFGQGT KVEIK |
| P2R32-F11_VL | 641 | EIVLTQSPATLSLSPGERATLSCRASQSVGSNLAWYQQKPGQAPRLLIYG ASNRATGIPARFSGSGSGTDFTLTISSLEPEDFAVYYCQQYGSSPLTFGQG TKVEIK |
| P2R32-G8_VL | 642 | QSVLTQPPSSVSGAPGQRVTISCSGSSSNIGSNYVHWYQQLPGTAPKLLIY DNDKRPSGVPDRFSGSKSGTSASLAITGLQSEDEADYYCAAWDSSLGVV FGGGTKLTVL |
| P2R32-G9_VL | 643 | EIVLTQSPATLSLSPGERATLSCRASQSVSSNLAWYQQKPGQAPRLLIYGA SNRATGIPARFSGSGSGTDFTLTISSLEPEDFAVYYCQQYYSSPPTFGQGT KVEIK |

### Example 2. Conversion of anti-MYCT1 scFv and Fab to Full-Length IgG Form and Production Thereof

### 2-1. Cloning of anti-MYCT1 scFv and Fab into Full-Length IgG Form

Polynucleotides having nucleotide sequences encoding the scFv and Fab antibodies selected in Example 1 were synthesized. The prepared polynucleotides were cloned into animal cell expression vectors (heavy chain expression vector: pcDNA3.4-HC and light chain expression vector: pcDNA3.4-LC). A total of 46 vectors containing polynucleotides encoding heavy chains and light chains for each of the 23 antibody clones in Table 7 were prepared. The vectors prepared for pcDNA3.4-HC and pcDNA3.4-LC each contained an IgG1-type sequence.

### 2-2. Expression and Culture of anti-MYCT1 Antibodies

Expression of the anti-MYCT1 antibodies converted to full-length IgG form from the scFv and Fab of Example 2-1 was performed using ExpiCHO-S^{™} cells (Thermo Fisher, A29127) developed by Thermo, following the protocol of the ExpiCHO^{™} Expression System Kit (Thermo Fisher, A29133). Specifically, ExpiCHO-S cells were cultured at 37°C, ≥80% humidity, 8% CO2, and 120 rpm. On the day of transfection, ExpiCHO-S cells at 6x10⁶ cells/mL cell concentration were diluted and prepared by adding ExpiCHO-S^{™} Expression Medium (Thermo Fisher, A29100-01). The heavy chain vector of Example 2-2 was diluted at 0.5 µg per mL of medium, and the light chain vector of Example 2-2 was diluted at 0.5 µg per mL in OptiPRO^{™} SFM medium (Thermo Fisher, 12309050), and ExpiFectamine^{™} CHO Reagent was diluted in OptiPRO^{™} SFM medium. The vector and ExpiFectamine^{™} CHO mixture were mixed and reacted at room temperature for 1 to 5 minutes, then cultured at 37°C, ≥80% humidity, 8% CO2, and 120 rpm for 20 hours. After 20 hours, ExpiCHO^{™} Enhancer and ExpiCHO^{™} Feed from the ExpiCHO^{™} Expression System Kit (Thermo Fisher, A29133) were added and cultured for 10 days. After culturing, the culture medium of the cells was centrifuged at 4°C and 3,600 rpm for 30 minutes, and the supernatant was separated and stored in a refrigerator.

### 2-3. Confirmation of Expression of anti-MYCT1 Antibodies

The culture status and expression of the anti-MYCT1 antibodies converted to full-length IgG form from the scFv and Fab of Example 2-2 were confirmed by measuring viable cell density and viability, and the titer was measured through Protein A HPLC.

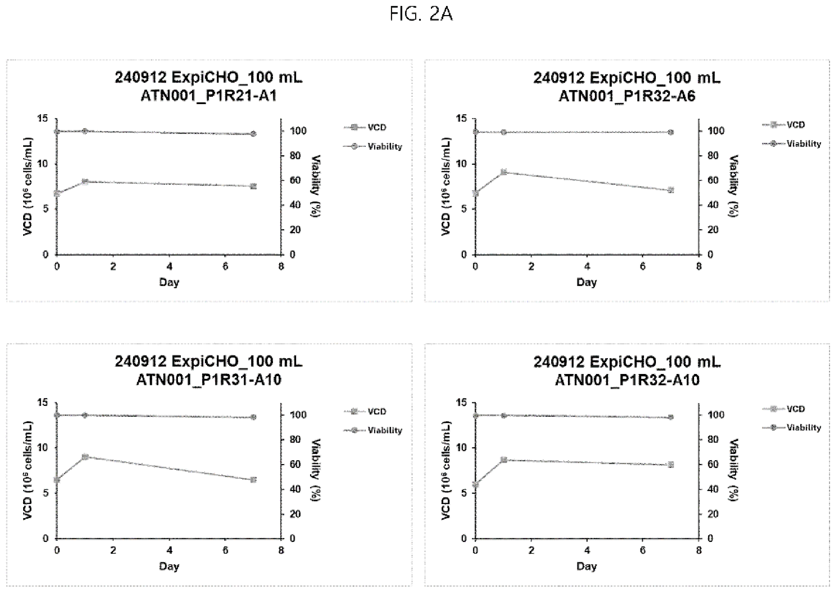

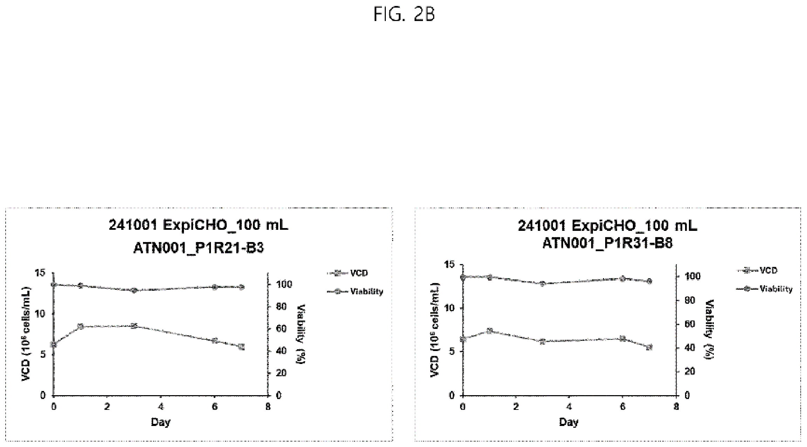

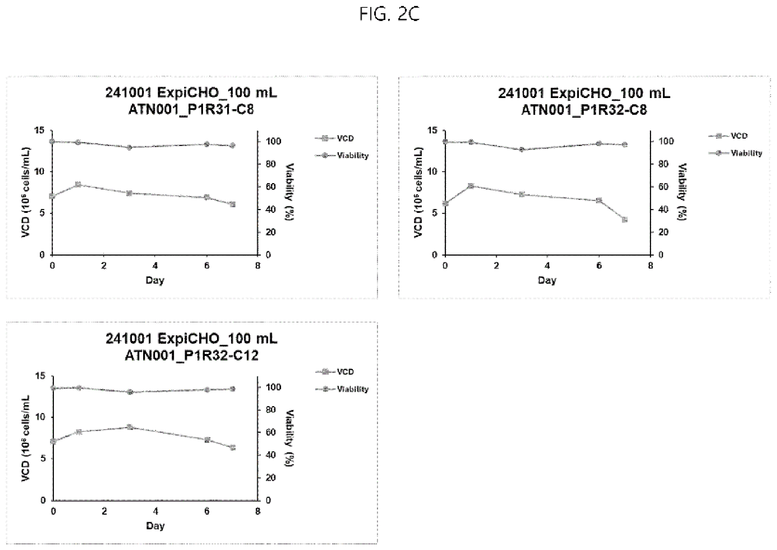

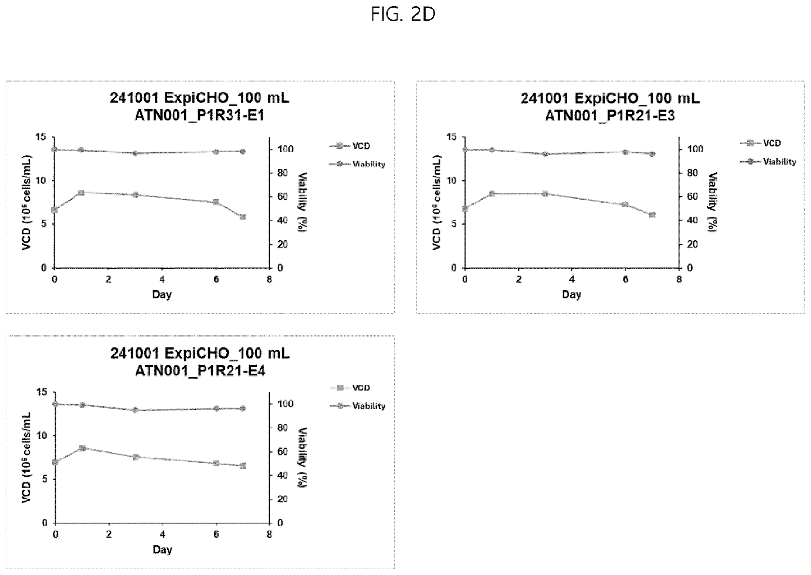

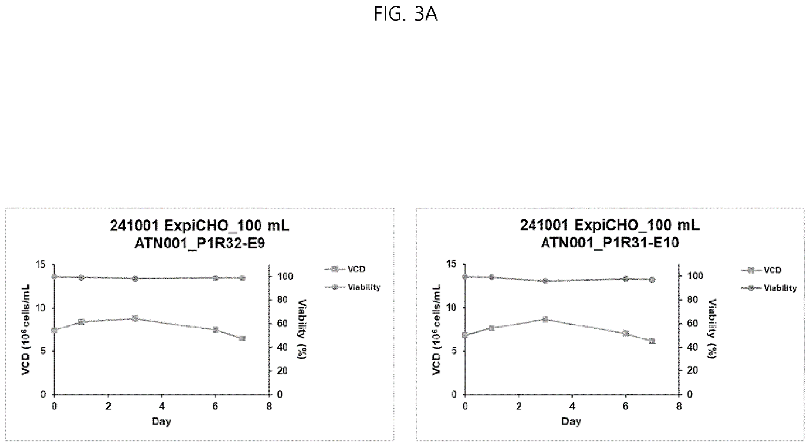

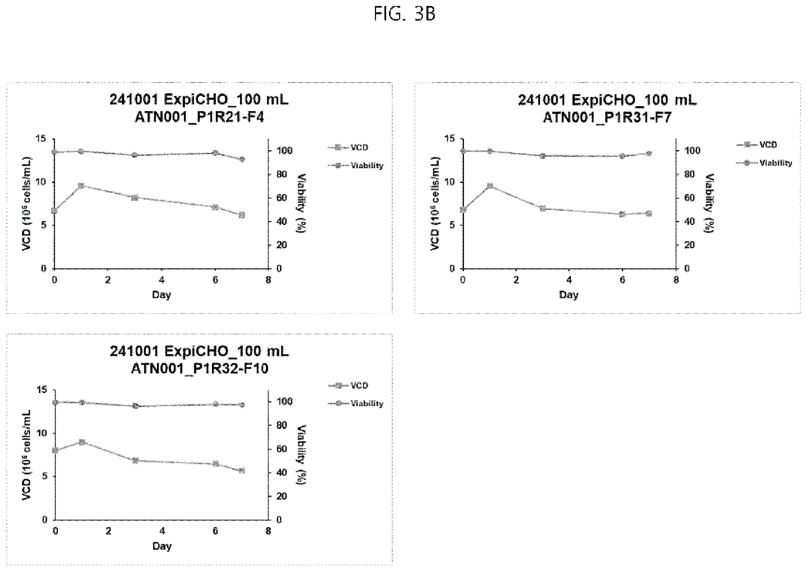

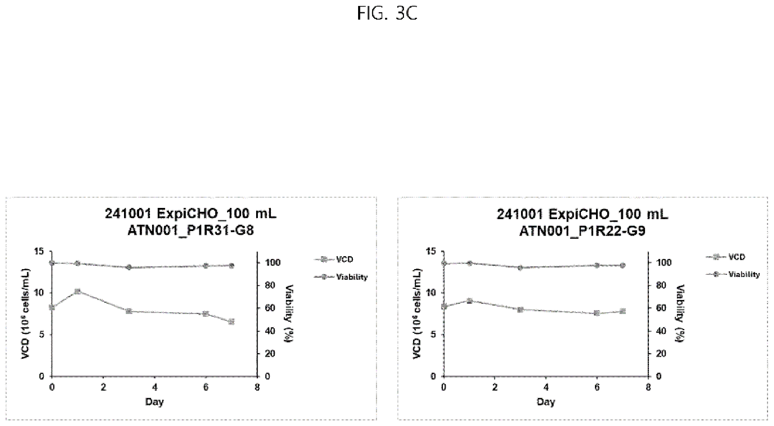

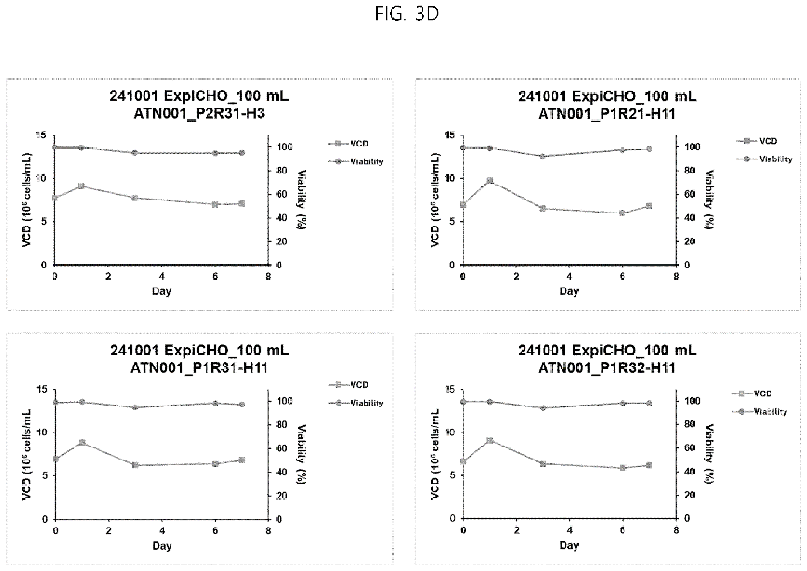

Specifically, culture medium (10 µL) was sampled on days 0, 1, 3, 6, and 7, and viable cell density and viability were measured using an EVE^{™} PLUS apparatus. To measure titer through Protein A HPLC, a TSKgel Protein A-5PW Column was equilibrated with 6 mL of equilibration buffer (20 mM sodium phosphate, pH 7.5) at a flow rate of 2 mL/min. Human IgG at 0.03, 0.06, 0.13, 0.25, 0.5, 1, and 2 mg/mL were each injected (50 µL) into the TSKgel Protein A-5PW Column and equilibration buffer was run at a flow rate of 2 mL/min for 0.5 minutes for binding, then elution buffer (20 mM sodium phosphate, pH 2.5) was run for 0.6 minutes to elute the antibody. A calibration curve was generated from the eluted peak, and it was confirmed that the R^2 value was 0.9998, and the titer was calculated using the corresponding calibration curve. The culture medium stored in Example 2-2 was filtered through a 0.22 µm PES Bottle Top Filter. The filtered sample (50 µL) was run through the TSKgel Protein A-5PW column in the same manner to elute the antibody. The eluted peak was used to calculate the titer of the antibody using the calibration curve, and the results are shown in FIGs. 2A, 2B, 2C, 2D and FIGs. 3A, 3B, 3C, 3D.

FIGs. 2A, 2B, 2C, 2D and FIGs. 3A, 3B, 3C, 3D show the results of measuring viable cell density and viability of ExpiCHO-S cells transfected with vectors containing the sequences of anti-MYCT1 scFv and Fab converted to full-length IgG form.

As shown in FIGs. 2A, 2B, 2C, 2D and FIGs. 3A, 3B, 3C, 3D, it was confirmed that the viable cell density and viability of cells (ExpiCHO-S cells) transfected with vectors containing the sequences of anti-MYCT1 antibodies in Example 2-2 were high. Also, the titer of the expressed antibodies was measured through Protein A HPLC. This indicates that the anti-MYCT1 scFv and Fab converted to full-length IgG form antibodies were well expressed in cells.

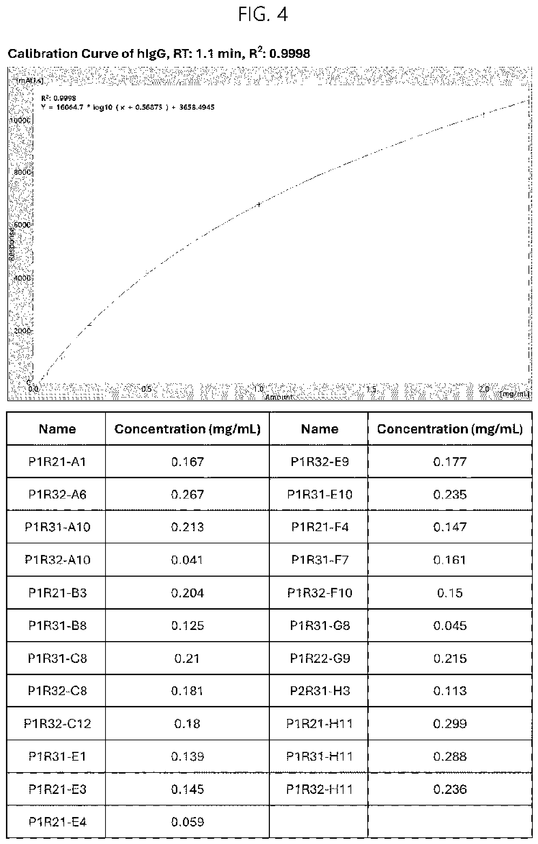

FIG. 4 shows the results of measuring the Protein A HPLC titer of vectors transfected with sequences of anti-MYCT1 scFv and Fab converted to full-length IgG form.

### 2-4: Isolation and Purification of anti-MYCT1 Antibodies

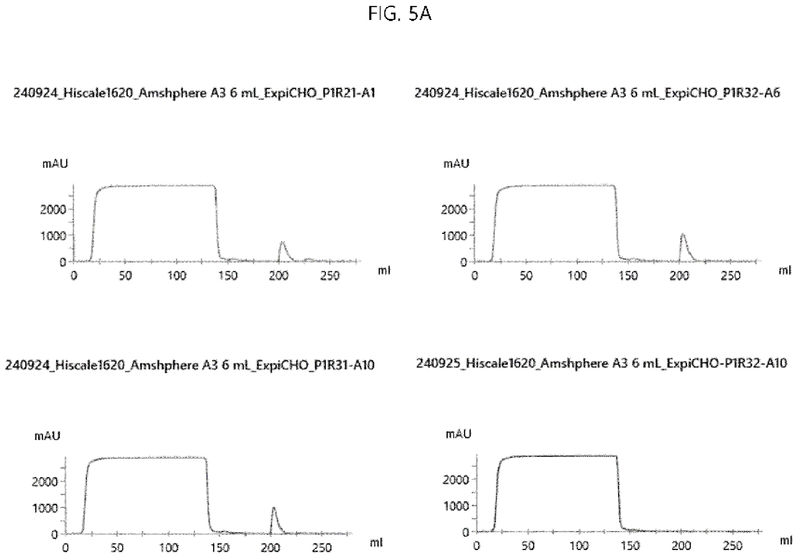

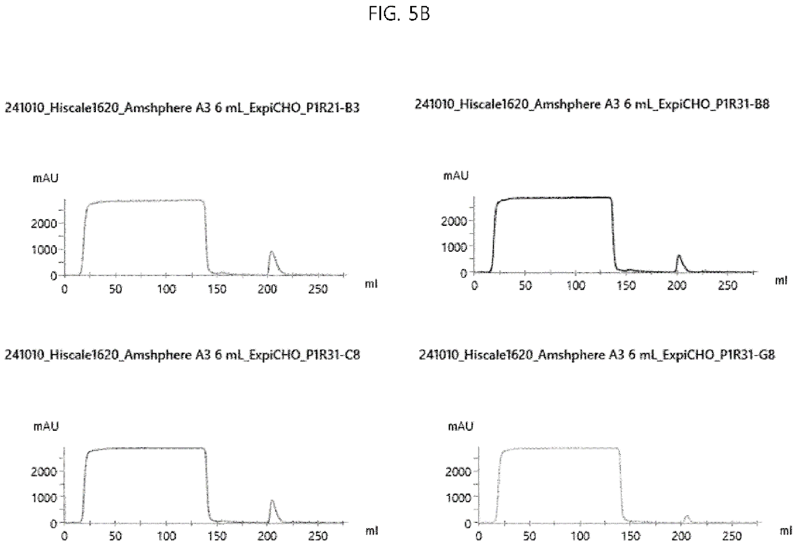

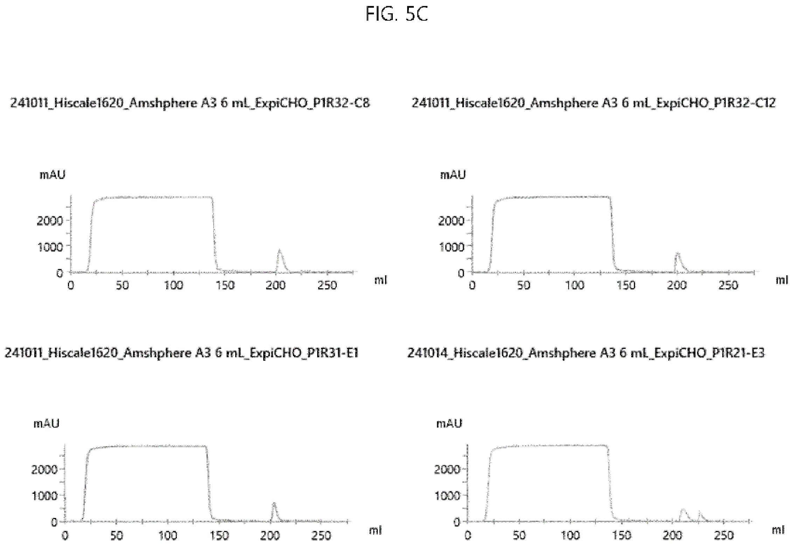

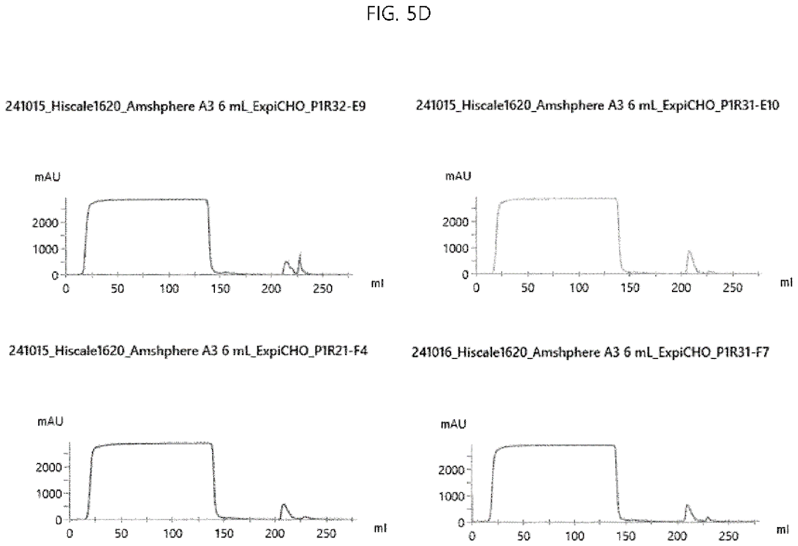

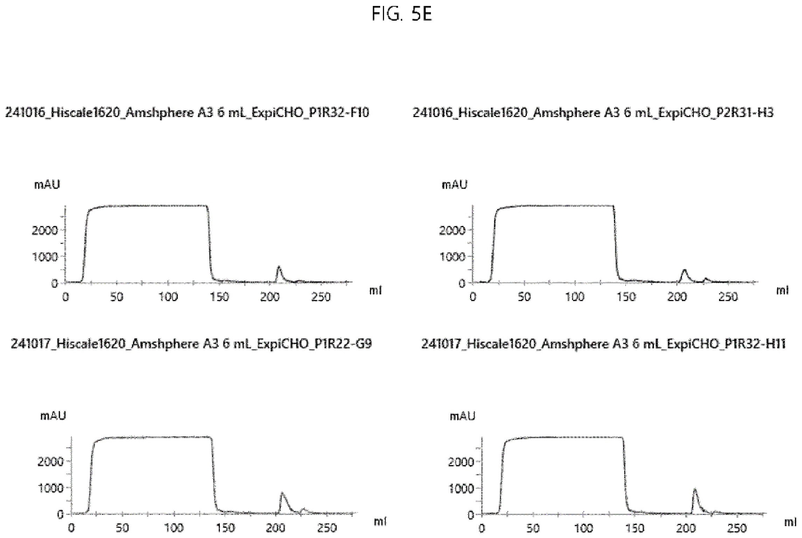

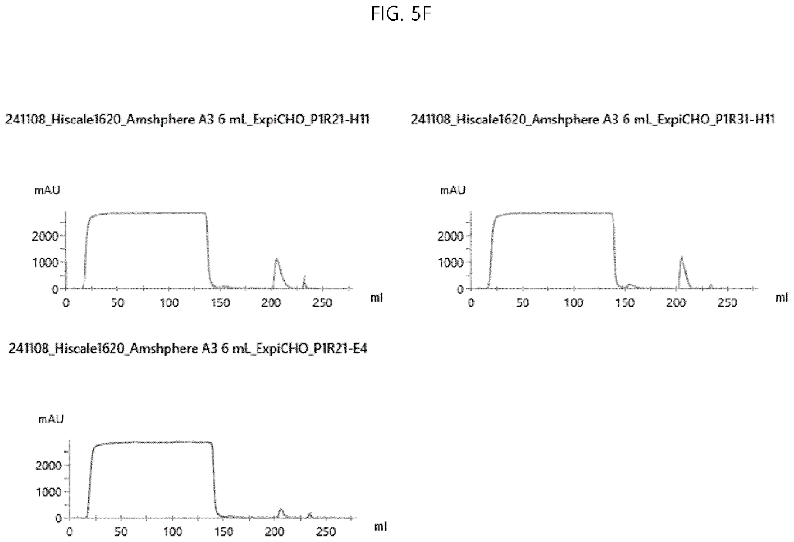

To isolate and purify the anti-MYCT1 antibodies of Example 2-2, the culture medium stored in Example 2-2 was filtered through a 0.22 µm PES Bottle Top Filter. Amsphere A3 resin (5 mL) was packed into a Hiscale 16/20 column and washed with 100 mL of triple-distilled water at a flow rate of 5 mL/min, then equilibrated with 25 mL of equilibration buffer (0.1 M Tris, pH 7.0). The filtered sample was run through the Amsphere A3 column at a flow rate of 1.5 mL/min for binding, then 10 mL of equilibration buffer was run to stabilize the UV 280 nm value to baseline. Wash buffer I (0.1 M Tris, 1 M NaCl, pH 6.0) (15 mL) was run at a flow rate of 5 mL/min to remove impurities, and Wash buffer II (0.1 M Tris, pH 6.0) (15 mL) was run to stabilize the UV 280 nm value to baseline. Elution buffer (0.1 M Glycine, pH 3.5) (25 mL) was run at a flow rate of 1.5 mL/min to elute the antibody, the fraction volume was set to 4 mL, and 600 µL of saturated Tris solution was added to each fraction tube. The eluted antibody was placed in a dilution tube and buffer-exchanged with 3 L of PBS, pH 7.4 buffer at 4°C for 4 hours, then exchanged with 3 L of fresh PBS, pH 7.4 buffer for 16 hours. After filtration with a 0.22 µm PES syringe filter, the concentration was calculated by measuring the absorbance at A280, and the results are shown in FIG. 5.

FIGs. 5A, 5B, 5C, 5D, 5E and 5F show the results of purification and isolation of anti-MYCT1 scFv and Fab converted to full-length IgG form as chromatograms.

As shown in FIGs. 5A, 5B, 5C, 5D, 5E and 5F it was confirmed that the anti-MYCT1 antibodies converted to full-length IgG form from MYCT1 Fab and scFv were well purified and isolated from the cell culture medium. Among the 23 candidate anti-MYCT1 antibodies, only 22 were purified and isolated.

### 2-5. Confirmation of Purity of Purified and Isolated anti-MYCT1 Antibodies

SDS-PAGE and SEC-HPLC were performed to confirm the purity of the anti-MYCT1 antibodies of Example 2-4.

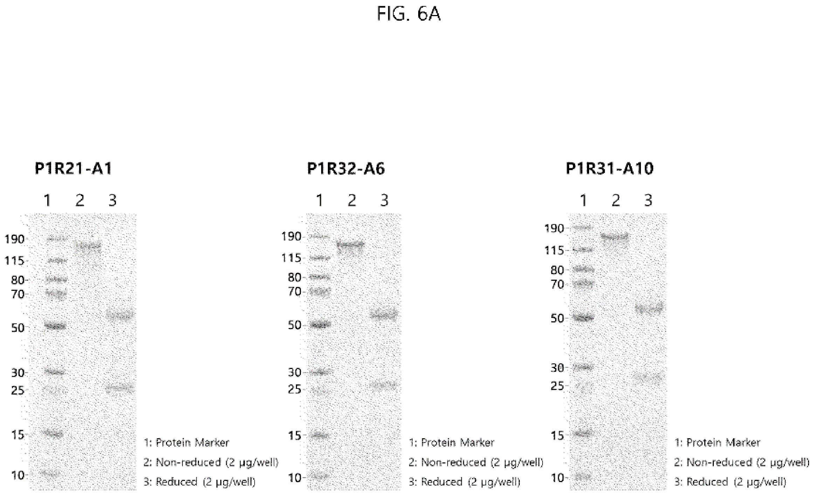

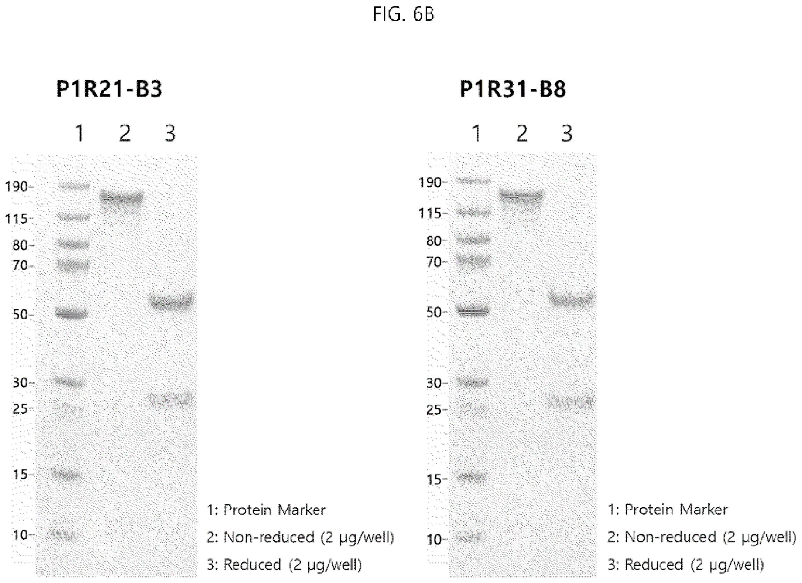

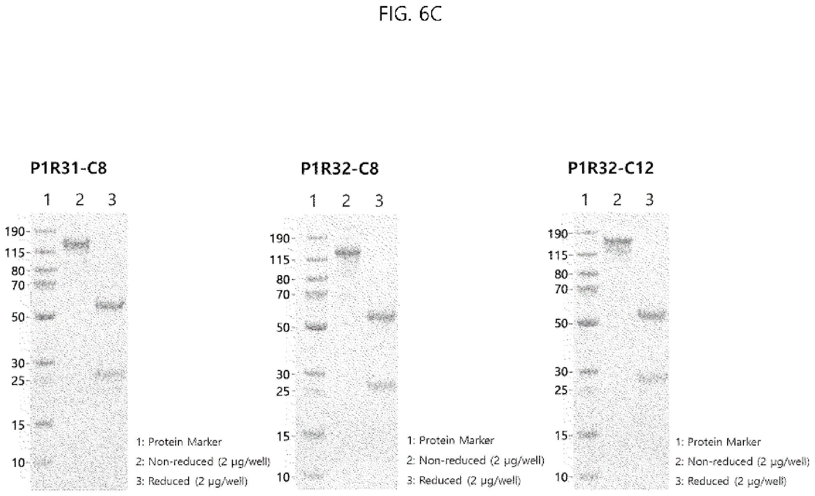

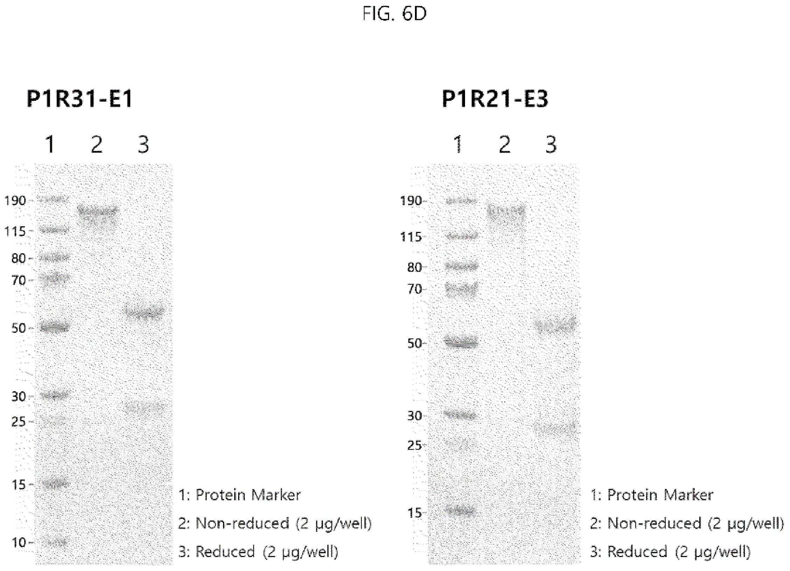

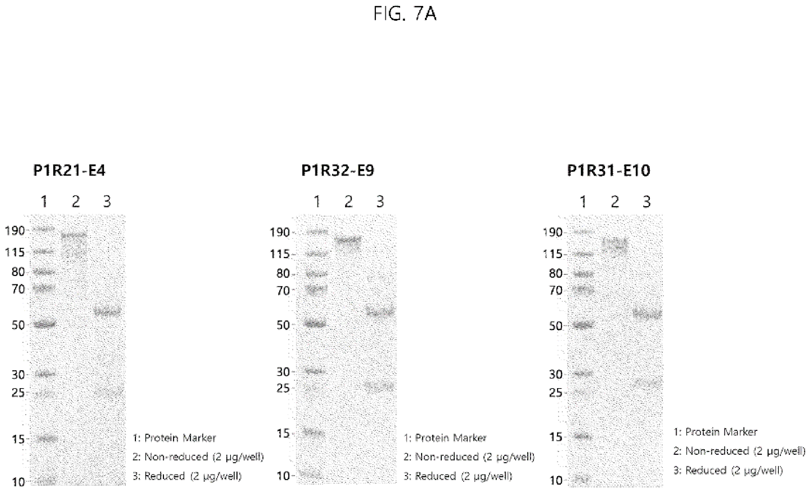

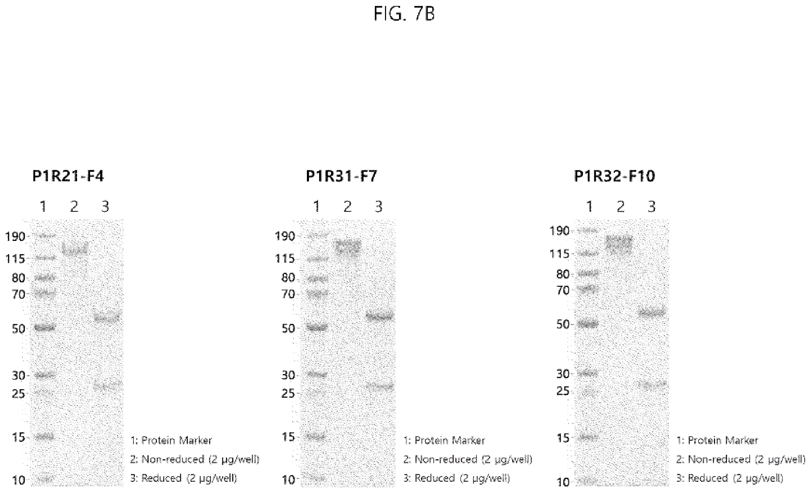

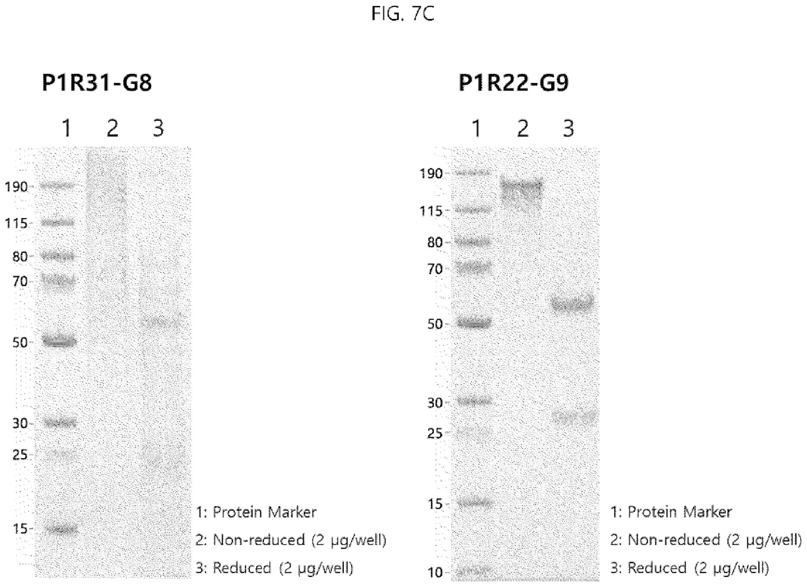

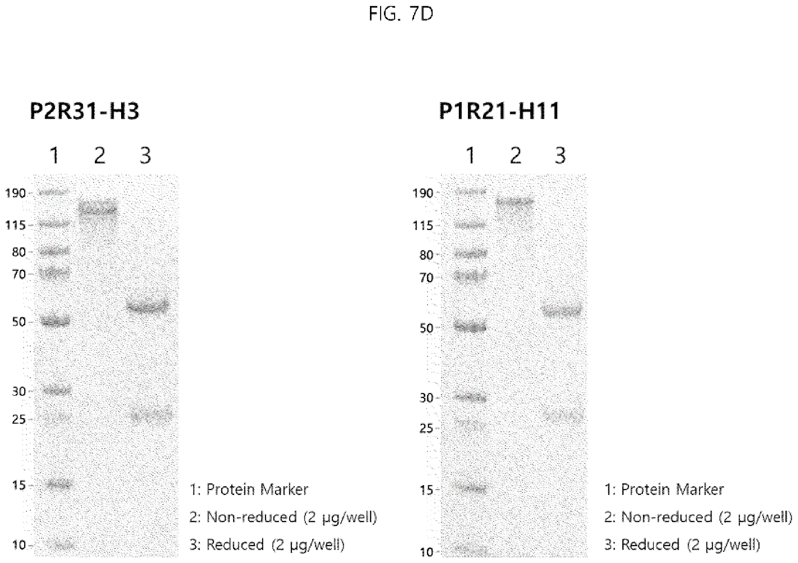

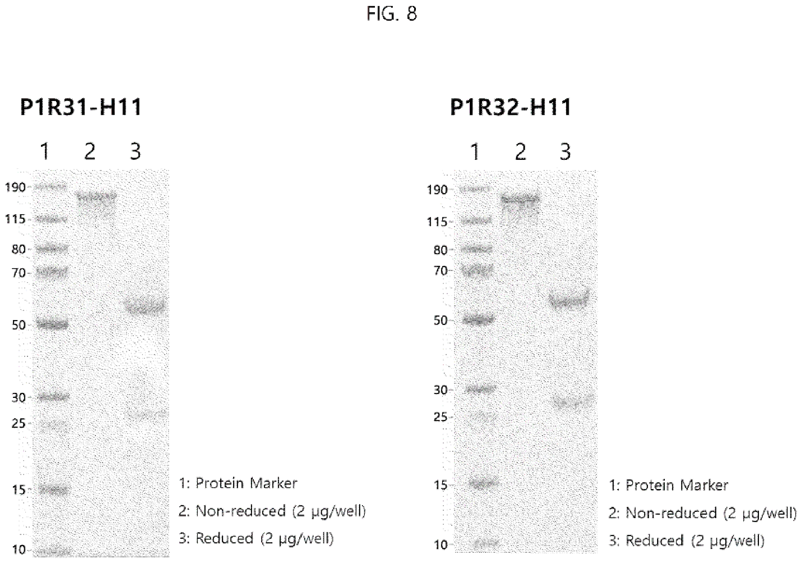

Specifically, the anti-MYCT1 antibody of Example 2-4, non-reducing samples and reducing samples were each prepared. A non-reducing sample was prepared by adding 4X LDS sample buffer (6 µL) to 2 µg/18 µL, vortexing at 3,300 rpm for 10 seconds, and centrifuging for 1 minute. A reducing sample was prepared by adding 4X LDS sample buffer (6 µL) and 10X sample reducing reagent (2.5 µL) to 2 µg/15.5 µL, vortexing at 3,300 rpm for 10 seconds, heating at 100°C for 10 minutes in a heating block, and centrifuging for 1 minute. Each sample, protein marker (5 µL) and test solution (24 µL) were loaded into a 4-12% Bis-Tris Mini Protein Gel, and electrophoresis was performed at 140 V for 60 minutes. After electrophoresis, EZ-Gel Staining Solution (20 mL) was added, staining was performed for 1 hour, and decolorization with triple-distilled water allowed observation of bands. To confirm purity by SEC-HPLC, a TSKgel G3000SWXL Column was used, a 10 µg sample was injected, and 50 mM sodium phosphate, 150 mM NaCl, pH 7.0 was run at a flow rate of 1 mL/min for 30 minutes, and the UV 214 nm value was measured to analyze purity (SEC-HPLC), and the results are shown in FIGs. 6 through 10. FIGs. 6A, 6B, 6C FIGs. 7A, 7B, 7C, 7D and FIG. 8 show the results of confirming purity (SDS-PAGE) of the anti-MYCT1 antibodies of Example 2-4. Table 23 shows the results of confirming purity (SEC-HPLC) of the anti-MYCT1 antibodies of Example 2-4. As shown in Table 23 and FIGs. 6A, 6B, 6C,, FIGs. 7A, 7B, 7C, 7D and FIG. 8, it was confirmed that the purity of the anti-MYCT1 antibodies of Example 2-4 was high.

**Table 23.**

| **Name** | **Purity (Area %)** | **Name** | **Purity (Area %)** |
|---|---|---|---|
| P1R21-A1 | 98.8 | P1R32-E9 | 99.4 |
| P1R32-A6 | 99.8 | P1R31-E10 | 98.7 |
| P1R31-A10 | 96.8 | P1R21-F4 | 82.2 |
| P1R32-A10 | ND | P1R31-F7 | 95.5 |
| P1R21-B3 | 98.4 | P1R32-F10 | 96.5 |
| P1R31-B8 | 97.5 | P1R31-G8 | ND |
| P1R31-C8 | 95.8 | P1R22-G9 | 96.9 |
| P1R32-C8 | 91.1 | P2R31-H3 | ND |
| P1R32-C12 | 99.7 | P1R21-H11 | 96.0 |
| P1R31-E1 | 98.1 | P1R31-H11 | 97.0 |
| P1R21-E3 | 98.4 | P1R32-H11 | 98.4 |
| P1R21-E4 | 94.8 | | |

### Example 3. Analysis of Binding Specificity of anti-MYCT1 Antibodies to MYCT1

### 3-1. Cloning of anti-MYCT1 scFv and Fab into Full-Length IgG Form

The specific binding capacity of the anti-MYCT1 antibodies purified in Example 2-4 to the synthetic peptide MYCT1 antigen was analyzed by ELISA method. Specifically, the extracellular domain of human MYCT1 was used as the antigen protein, and biotin conjugation was performed at the N-terminus and C-terminus, respectively. Streptavidin was used together to ensure effective exposure of the epitope of the antigen. A secondary antibody of HRP-conjugated anti-human polyclonal antibody reagent (Sigma, cat# A8667-2ML) was used, and the test was performed as follows.

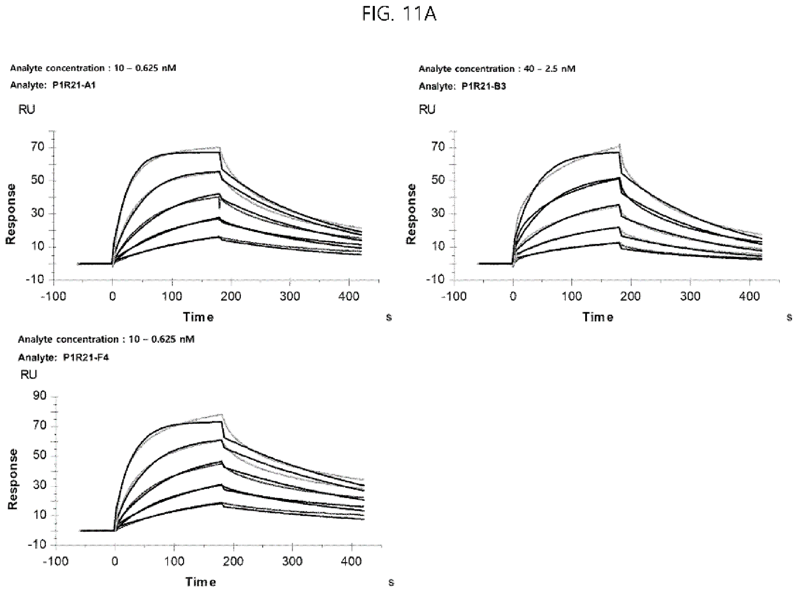

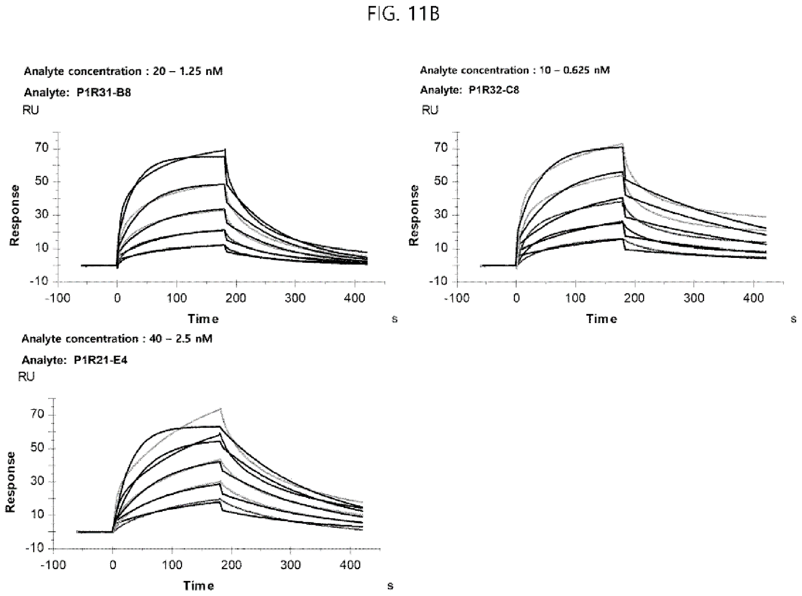

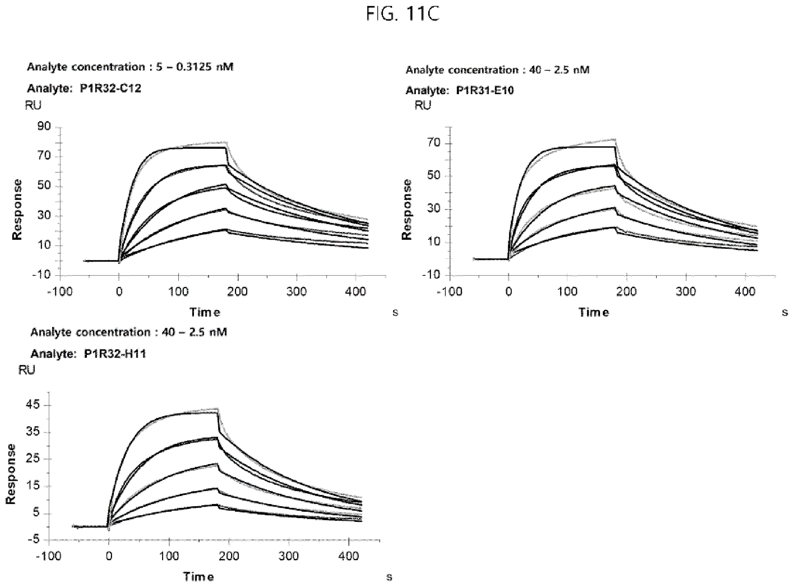

Fifty nM of streptavidin and 50 nM of synthetic peptide antigen MYCT1 were coated on an ELISA plate per well, and 3% BSA was incubated to prevent non-specific binding, then the plate was washed with PBS-T (0.05%). The prepared anti-MYCT1 antibodies were added to each well and serially diluted 3-fold from a concentration of 100 nM in 12 steps, then incubated for 1 hour at room temperature, and the plate was washed three times with PBS-T. Subsequently, color development was performed using HRP-conjugated anti-human polyclonal antibody and TMB (Sigma, cat# T4319-4X100ML), and the absorbance was measured at 450 nm, and the results were analyzed, with results shown in FIG. 11.

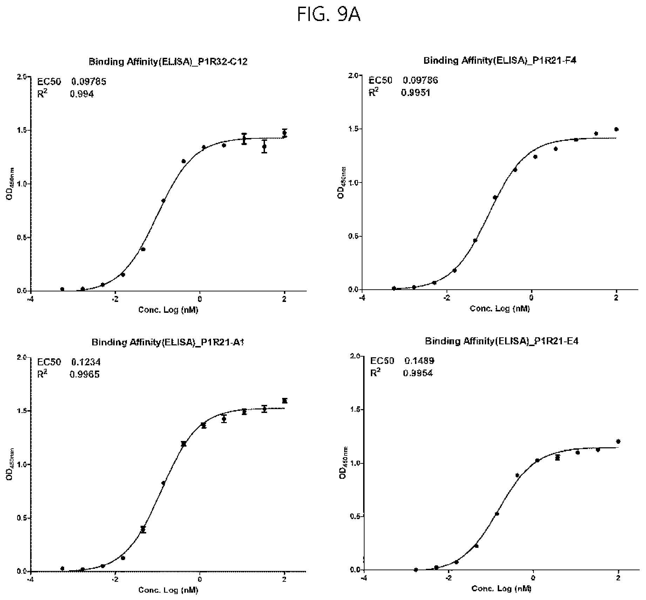

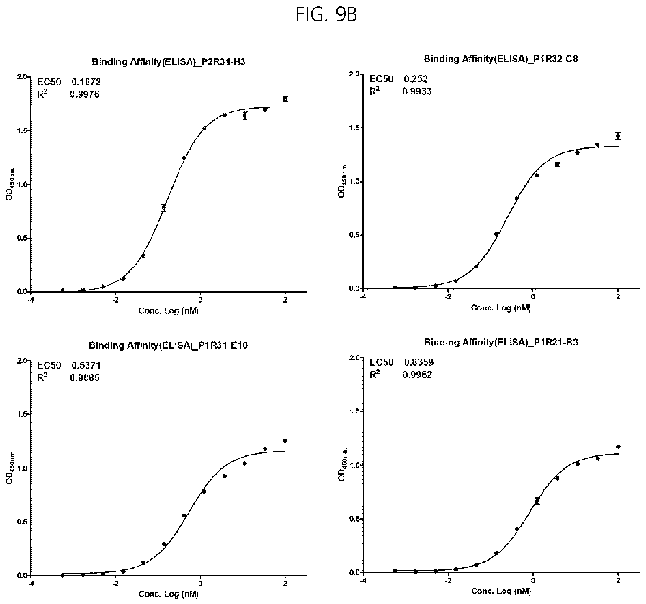

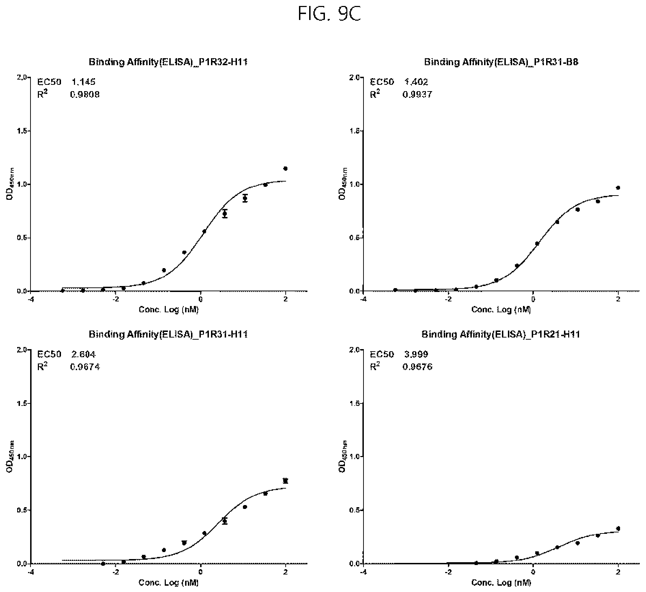

FIGs. 9A, 9B and 9C are graphs showing the results of analyzing the specific binding capacity of the purified anti-MYCT1 antibodies to the synthetic peptide antigen MYCT1 by ELISA method. Among the 22 anti-MYCT1 antibodies, the top 12 antibodies with high binding capacity were selected.

### 3-2. Measurement of Binding Ability of anti-MYCT1 Antibodies to MYCT1 Antigen Expressed on Cell Surface

The degree of binding of the selected anti-MYCT1 antibodies to MYCT1 expressed on the cell surface was analyzed through a FACS system. Specifically, MLEC-HA cells overexpressing human MYCT1 were prepared and compared with primary HUVEC cells.

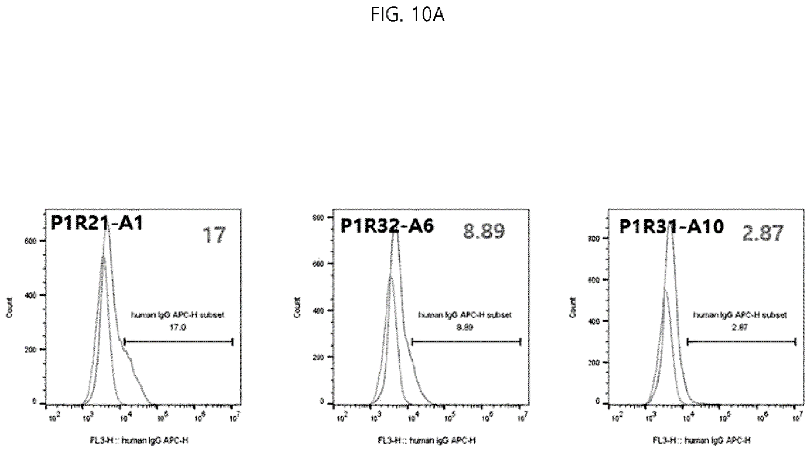

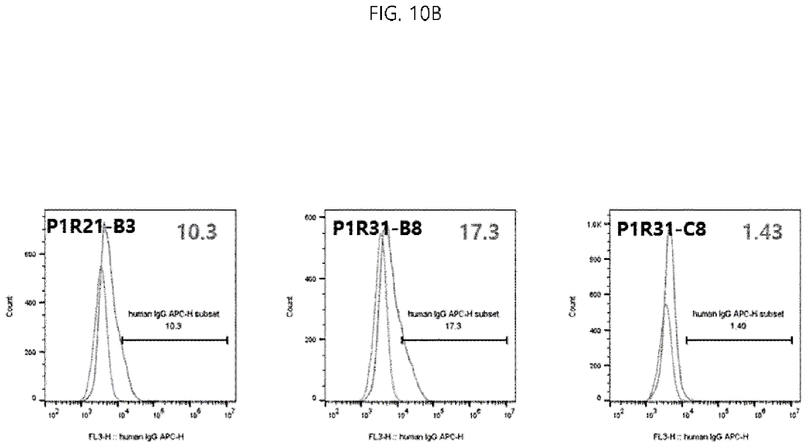

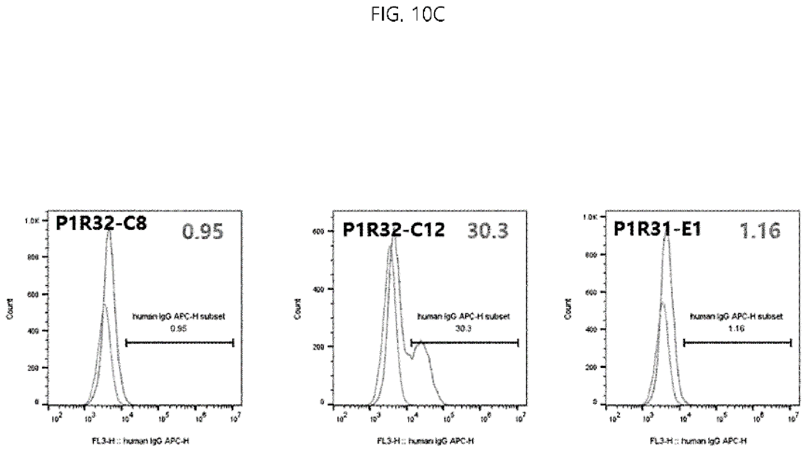

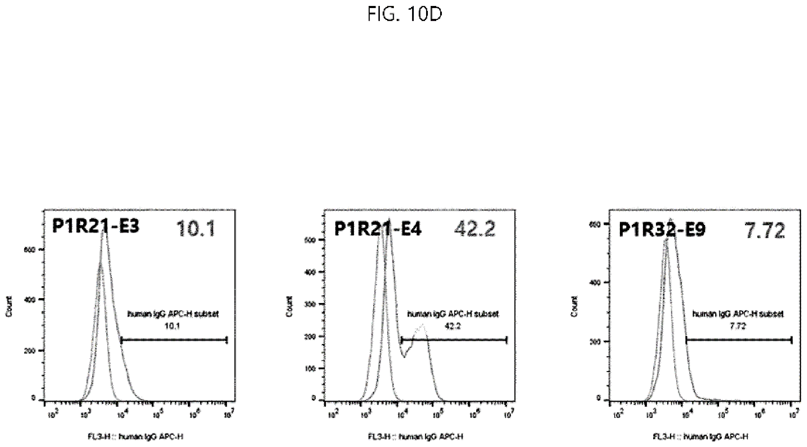

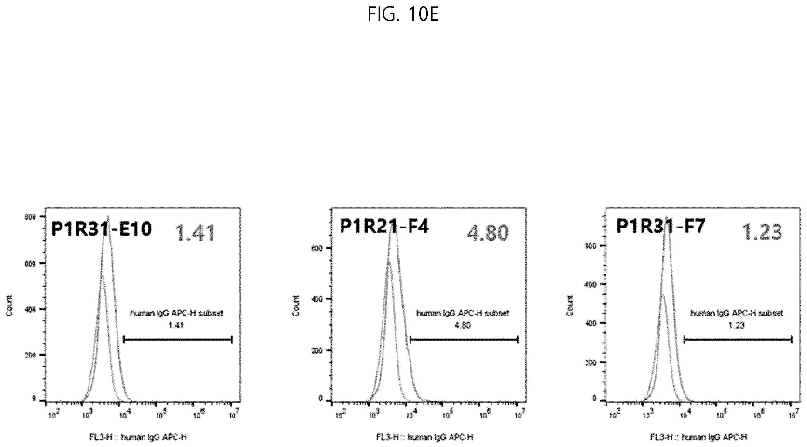

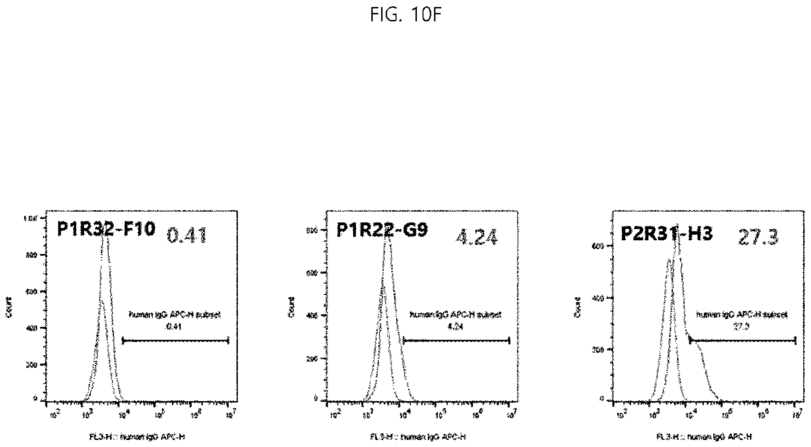

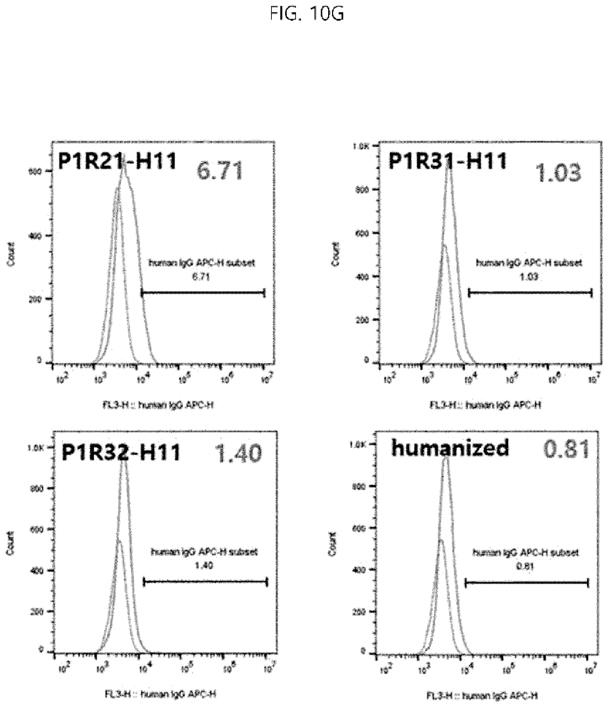

Anti-MYCT1 antibodies purified in Example 2-4 were added to the prepared cells at 300 nM, incubated at 4°C for 1 hour, then washed twice with PBS. Anti-human IgG antibody-APC diluted to 10 nM was incubated at 4°C for 30 minutes, then washed three times with PBS, and the fluorescence intensity of cells was measured using a CytoFLEX S (Beckman) apparatus, with results shown in FIGs. 10A, 10B, 10C, 10D, 10E, 10F and 10G.

FIGs. 10A, 10B, 10C, 10D, 10E, 10F and 10g are graphs showing the results of analyzing the degree of binding of the anti-MYCT1 antibodies purified in Example 2-4 to MYCT1 expressed on the cell surface through a FACS system.

### 3-3. SPR Full Kinetic Analysis of anti-MYCT1 Antibodies

SPR full kinetic analysis was performed to determine the binding affinity of the selected anti-MYCT1 antibodies to human MYCT1 peptide. The SA sensor chip used for SPR analysis was based on biotinylated ligand capture method, and the MYCT1 peptide ligand was immobilized at approximately 28 RU to evaluate binding capacity. Binding constants (Ka, Kd, KD) for each antibody were derived, and data reliability was evaluated, and the corresponding example was used to quantify specific binding capacity between antigen and antibody and to demonstrate the superiority of the antibodies.

Specifically, the ligand used was biotinylated MYCT1 peptide, and from the 12 types with high ELISA binding capacity in Example 3-1, 10 types of antibodies were selected as analytes. The running buffer used for analysis was HBS-EP+ pH 7.4 buffer, and the regeneration buffer was 10 mM glycine-HCl pH 2.0 buffer. The injection conditions were contact time of 180 seconds, dissociation time of 240 seconds, and flow rate of 30 µL/min, and analysis was performed based on a 1:1 binding model. Each binding constant was within the criteria of reliable statistical indicator Chi2 value (below 10% of Rmax) and U-value (not above~25), and binding affinity results were obtained from all antibodies except the P2R31-H3 antibody, which appeared to show non-specific binding to the SA sensor chip. The results are shown in Table 24 and FIGs. 11A, 11B and 11C.

**Table 24.**

| Order | Ligand | Analyte | Kₐ(1/Ms) | Kₐ(1/s) | Kₚ(M) | Rmax (RU) | CHi² (RU²) | U-value |
|---|---|---|---|---|---|---|---|---|
| 1 | | P1R21-A1 | 4.61x10⁶ | 5.67x10⁻³ | 1.23x10⁻⁹ | 65.78 | 1.91 | 1.43 |
| 2 | | P1R21-B3 | 5.00x10⁵ | 5.38x10⁻³ | 1.08×10⁻³ | 71.83 | 2.25 | 143 |
| 3 | | P1R31-B8 | 1.41x10⁶ | 1.05x10⁻² | 7.47×10⁻⁹ | 69.63 | 2.75 | 1.86 |
| 4 | | P1R32-C8 | 2.14x10⁶ | 3.53x10⁻³ | 1.65x10⁻⁹ | 61.76 | 5.75 | 2.41 |
| 5 | Biotinylated MYCTI peptide | P1R32-C12 | 1.19x10⁷ | 5.11x10⁻³ | 4.29x10⁻¹⁰ | 71.55 | 3.44 | 1.86 |
| 6 | | P1R31-E10 | 1.18x10⁶ | 5.38x10⁻³ | 4.57x10⁻⁹ | 63.41 | 2.97 | 1.43 |
| 7 | | P1R21-F4 | 3.13x10⁵ | 3.06x10⁻³ | 9.76x10⁻¹⁰ | 69.74 | 3.45 | 1.43 |
| 8 | | P1R31-H3 | - | - | - | - | - | - |
| 9 | | P1R21-E4 | 7.66x10⁵ | 6.13×10⁻³ | 8.00×10⁻⁹ | 75.08 | 5.25 | 1.86 |
| 10 | | P1R32-H11 | 8.32x10⁵ | 6.80x10⁻³ | 8.17x10⁻⁹ | 43.45 | 0.57 | 1.43 |

| | | | | | | | | |
|---|---|---|---|---|---|---|---|---|
| * CHi2: Below 10% of Rmax ** U-value: Not above ~25 | | | | | | | | |

### Example 4. Evaluation of Anti-angiogenic Efficacy of anti-MYCT1 Antibodies (Migration Assay)

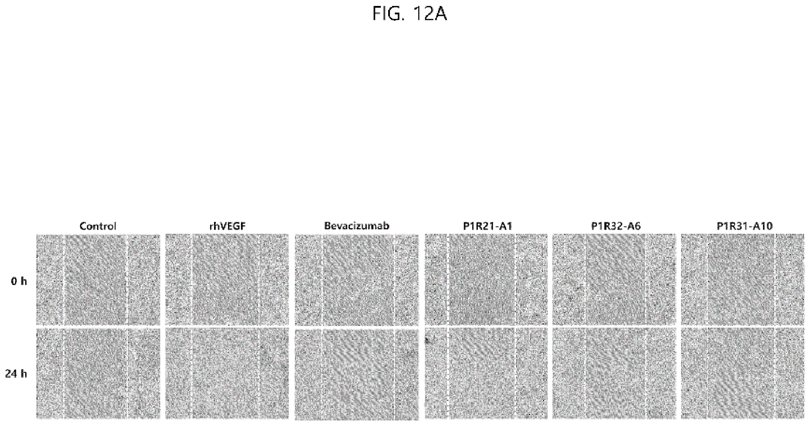

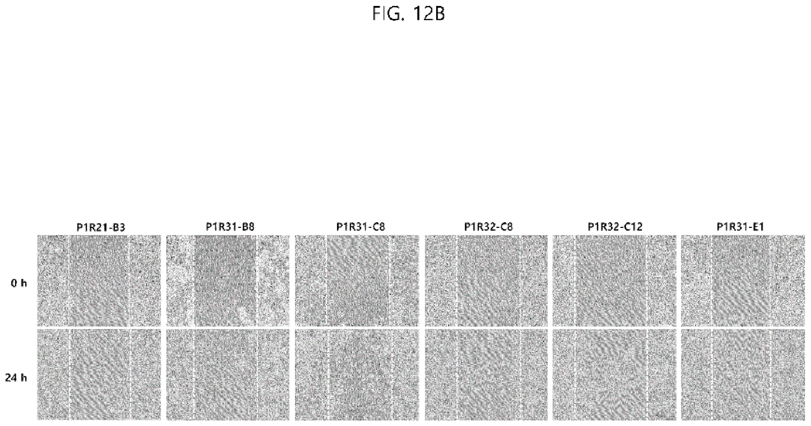

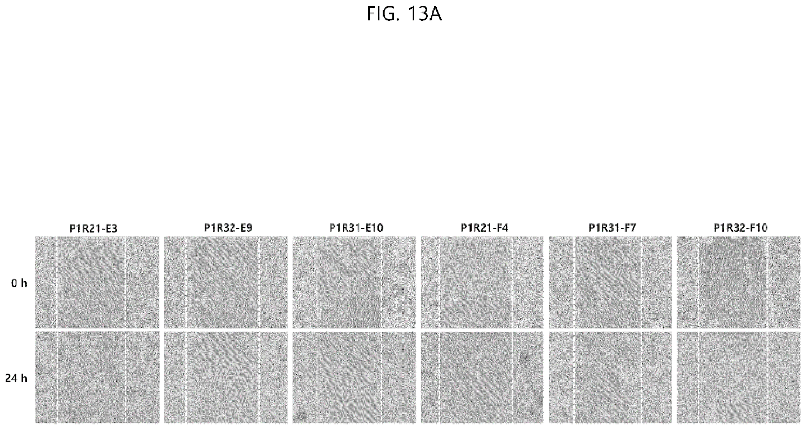

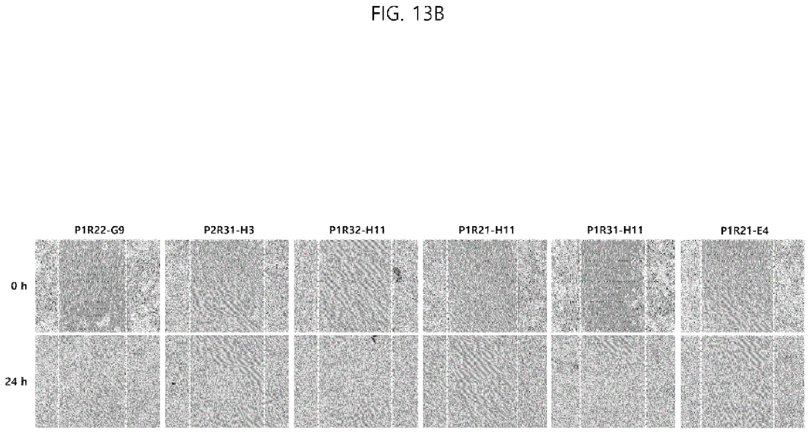

Human umbilical vein endothelial cells (HUVEC) (Lonza, Switzerland) were cultured in EGM-2 (endothelial growth medium, Lonza, Switzerland) containing 10% FBS (fetal bovine serum), insulin (5 µg/mL), rhFGF (recombinant human basic fibroblast growth factor; 1 µg/mL), rhEGF (recombinant human epidermal growth factor; 0.1 µg/mL), gentamicin (50 µg/mL) and amphotericin B (50 µg/mL) medium, and cells of passage 4 to 5 were used. To obtain cells, cells were washed with PBS, treated with 0.25% trypsin-EDTA (Gibco, USA), and after 2 minutes, M199 (Lonza, Switzerland) containing 10% fetal bovine serum was added to neutralize the enzyme activity. After detachment, cells were collected and centrifuged at 1,700 rpm for 3 minutes, EGM-2 was added, and cells were diluted and suspended to reach the desired cell number. Subsequently, a woundhealing assay was performed according to the manufacturer's protocol (Ibidi, Martinsried, Germany). Specifically, cells were seeded into each well, and with the exception of control, recombinant human VEGF at 50 ng/mL was added to all experimental groups, and anti-MYCT1 antibodies purified in Example 2-4 were added to each well at 300 nM, then cultured for 24 hours, and images were captured under a microscope and evaluated through image analysis using an image analysis program Image J (NIH, USA). Bevacizumab-treated group was used as a positive control. The results are shown in FIGs. 12A, 12B and FIGs. 13A and 13B.

As shown in FIGs. 12A, 12B and FIGs. 13A and 13B, it was confirmed that the evaluated anti-MYCT1 human antibodies had equivalent or superior ability to inhibit cell migration compared to the control group.

As described above, although the examples have been explained by limited drawings, those skilled in the art can apply various technical modifications and variations based on the above. For example, the described techniques may be performed in a different order than described, and/or components of the described systems, structures, devices, circuits, etc. may be combined or combined in forms different from those described, or may be replaced or substituted with other components or equivalents, and appropriate results can still be achieved.

Therefore, other implementations, other examples, and those equivalent to the claims are also within the scope of the claims described below.

## Claims

1. An anti-MYCT1 human antibody or an antigen-binding fragment thereof, specifically binding to human MYCT1, comprising:
a heavy chain CDR1 (HCDR-1) comprising an amino acid sequence selected from the group consisting of SEQ ID NOs: 4 to 43;
a heavy chain CDR2 (HCDR-2) comprising an amino acid sequence selected from the group consisting of SEQ ID NOs: 44 to 85;
a heavy chain CDR3 (HCDR-3) comprising an amino acid sequence selected from the group consisting of SEQ ID NOs: 86 to 187;
a light chain CDR1 (LCDR-1) comprising an amino acid sequence selected from the group consisting of SEQ ID NOs: 188 to 264;
a light chain CDR2 (LCDR-2) comprising an amino acid sequence selected from the group consisting of SEQ ID NOs: 265 to 320; and
a light chain CDR3 (LCDR-3) comprising an amino acid sequence selected from the group consisting of SEQ ID NOs: 321 to 411.

2. The anti-MYCT1 human antibody or antigen-binding fragment thereof of claim 1, wherein the HCDR-1, HCDR-2, HCDR-3, LCDR-1, LCDR-2, and LCDR-3 comprise, in order, CDR combinations of amino acid sequences presented in the following:
SEQ ID NOs: 13, 76, 105, 222, 301, and 346; SEQ ID NOs: 40, 53, 160, 262, 278, and 404;
SEQ ID NOs: 42, 56, 113, 262, 283, and 407; SEQ ID NOs: 26, 44, 147, 201, 266, and 358;
SEQ ID NOs: 25, 77, 110, 251, 293, and 388; SEQ ID NOs: 6, 68, 143, 230, 304, and 325;
SEQ ID NOs: 25, 77, 109, 252, 293, and 382; SEQ ID NOs: 42, 56, 112, 262, 283, and 409;
SEQ ID NOs: 40, 52, 160, 245, 279, and 401; SEQ ID NOs: 42, 56, 98, 233, 272, and 338;
SEQ ID NOs: 42, 56, 95, 237, 310, and 324; SEQ ID NOs: 25, 77, 114, 246, 275, and 403;
SEQ ID NOs: 25, 77, 126, 251, 293, and 386; SEQ ID NOs: 30, 48, 130, 194, 320, and 378;
SEQ ID NOs: 25, 77, 128, 249, 307, and 328; SEQ ID NOs: 33, 72, 91, 205, 296, and 368;
SEQ ID NOs: 42, 56, 156, 222, 301, and 346; SEQ ID NOs: 16, 63, 131, 262, 282, and 397;
SEQ ID NOs: 25, 77, 125, 251, 293, and 384; SEQ ID NOs: 42, 56, 118, 264, 277, and 392;
SEQ ID NOs: 25, 77, 117, 251, 293, and 387; SEQ ID NOs: 42, 56, 95, 239, 310, and 324;
SEQ ID NOs: 20, 74, 138, 200, 297, and 354; SEQ ID NOs: 6, 65, 123, 193, 319, and 373;
SEQ ID NOs: 42, 56, 95, 239, 310, and 322; SEQ ID NOs: 42, 59, 90, 254, 279, and 395;
SEQ ID NOs: 42, 56, 94, 239, 310, and 325; SEQ ID NOs: 42, 56, 88, 261, 276, and 393;
SEQ ID NOs: 8, 66, 116, 189, 315, and 340; SEQ ID NOs: 42, 56, 133, 260, 295, and 394;
SEQ ID NOs: 42, 56, 87, 241, 290, and 334; SEQ ID NOs: 24, 79, 137, 262, 283, and 405;
SEQ ID NOs: 7, 64, 186, 227, 273, and 342; SEQ ID NOs: 30, 48, 129, 197, 319, and 378;
SEQ ID NOs: 25, 77, 99, 251, 293, and 387; SEQ ID NOs: 25, 77, 164, 203, 296, and 369;
SEQ ID NOs: 42, 56, 163, 250, 294, and 383; SEQ ID NOs: 34, 54, 146, 229, 303, and 333;
SEQ ID NOs: 39, 81, 127, 244, 312, and 335; SEQ ID NOs: 5, 67, 144, 228, 310, and 321;
SEQ ID NOs: 26, 44, 150, 235, 309, and 330; SEQ ID NOs: 42, 56, 104, 247, 293, and 385;
SEQ ID NOs: 27, 77, 172, 262, 278, and 408; SEQ ID NOs: 42, 56, 101, 195, 319, and 379;
SEQ ID NOs: 26, 44, 161, 239, 310, and 324; SEQ ID NOs: 42, 56, 97, 197, 319, and 375;
SEQ ID NOs: 43, 80, 149, 243, 317, and 323; SEQ ID NOs: 27, 51, 152, 210, 268, and 357;
SEQ ID NOs: 25, 78, 119, 231, 293, and 387; SEQ ID NOs: 15, 47, 107, 215, 301, and 341;
SEQ ID NOs: 42, 56, 102, 258, 274, and 402; SEQ ID NOs: 35, 61, 151, 214, 301, and 347;
SEQ ID NOs: 42, 56, 95, 239, 310, and 325; SEQ ID NOs: 8, 66, 116, 188, 314, and 340;
SEQ ID NOs: 14, 85, 153, 220, 299, and 349; SEQ ID NOs: 39, 81, 127, 244, 313, and 335;
SEQ ID NOs: 42, 57, 132, 191, 305, and 389; SEQ ID NOs: 36, 75, 121, 236, 280, and 337;
SEQ ID NOs: 25, 77, 168, 221, 301, and 345; SEQ ID NOs: 31, 56, 148, 197, 319, and 378;
SEQ ID NOs: 42, 56, 106, 256, 285, and 396; SEQ ID NOs: 26, 44, 167, 255, 284, and 398;
SEQ ID NOs: 42, 56, 95, 190, 310, and 324; SEQ ID NOs: 6, 65, 124, 197, 319, and 373;
SEQ ID NOs: 17, 62, 120, 196, 319, and 380; SEQ ID NOs: 4, 65, 166, 226, 318, and 336;
SEQ ID NOs: 42, 57, 96, 239, 310, and 325; SEQ ID NOs: 25, 77, 154, 217, 302, and 343;
SEQ ID NOs: 42, 56, 115, 263, 281, and 411; SEQ ID NOs: 32, 49, 108, 225, 307, and 410;
SEQ ID NOs: 42, 56, 95, 242, 308, and 331; SEQ ID NOs: 26, 44, 150, 234, 316, and 326;
SEQ ID NOs: 42, 56, 95, 192, 311, and 322; SEQ ID NOs: 26, 44, 155, 235, 309, and 330;
SEQ ID NOs: 25, 77, 168, 213, 301, and 345; SEQ ID NOs: 24, 79, 137, 262, 283, and 406;
SEQ ID NOs: 42, 56, 100, 248, 307, and 391; SEQ ID NOs: 26, 44, 183, 197, 319, and 378;
SEQ ID NOs: 25, 77, 93, 251, 293, and 387; SEQ ID NOs: 42, 55, 103, 259, 283, and 400;
SEQ ID NOs: 42, 56, 92, 262, 278, and 399; SEQ ID NOs: 26, 44, 170, 198, 288, and 352;
SEQ ID NOs: 25, 77, 165, 251, 291, and 387; SEQ ID NOs: 42, 56, 102, 257, 274, and 402;
SEQ ID NOs: 25, 77, 111, 253, 292, and 381; SEQ ID NOs: 38, 58, 86, 219, 301, and 348;
SEQ ID NOs: 42, 56, 122, 262, 283, and 405; SEQ ID NOs: 42, 56, 134, 209, 289, and 364;
SEQ ID NOs: 32, 50, 162, 216, 300, and 344; SEQ ID NOs: 20, 69, 141, 211, 269, and 356;
SEQ ID NOs: 12, 73, 140, 199, 297, and 353; SEQ ID NOs: 28, 70, 145, 218, 300, and 390;
SEQ ID NOs: 21, 74, 135, 210, 267, and 370; SEQ ID NOs: 20, 45, 177, 210, 289, and 363;
SEQ ID NOs: 28, 46, 184, 204, 287, and 366; SEQ ID NOs: 20, 45, 175, 209, 265, and 350;
SEQ ID NOs: 26, 74, 178, 209, 286, and 377; SEQ ID NOs: 21, 74, 187, 209, 268, and 374;
SEQ ID NOs: 19, 44, 142, 200, 297, and 355; SEQ ID NOs: 23, 74, 174, 211, 268, and 351;
SEQ ID NOs: 10, 74, 89, 209, 289, and 372; SEQ ID NOs: 37, 60, 139, 206, 286, and 339;
SEQ ID NOs: 41, 83, 157, 232, 306, and 329; SEQ ID NOs: 18, 74, 178, 209, 268, and 372;
SEQ ID NOs: 32, 49, 179, 224, 300, and 344; SEQ ID NOs: 20, 74, 176, 210, 287, and 359;
SEQ ID NOs: 26, 74, 178, 209, 287, and 361; SEQ ID NOs: 22, 71, 171, 210, 286, and 360;
SEQ ID NOs: 11, 44, 159, 208, 286, and 371; SEQ ID NOs: 20, 44, 185, 212, 287, and 365;
SEQ ID NOs: 41, 84, 136, 238, 271, and 332; SEQ ID NOs: 20, 44, 173, 202, 298, and 367;
SEQ ID NOs: 20, 74, 182, 209, 286, and 376; SEQ ID NOs: 9, 74, 180, 207, 286, and 362;
SEQ ID NOs: 41, 82, 158, 240, 270, and 327; or SEQ ID NOs: 29, 44, 181, 209, 286, and 376.

3. The anti-MYCT1 human antibody or antigen-binding fragment thereof of claim 1, comprising at least one variable heavy chain region (VH) selected from the group consisting of SEQ ID NOs: 412 to 527.

4. The anti-MYCT1 human antibody or antigen-binding fragment thereof of claim 1, comprising at least one variable light chain region (VL) selected from the group consisting of SEQ ID NOs: 528 to 643.

5. The anti-MYCT1 human antibody or antigen-binding fragment thereof of claim 1, comprising:
the VH sequence of SEQ ID NO: 412 and the VL sequence of SEQ ID NO: 528;
the VH sequence of SEQ ID NO: 413 and the VL sequence of SEQ ID NO: 529;
the VH sequence of SEQ ID NO: 414 and the VL sequence of SEQ ID NO: 530;
the VH sequence of SEQ ID NO: 415 and the VL sequence of SEQ ID NO: 531;
the VH sequence of SEQ ID NO: 416 and the VL sequence of SEQ ID NO: 532;
the VH sequence of SEQ ID NO: 417 and the VL sequence of SEQ ID NO: 533;
the VH sequence of SEQ ID NO: 418 and the VL sequence of SEQ ID NO: 534;
the VH sequence of SEQ ID NO: 419 and the VL sequence of SEQ ID NO: 535;
the VH sequence of SEQ ID NO: 420 and the VL sequence of SEQ ID NO: 536;
the VH sequence of SEQ ID NO: 421 and the VL sequence of SEQ ID NO: 537;
the VH sequence of SEQ ID NO: 422 and the VL sequence of SEQ ID NO: 538;
the VH sequence of SEQ ID NO: 423 and the VL sequence of SEQ ID NO: 539;
the VH sequence of SEQ ID NO: 424 and the VL sequence of SEQ ID NO: 540;
the VH sequence of SEQ ID NO: 425 and the VL sequence of SEQ ID NO: 541;
the VH sequence of SEQ ID NO: 426 and the VL sequence of SEQ ID NO: 542;
the VH sequence of SEQ ID NO: 427 and the VL sequence of SEQ ID NO: 543;
the VH sequence of SEQ ID NO: 428 and the VL sequence of SEQ ID NO: 544;
the VH sequence of SEQ ID NO: 429 and the VL sequence of SEQ ID NO: 545;
the VH sequence of SEQ ID NO: 430 and the VL sequence of SEQ ID NO: 546;
the VH sequence of SEQ ID NO: 431 and the VL sequence of SEQ ID NO: 547;
the VH sequence of SEQ ID NO: 432 and the VL sequence of SEQ ID NO: 548;
the VH sequence of SEQ ID NO: 433 and the VL sequence of SEQ ID NO: 549; or
the VH sequence of SEQ ID NO: 434 and the VL sequence of SEQ ID NO: 550.

6. The anti-MYCT1 human antibody or antigen-binding fragment thereof of claim 1, comprising:
the VH sequence of SEQ ID NO: 435 and the VL sequence of SEQ ID NO: 551;
the VH sequence of SEQ ID NO: 436 and the VL sequence of SEQ ID NO: 552;
the VH sequence of SEQ ID NO: 437 and the VL sequence of SEQ ID NO: 553;
the VH sequence of SEQ ID NO: 438 and the VL sequence of SEQ ID NO: 554;
the VH sequence of SEQ ID NO: 439 and the VL sequence of SEQ ID NO: 555;
the VH sequence of SEQ ID NO: 440 and the VL sequence of SEQ ID NO: 556;
the VH sequence of SEQ ID NO: 441 and the VL sequence of SEQ ID NO: 557;
the VH sequence of SEQ ID NO: 442 and the VL sequence of SEQ ID NO: 558;
the VH sequence of SEQ ID NO: 443 and the VL sequence of SEQ ID NO: 559;
the VH sequence of SEQ ID NO: 444 and the VL sequence of SEQ ID NO: 560;
the VH sequence of SEQ ID NO: 445 and the VL sequence of SEQ ID NO: 561;
the VH sequence of SEQ ID NO: 446 and the VL sequence of SEQ ID NO: 562;
the VH sequence of SEQ ID NO: 447 and the VL sequence of SEQ ID NO: 563;
the VH sequence of SEQ ID NO: 448 and the VL sequence of SEQ ID NO: 564;
the VH sequence of SEQ ID NO: 449 and the VL sequence of SEQ ID NO: 565;
the VH sequence of SEQ ID NO: 450 and the VL sequence of SEQ ID NO: 566;
the VH sequence of SEQ ID NO: 451 and the VL sequence of SEQ ID NO: 567;
the VH sequence of SEQ ID NO: 452 and the VL sequence of SEQ ID NO: 568;
the VH sequence of SEQ ID NO: 453 and the VL sequence of SEQ ID NO: 569;
the VH sequence of SEQ ID NO: 454 and the VL sequence of SEQ ID NO: 570;
the VH sequence of SEQ ID NO: 455 and the VL sequence of SEQ ID NO: 571;
the VH sequence of SEQ ID NO: 456 and the VL sequence of SEQ ID NO: 572;
the VH sequence of SEQ ID NO: 457 and the VL sequence of SEQ ID NO: 573;
the VH sequence of SEQ ID NO: 458 and the VL sequence of SEQ ID NO: 574;
the VH sequence of SEQ ID NO: 459 and the VL sequence of SEQ ID NO: 575;
the VH sequence of SEQ ID NO: 460 and the VL sequence of SEQ ID NO: 576;
the VH sequence of SEQ ID NO: 461 and the VL sequence of SEQ ID NO: 577;
the VH sequence of SEQ ID NO: 462 and the VL sequence of SEQ ID NO: 578;
the VH sequence of SEQ ID NO: 463 and the VL sequence of SEQ ID NO: 579;
the VH sequence of SEQ ID NO: 464 and the VL sequence of SEQ ID NO: 580;
the VH sequence of SEQ ID NO: 465 and the VL sequence of SEQ ID NO: 581;
the VH sequence of SEQ ID NO: 466 and the VL sequence of SEQ ID NO: 582;
the VH sequence of SEQ ID NO: 467 and the VL sequence of SEQ ID NO: 583;
the VH sequence of SEQ ID NO: 468 and the VL sequence of SEQ ID NO: 584;
the VH sequence of SEQ ID NO: 469 and the VL sequence of SEQ ID NO: 585;
the VH sequence of SEQ ID NO: 470 and the VL sequence of SEQ ID NO: 586;
the VH sequence of SEQ ID NO: 471 and the VL sequence of SEQ ID NO: 587;
the VH sequence of SEQ ID NO: 472 and the VL sequence of SEQ ID NO: 588;
the VH sequence of SEQ ID NO: 473 and the VL sequence of SEQ ID NO: 589;
the VH sequence of SEQ ID NO: 474 and the VL sequence of SEQ ID NO: 590;
the VH sequence of SEQ ID NO: 475 and the VL sequence of SEQ ID NO: 591;
the VH sequence of SEQ ID NO: 476 and the VL sequence of SEQ ID NO: 592;
the VH sequence of SEQ ID NO: 477 and the VL sequence of SEQ ID NO: 593;
the VH sequence of SEQ ID NO: 478 and the VL sequence of SEQ ID NO: 594;
the VH sequence of SEQ ID NO: 479 and the VL sequence of SEQ ID NO: 595;
the VH sequence of SEQ ID NO: 480 and the VL sequence of SEQ ID NO: 596;
the VH sequence of SEQ ID NO: 481 and the VL sequence of SEQ ID NO: 597;
the VH sequence of SEQ ID NO: 482 and the VL sequence of SEQ ID NO: 598;
the VH sequence of SEQ ID NO: 483 and the VL sequence of SEQ ID NO: 599;
the VH sequence of SEQ ID NO: 484 and the VL sequence of SEQ ID NO: 600;
the VH sequence of SEQ ID NO: 485 and the VL sequence of SEQ ID NO: 601;
the VH sequence of SEQ ID NO: 486 and the VL sequence of SEQ ID NO: 602;
the VH sequence of SEQ ID NO: 487 and the VL sequence of SEQ ID NO: 603;
the VH sequence of SEQ ID NO: 488 and the VL sequence of SEQ ID NO: 604;
the VH sequence of SEQ ID NO: 489 and the VL sequence of SEQ ID NO: 605;
the VH sequence of SEQ ID NO: 490 and the VL sequence of SEQ ID NO: 606;
the VH sequence of SEQ ID NO: 491 and the VL sequence of SEQ ID NO: 607;
the VH sequence of SEQ ID NO: 492 and the VL sequence of SEQ ID NO: 608;
the VH sequence of SEQ ID NO: 493 and the VL sequence of SEQ ID NO: 609;
the VH sequence of SEQ ID NO: 494 and the VL sequence of SEQ ID NO: 610;
the VH sequence of SEQ ID NO: 495 and the VL sequence of SEQ ID NO: 611;
the VH sequence of SEQ ID NO: 496 and the VL sequence of SEQ ID NO: 612;
the VH sequence of SEQ ID NO: 497 and the VL sequence of SEQ ID NO: 613;
the VH sequence of SEQ ID NO: 498 and the VL sequence of SEQ ID NO: 614;
the VH sequence of SEQ ID NO: 499 and the VL sequence of SEQ ID NO: 615;
the VH sequence of SEQ ID NO: 500 and the VL sequence of SEQ ID NO: 616;
the VH sequence of SEQ ID NO: 501 and the VL sequence of SEQ ID NO: 617;
the VH sequence of SEQ ID NO: 502 and the VL sequence of SEQ ID NO: 618;
the VH sequence of SEQ ID NO: 503 and the VL sequence of SEQ ID NO: 619;
the VH sequence of SEQ ID NO: 504 and the VL sequence of SEQ ID NO: 620;
the VH sequence of SEQ ID NO: 505 and the VL sequence of SEQ ID NO: 621;
the VH sequence of SEQ ID NO: 506 and the VL sequence of SEQ ID NO: 622;
the VH sequence of SEQ ID NO: 507 and the VL sequence of SEQ ID NO: 623;
the VH sequence of SEQ ID NO: 508 and the VL sequence of SEQ ID NO: 624;
the VH sequence of SEQ ID NO: 509 and the VL sequence of SEQ ID NO: 625;
the VH sequence of SEQ ID NO: 510 and the VL sequence of SEQ ID NO: 626;
the VH sequence of SEQ ID NO: 511 and the VL sequence of SEQ ID NO: 627;
the VH sequence of SEQ ID NO: 512 and the VL sequence of SEQ ID NO: 628;
the VH sequence of SEQ ID NO: 513 and the VL sequence of SEQ ID NO: 629;
the VH sequence of SEQ ID NO: 514 and the VL sequence of SEQ ID NO: 630;
the VH sequence of SEQ ID NO: 515 and the VL sequence of SEQ ID NO: 631;
the VH sequence of SEQ ID NO: 516 and the VL sequence of SEQ ID NO: 632;
the VH sequence of SEQ ID NO: 517 and the VL sequence of SEQ ID NO: 633;
the VH sequence of SEQ ID NO: 518 and the VL sequence of SEQ ID NO: 634;
the VH sequence of SEQ ID NO: 519 and the VL sequence of SEQ ID NO: 635;
the VH sequence of SEQ ID NO: 520 and the VL sequence of SEQ ID NO: 636;
the VH sequence of SEQ ID NO: 521 and the VL sequence of SEQ ID NO: 637;
the VH sequence of SEQ ID NO: 522 and the VL sequence of SEQ ID NO: 638;
the VH sequence of SEQ ID NO: 523 and the VL sequence of SEQ ID NO: 639;
the VH sequence of SEQ ID NO: 524 and the VL sequence of SEQ ID NO: 640;
the VH sequence of SEQ ID NO: 525 and the VL sequence of SEQ ID NO: 641;
the VH sequence of SEQ ID NO: 526 and the VL sequence of SEQ ID NO: 642; or
the VH sequence of SEQ ID NO: 527 and the VL sequence of SEQ ID NO: 643.

7. The anti-MYCT1 human antibody or antigen-binding fragment thereof of claim 1, wherein the antigen-binding fragment comprises of Fd, Fab, Fab', F(ab')2, dsFv, scFv, and a single domain antibody (sdAb).

8. A nucleic acid molecule encoding the antibody or antigen-binding fragment thereof of any one of claims 1 to 7.

9. A recombinant expression vector comprising the nucleic acid molecule of claim 8.

10. A host cell transfected with the expression vector of claim 9.

11. An immunoconjugate comprising the antibody or antigen-binding fragment thereof of any one of claims 1 to 7, and a cytotoxic agent.

12. A pharmaceutical composition for preventing or treating a MYCT1-related disease, comprising the antibody or antigen-binding fragment thereof of any one of claims 1 to 7 as an active ingredient.

13. The pharmaceutical composition, wherein the MYCT1-related disease is an angiogenesis-related disease or cancer.

14. The pharmaceutical composition of claim 13, wherein the pharmaceutical composition is administered in combination with at least one additional therapeutic agent or therapeutic procedure.

15. The pharmaceutical composition of claim 14, wherein the at least one additional therapeutic agent or therapeutic procedure is selected from one or more of chemotherapy, targeted cancer therapy, oncolytic drugs, cytotoxic agents, immune-based therapy, cytokines, surgical procedures, radiation therapy, vaccines, or cell therapy.

16. A diagnostic composition for a MYCT1-related disease, comprising the antibody or antigen-binding fragment thereof of any one of claims 1 to 7 as an active ingredient.

17. A pharmaceutical composition for enhancing sensitivity to an immune anticancer agent in a carcinoma, comprising the antibody or antigen-binding fragment thereof of any one of claims 1 to 7 as an active ingredient.

18. A chimeric antigen receptor (CAR) comprising an antigen-binding domain specifically binding to MYCT1, wherein the antigen-binding domain comprises an antigen-binding fragment comprising:
a heavy chain CDR1 (HCDR-1) comprising an amino acid sequence selected from the group consisting of SEQ ID NOs: 4 to 43;
a heavy chain CDR2 (HCDR-2) comprising an amino acid sequence selected from the group consisting of SEQ ID NOs: 44 to 85;
a heavy chain CDR3 (HCDR-3) comprising an amino acid sequence selected from the group consisting of SEQ ID NOs: 86 to 187;
a light chain CDR1 (LCDR-1) comprising an amino acid sequence selected from the group consisting of SEQ ID NOs: 188 to 264;
a light chain CDR2 (LCDR-2) comprising an amino acid sequence selected from the group consisting of SEQ ID NOs: 265 to 320; and
a light chain CDR3 (LCDR-3) comprising an amino acid sequence selected from the group consisting of SEQ ID NOs: 321 to 411.

19. An immune effector cell expressing the chimeric antigen receptor of claim 18.

20. A method of treating a MYCT1-related disease in a subject, comprising administering to the subject a composition comprising a therapeutically effective amount of the antibody or antigen-binding fragment thereof of any one of claims 1 to 7.

21. A method of enhancing sensitivity to an immune anticancer agent in a subject having resistance to the immune anticancer agent, comprising administering to the subject a composition comprising a therapeutically effective amount of the antibody or antigen-binding fragment thereof of any one of claims 1 to 7.
